# EUROPEAN PATENT APPLICATION

(11) **EP 4 669 030 A2**
(43) Date of publication of application: **24.12.2025**
(21) Application number: 25214046.2
(22) Date of filing: 02.12.2019
(51) Int. Cl.: H05B 3/34

(54) **SYSTEMS AND METHODS FOR REGULATING TEMPERATURE OF AN ARTICLE OF FURNITURE**

(30) Priority: 03.12.2018 US 201862774659 P; 12.02.2019 US 201962804729 P
(62) Divisional of application: 19892782.4
(71) Applicant: Eight Sleep Inc., New York, NY 10010 (US)
(72) Inventor: FRANCESCHETTI, Matteo, 10010 New York (US); LEE, Daipan, 10010 New York (US); GOETHALS, William, 10010 New York (US); COWLES, William Henry Harrison, 10010 New York (US); BASSI, Massimo Andreasi, 10010 New York (US); STEVENSON, Daan, 10010 New York (US); IRICK, Charles, 10010 New York (US); KATZ, Paul, 10010 New York (US)
(74) Representative: Murgitroyd & Company

(57) **Abstract**

The present disclosure provides a system for regulating a temperature of a portion of an article of furniture, and methods of use thereof. The system may comprise at least one sensor configured to detect a biological signal of a user of the article of furniture.

## Description

### CROSS-REFERENCE

This application claims the benefit of U.S. Provisional Patent Application No. 62/774,659, filed December 3, 2018 and U.S. Provisional Patent Application No. 62/804,729, filed February 12, 2019, each of which is entirely incorporated herein by reference.

### BACKGROUND

Regulating a temperature of an article of furniture (e.g., a bed) can help improve a quality of a person's activity on the furniture (e.g., sleeping on the bed). Current methods of, supporting and/or improving the user's sleep can comprise an electric blanket, a heated pad, or a bed warmer. The electric blanket, for example, may be a blanket with an integrated electrical heating device which can be placed above the top of a bed sheet or below the bottom of the bed sheet. The electric blanket may be used to pre-heat the bed before use or to keep the occupant warm while in bed. However, turning on the electric blanket may require the user to turn it on manually. Further, the electric blanket provides no additional functionality besides warming the bed.

### SUMMARY

The present disclosure describes technologies relating to regulating a temperature of an article of furniture, and more specifically the present disclosure describes using a fluid (e.g., a liquid or gas) and one or more temperature regulators of the fluid to regulate the temperature of a portion of the article of furniture.

In one aspect, the present disclosure provides a system for changing a temperature of a portion of an article of furniture, comprising: (a) at least one sensor that is a part of the article of furniture, wherein the at least one sensor is configured to detect a biological signal of a user of the article of furniture; (b) a temperature control device coupled to the portion of the article of furniture, wherein the temperature control device is configured to change the temperature of the portion of the article of furniture; and (c) a processor communicatively coupled to the sensor and the temperature control device, wherein the processor is configured to (i) designate, while the user is asleep on the article of furniture, a time for the article of furniture to wake up the user based on the biological signal of the user that is detected by the at least one sensor while the user is using the article of furniture, and (ii) change the temperature of the portion of the article of furniture by the temperature control device prior to the time.

In one aspect, the present disclosure provides a method of regulating a temperature of a portion of an article of furniture, comprising: (a) providing (i) at least one sensor that is a part of the article of furniture, wherein the at least one sensor is configured to detect a biological signal of a user of the article of furniture, (ii) a temperature control device coupled to the portion of the article of furniture, wherein the temperature control device is configured to change the temperature of the portion of the article of furniture, and (iii) a processor communicatively coupled to the at least one sensor and the temperature control device; (b) with aid of the at least one sensor, detecting the biological signal of the user of the article of furniture while the user is using the article of furniture; (c) with aid of the processor, designating, while the user is asleep on the article of furniture, a time for the article of furniture to wake up the user based at least in part on the detected biological signal of the user; and (d) with the aid of the processor, changing the temperature of the portion of the article of furniture by the temperature control device prior to the time.

In one aspect, the present disclosure provides a system for regulating a temperature of a portion of an article of furniture, comprising: (a) a temperature control device operatively coupled to the portion of the article of furniture, configured to change the temperature of the portion of the article of furniture; and (b) a processor communicatively coupled to the temperature control device, configured to designate a time to change the temperature of the portion of the article of furniture by the temperature control device based at least in part on a predetermined wake-up time of a user, wherein the time is prior to the predetermined wake-up time of the user.

In one aspect, the present disclosure provides a method of regulating a temperature of a portion of an article of furniture, comprising: (a) providing (i) a temperature control device operatively coupled to the portion of the article of furniture, configured to change the temperature of the portion of the article of furniture, and (ii) a processor communicatively coupled to the temperature control device; and (b) with aid of the processor, designating a time to change the temperature of the portion of the article of furniture by the temperature control device based at least in part on a predetermined wake-up time of a user, wherein the time is prior to the predetermined wake-up time of the user.

In one aspect, the present disclosure provides a system for regulating a temperature of an article of furniture, the system comprising: (a) a portion of the article of furniture configured to hold a fluid; (b) a reservoir in fluid communication with the portion of the article of furniture, wherein the reservoir is configured to contain the fluid; (c) a temperature regulator in fluid communication with the portion of the article of furniture and the reservoir, wherein the temperature regulator is configured to modulate a temperature of the fluid when the fluid is not contained in the reservoir; and (d) a processor operatively coupled to the temperature regulator, wherein the processor is programmed to control the temperature regulator to modulate the temperature of the fluid, thereby to regulate the temperature of the portion of the article of furniture.

In one aspect, the present disclosure provides a method for regulating a temperature of an article of furniture, the method comprising: (a) providing a temperature regulator in fluid communication with (i) the portion of the article of furniture capable of holding a fluid, and (ii) a reservoir capable of containing the fluid, wherein the temperature regulator is capable of modulating a temperature of the fluid when the fluid is not contained in the reservoir; and (b) controlling, by a computer system, the temperature regulator to modulate the temperature of the fluid, thereby regulating the temperature of the portion of the article of furniture.

In one aspect, the present disclosure provides a system for regulating a temperature of an article of furniture, comprising: (a) the article of furniture comprising a first portion and a second portion, wherein each of the first and second portions is configured to hold a fluid; (b) a common temperature controller configured to modulate a temperature of the fluid, wherein the common temperature controller comprises (i) a first channel in fluid communication with the first portion of the article of furniture, and (ii) a second channel in fluid communication with the second portion of the article of furniture, wherein the first and second channels are configured to hold the fluid; and (c) a processor operatively coupled to the common temperature controller, programmed to control the common temperature controller to modulate the temperature of the fluid, thereby to independently regulate a first temperature of the first portion of the article of furniture and a second temperature of the second portion of the article of furniture.

In one aspect, the present disclosure provides a method for regulating a temperature of an article of furniture, comprising: (a) providing a common temperature controller configured to modulate a temperature of a fluid, wherein the common temperature controller comprises (i) a first channel in fluid communication with a first portion of the article of furniture, and (ii) a second channel in fluid communication with a second portion of the article of furniture, wherein the first and second portions of the article of furniture are configured to hold a fluid, and wherein the first and second channels are configured to hold the fluid; and (b) controlling the common temperature controller to modulate the temperature of the fluid, thereby independently regulating a first temperature of the first portion of the article of furniture and a second temperature of the second portion of the article of furniture.

Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG. 1** is a diagram of a bed device, according to one embodiment.
**FIG. 2** illustrates an example of a bed device, according to one embodiment.
**FIG. 3** illustrates an example of layers comprising a bed pad device, according to one embodiment.
**FIG. 4A** illustrates a user sensor placed on a sensor strip, according to one embodiment.
**FIG. 4B** is the sensor strip, according to one embodiment.
**FIG. 4C** is a flowchart of a process to manufacture the body of the sensor strip, according to one embodiment.
**FIG. 4D** is a flowchart of a process to manufacture the tail part of the sensor strip, according to one embodiment.
**FIGs. 5A, 5B****,** **5C,** and **5D** show different configurations of a sensor strip, to fit different size mattresses, according to one embodiment.
**FIG. 6A** illustrates the division of the heating coil into zones and subzones, according to one embodiment.
**FIGs. 6B** and **6C** illustrate the independent control of the different subzones, according to one embodiment.
**FIGs. 7A****,** **7B****,** and **7C** are a flowchart of the process for deciding when to heat or cool the bed device, according to various embodiments.
**FIG. 8** is a flowchart of the process for recommending a bed time to a user, according to one embodiment.
**FIG. 9** is a flowchart of the process for activating the user's alarm, according to one embodiment.
**FIG. 10** is a flowchart of the process for turning off an appliance, according to one embodiment.
**FIG. 11** is a diagram of a system capable of automating the control of the home appliances, according to one embodiment.
**FIG. 12** is an illustration of the system capable of controlling an appliance and a home, according to one embodiment.
**FIG. 13** is a flowchart of the process for controlling an appliance, according to one embodiment.
**FIG. 14** is a flowchart of the process for controlling an appliance, according to another embodiment.
**FIG. 15** is a diagram of a system for monitoring biological signals associated with a user, and providing notifications or alarms, according to one embodiment.
**FIG. 16** is a flowchart of a process for generating a notification based on a history of biological signals associated with a user, according to one embodiment.
**FIG. 17** is a flowchart of a process for generating a comparison between a biological signal associated with a user and a target biological signal, according to one embodiment.
**FIG. 18** is a flowchart of a process for detecting the onset of a disease, according to one embodiment.
**FIG. 19** is a diagrammatic representation of a machine in the example form of a computer system within which a set of instructions, for causing the machine to perform any one or more of the methodologies or modules discussed herein, may be executed.
**FIG. 20** is an example of adjusting a temperature of a bed.
**FIG. 21** is an example of a block diagram for adjusting a temperature of a bed.
**FIG. 22** is an example of a block diagram for adjusting current provided to thermoelectric elements for adjusting a temperature of a bed.
**FIGs. 23A to 23H** illustrate examples of a system for regulating a temperature of a portion of an article of furniture.
**FIGs. 24A to 24G** illustrate examples of a system for regulating temperatures of a plurality of portions of an article of furniture.
**FIGs. 25** and **26** illustrate examples of a method for regulating a temperature of an article of furniture.
**FIGs. 27** and **28** illustrate different examples of a method for regulating a temperature of an article of furniture.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

The terms "a furniture," "an article of furniture," or "a piece of furniture," as used interchangeably herein, can refer to a bed, crib, bassinet, chair, seat, loveseat, sofa, couch, head rest, stool, ottoman, bench, or any panel intended to be covered with a fabric. The article of furniture can be intended for use in a home, an office, a medical facility (e.g., a hospital), or on a vehicle of transportation such as a car, truck, boat, bus, train or the like. The article of furniture can be intended for use for at least one person (and/or at least one animal, such as a pet). The article of furniture can be intended for use for at least about 1, 2, 3, 4, 5, 6, 7, 8, 9, 10, or more persons. The article of furniture can be intended for use for at most about 10, 9, 8, 7, 6, 5, 4, 3, 2, or 1 person. In an example, the article of furniture may be a bed, and the bed may comprise a plurality of sizes comprising single, single extra-long, double, queen, king, super king, etc. In another example, the article of furniture may be an infant warmer (i.e., a babytherm) to provide heat at one or more temperatures to an infant.

The terms "bed" or "bed device," as used interchangeably herein, may be an article of furniture used for sleep or rest. The bed may comprise a mattress, a mattress pad, and/or a covering (e.g., a blanket). One or more users may sleep or rest on and/or adjacent to a surface of the bed. The surface may be a top surface of the bed. The top surface of the bed may be flat or textured. The bed may be the mattress. The bed may be the mattress pad that covers at least a portion of a surface of a mattress or at least a surface of the mattress. Alternatively or in addition to, the user(s) may sleep under a surface of the bed. The surface may be one or more surfaces of a covering, such as, for example, a blanket. The blanket may be disposed on top of at least a part of the user(s). The bed may be the blanket.

The bed of the present disclosure may assist the user(s) to fall asleep (e.g., assist the user(s) to fall asleep faster) on the bed. The bed of the present disclosure may assist the user(s) to fall asleep for at least about 0.1 hour faster as compared to sleeping on a different bed. The bed of the present disclosure may assist the user(s) to fall asleep for at least about 0.1, 0.2, 0.3, 0.4, 0.5, 0.6, 0.7, 0.8, 0.9, 1, 1.5, 2, or more hours as compared to sleeping on a different bed. The bed of the present disclosure may assist the user(s) to fall asleep for at most about 2, 1.5, 1, 0.9, 0.8, 0.7, 0.6, 0.5, 0.4, 0.3, 0.2, 0.1, or less hours as compared to sleeping on a different bed. The bed of the present disclosure may assist the user(s) to stay asleep longer (e.g., for a non-determined period of time or a predetermined period of time) on the bed. The bed of the present disclosure may assistant the user(s) to stay asleep for at least about 0.5 hour as compared to sleeping on a different bed. The bed of the present disclosure may assist the user(s) to stay asleep for at least about 0.1, 0.2, 0.3, 0.4, 0.5, 1, 1,5, 2, 2.5, 3, 3.5, 4, 4.5, 5, or more hours as compared to sleeping on a different bed. The bed of the present disclosure may assist the user(s) to stay asleep for at least about 5, 4.5, 4, 3.5, 3, 2.5, 2, 1.5, 1, 0.5, 0.4, 0.3, 0.2, 0.1, or less hours as compared to sleeping on a different bed. The bed may shorten or extend a sleep phase of the user(s) while sleeping or resting on the bed. The bed may assist the user(s) to enter or exit a sleep phase while awake, sleeping, or resting on the bed. The bed may improve quality of sleep of the user(s).

The bed of the present disclosure may assist the user to wake up from sleeping. The bed of the present disclosure may use one or more alarm mechanisms to wake up the user from sleeping. The alarm mechanism(s) may include a personal device (e.g., a mobile device, a computer, a digital alarm clock, etc.) or the bed itself (e.g., mattress, bed sheet, blanket, pillow, mattress frame, etc.). In some cases, the bed may regulate (or adjust) one or more settings of the bed. Such setting(s) of the bed may comprise temperature, position relative to a rest position of the bed, movement (e.g., vibration, translation, rotation, etc.). In an example, the bed may be capable of increasing and/or decreasing a temperature of a portion of the bed (e.g., a portion of a surface of the bed) to wake up the user that is sleeping on the portion of the bed. Such bed may be referred to as a thermal alarm. In some cases, the bed may be configured to wake up the user at a predetermined wake-up time that is input by the user prior to sleeping. In some cases, the bed may not receive data indicative of a predetermined wake-up time from the user. In some cases, the bed may be configured to automatically determine a wake-up time (e.g., an optimal wake-up time) to wake up the user based at least in part on one or more detected biological signals of the user of the bed. The bed may be able to use one or more sensors to detect a movement, presence, and/or absence of the user of the bed, thereby to determine whether the user is awake and/or out of the bed. Additionally, the bed may be configured to automatically diminish and/or turn off the alarm mechanism(s) when it is determined, at least in part by the one or more sensors, that the user is awake and/or out of the bed.

A temperature of the article of furniture (e.g., the bed, such as the mattress, the mattress pad, or the blanket) may be controlled (e.g., increasing, decreasing, or maintaining the temperature of the bed). A temperature of at least a portion of the article of furniture may be controlled. The temperature of the article of furniture may be adjustable or maintained prior to, during, or subsequent to a use (e.g., sleeping or resting for a period of time) by the user(s). In an example, the bed may be pre-warmed (e.g., automatically or per user preference) prior to the use by the user(s). In some cases, temperatures or two or more portions of the article of furniture (e.g., the bed) may be controlled separately or in sync.

The terms "biological signal" and "bio signal" can be used interchangeably. Examples of the biological signal can include a heart signal (e.g., heart rate or sound), a respiration (breathing) signal (e.g., respiration rate or sound), a motion, a temperature, a movement, perspiration, sound, neural activity, etc. The article of furniture (e.g., the bed) may be capable of detecting one or more biological signals of the user(s). The article of furniture may be capable of adjusting a property of the article of furniture (e.g., temperature or movement of the article of furniture, such as vibration, geometric configuration, etc.) to control (e.g., increase, decrease, or maintain) the biological signal(s) of the user(s) of the article of furniture.

The term "sleep phase," as used herein, can refer to a light sleep, deep sleep, or rapid eye movement ("REM") sleep. There can be two major stages of sleep: a non-REM sleep and a REM sleep. A person can experience a non-REM sleep first, followed by a shorter period of REM sleep. In some cases, the person can experience a continued cycle of the non-REM sleep and the REM sleep. There may be three stages of non-REM sleep. Each stage can last from 5 to 15 minutes. The person can go through all three stages before reaching REM sleep. In stage one, the person's eyes may be closed, but the person may be easily woken up. This stage may last for 5 to 10 minutes. This stage may be considered as a light sleep. In stage two, the person may be in light sleep. The person's heart rate may slow and the person's body temperature may drop. The person's body may be getting ready for deep sleep. This stage may also be considered as a light sleep. Stage three may be a deep sleep stage. The person may be harder to rouse during this stage, and if the person was woken up, the person would feel disoriented for a few minutes. During the deep stage of the non-REM sleep, the body may repair and regrow tissues, build bone and muscle, and strengthen the immune system. The REM sleep can happen 90 minutes after a person falls asleep. In some cases, the person may have dreams during the REM sleep. An initial period of the REM sleep may typically last 10 minutes. Any latter period of the REM sleep may get longer, and the final period of the REM sleep may last up to about an hour. The person's heart rate and respiration may quicken during the REM sleep (e.g., during the final period of the REM sleep). The person may have intense dreams during the REM sleep, since the brain is more active. The REM sleep may affect learning of certain mental skills.

A "sleep pattern", as used herein, can indicate a recurrence or change in (i) one or more biological signals and/or (i) one or more sleep phases of the user of the bed. The sleep pattern may be described over a period of time (e.g., 0.5 hour, 1 hour, 2 hour, 3 hour, 4 hour, 5 hour, 6 hour, etc.), along with a count of the biological signal(s) or the sleep phase(s). The sleep pattern may comprise a preferred setting of the biological signal(s) or sleep phase(s) of the user. The preferred setting of the biological signal(s) may comprise a type of the biological signal(s), along with a preferred value or range of values of the biological signal(s) (e.g., a preferred body temperature or range of body temperature of the user). The preferred setting of the sleep phase(s) may comprise a type of the sleep phase(s), along with a preferred value or range of values of the sleep phase(s).

The bed may identify a sleep disorder of the user(s). Examples of the sleep disorder may include dyssomnias, such as insomnia, primary hypersomnia (e.g., narcolepsy, idiopathic hypersomnia, recurrent hypersomnia, posttraumatic hypersomnia, menstrual-related hypersomnia), sleep disordered breathing (e.g., sleep apnea, snoring, upper airway resistance syndrome), circadian rhythm sleep disorders (e.g., delayed sleep phase disorder, advanced sleep phase disorder, non-24-hour sleep-wake disorder), parasomnias (e.g., bedwetting, bruxism, catathrenia, exploding head syndrome, sleep terror, REM sleep behavior disorder, sleep talking), jet lag, restless legs syndrome, etc. Methods and systems for monitoring a person's sleep patterns on a bed and detecting the person's sleep disorder (e.g., snoring, sleep apnea, etc.) are described in U.S. Patent Publication No. 2017/0135632 ("DETECTING SLEEPING DISORDERS"), which is entirely incorporated herein by reference.

The article of furniture (e.g., the bed) may use one or more sensors and/or one or more computer systems to identify the biological signal(s) and/or the sleep order of the user(s). The sensor(s) may or may not be a part of the article of furniture. The sensor(s) may be a part of a space (e.g., room) surrounding the article of furniture. The sensor(s) may be worn by the user(s). The sensor(s) may be used to detect a property (e.g., temperature, movement, etc.) of the article of furniture.

The term "module" refers broadly to software, hardware, or firmware components (or any combination thereof). Modules are typically functional components that can generate useful data or another output using specified input(s). A module may or may not be self-contained. An application program (also called an "application") may include one or more modules, or a module may include one or more application programs.

The term "on top of" can mean that the two objects, where the first object is "on top of" the second object, can be rotated so that the first object is above the second object relative to the ground. The two objects can be in direct or indirect contact, or may not be in contact at all.

### Systems and methods for regulating a temperature of an article of furniture

The present disclosure provides systems for regulating a temperature of an article of furniture, and methods of use thereof. In some embodiments, the system may comprise an article of furniture. The article of furniture may be operatively coupled to at least one sensor (e.g., at least one user sensor) configured to detect one or more biological signals of at least one user of the article of furniture (e.g., while the at least one user is on the article of furniture). The one or more biological signals that are detected may be used for regulating the temperature of the article of furniture. In some cases, the at least one sensor may be a part of the article of furniture. Alternatively, the at least one sensor may not be a part of the article of furniture.

In some embodiments, the system may comprise a temperature control device (or a temperature controller, as used interchangeably herein) configured to regulate the temperature of the article of furniture. The temperature control device may be operatively coupled to the article of furniture. The temperature control device may not be coupled to the article of furniture. Alternatively, at least a portion of the temperature control device may be coupled to the article of furniture (e.g., may be disposed above or beneath the article of furniture, may be disposed within the article of furniture, etc.). In some cases, the temperature control device may comprise a temperature regulator that is capable of modulating a temperature of at least a portion of the temperature control device, such that the temperature control device can direct a transfer of heat (i) from the temperature control device and towards at least a portion of the article of furniture or (ii) from the at least the portion of the article of furniture and towards the temperature control device. In some cases, the temperature regulator may be capable of modulating a temperature of a fluid that is in thermal communication with the temperature control device and the at least a portion of the article of furniture. Upon temperature modulation, such fluid may direct a transfer of heat (i) from the temperature control device and towards the at least the portion of the article of furniture or (ii) from the at least the portion of the article of furniture and towards the temperature control device.

In some embodiments, the system may comprise a processor. The processor may be operatively coupled to the at least one sensor (e.g., or one or more components within the at least one sensor), the temperature control device (e.g., or one or more components within the temperature control device), or both. The processor may be configured to direct (e.g., automatically direct) regulation of the temperature of the at least a portion of the article of furniture. In some cases, regulation of the temperature of the at least a portion of the article of furniture may effect a user of the article of furniture to, for example, improve a quality of sleep, fall asleep, or wake up.

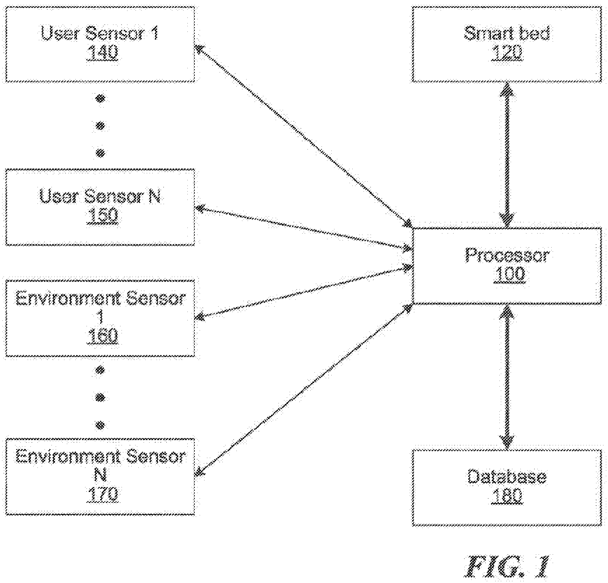

FIG. 1 is a diagram of an example article of furniture, specifically a bed device (e.g., a mattress or a bed pad), according to one embodiment. Any number of sensors (or user sensors) 140, 150 monitor the bio signals associated with a user, such as the heart rate, the respiration rate, the temperature, motion, or presence, associated with the user. Any number of environment sensors 160, 170 monitor environment properties, such as temperature, sound, light, or humidity. The user sensors 140, 150 and the environment sensors 160, 170 communicate their measurements to the processor 100. The environment sensors 160, 170, measure the properties of the environment that the environment sensors 160, 170 are associated with. In one embodiment, the environment sensors 160, 170 are placed next to the bed. The processor 100 determines, based on the bio signals associated with the user, historical bio signals associated with the user, user-specified preferences, exercise data associated with the user, or the environment properties received, a control signal, and a time to send the control signal to a bed device 120.

According to one embodiment, the processor 100 is connected to a database 180, which stores the biological signals associated with a user or plurality of users of said article of furniture (e.g., the bed device). Additionally, the database 180 can store average biological signals associated with the user, history of biological signals associated with a user, etc. The database 180 can be associated with a user, or the database 180 can be associated with article of furniture (e.g., the bed device).

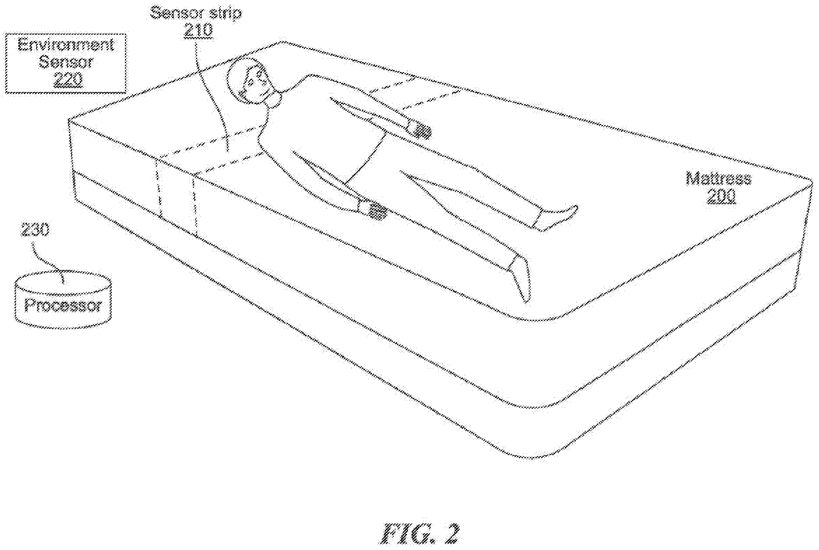

FIG. 2 illustrates an example of the article of furniture (e.g., the bed device) of FIG. 1, according to one embodiment. A sensor (e.g., a sensor strip) 210, associated with a mattress 200 of the bed device 120, monitors bio signals associated with a user sleeping on the mattress 200. The sensor strip 210 can be built into the mattress 200, or can be part of a bed pad device. Alternatively, the sensor 210 can be a part of any other piece of furniture, such as a rocking chair, a couch, an armchair etc. The sensor 210 comprises a temperature sensor, or a piezo sensor. The environment sensor 220 measures environment properties such as temperature, sound, light or humidity. According to one embodiment, the environment sensor 220 is associated with the environment surrounding the mattress 200. The sensor 210 and the environment sensor 220 communicate the measured environment properties to the processor 230. In some embodiments, the processor 230 can be similar to the processor 100 of FIG. 1. A processor 230 can be connected to the sensor 210, or the environment sensor 220 by a computer bus, such as an I2C bus. Also, the processor 230 can be connected to the sensor 210, or the environment sensor 220 by a communication network.

By way of example, the communication network connecting the processor 230 to the sensor 210, or the environment sensor 220 includes one or more networks such as a data network, a wireless network, a telephony network, or any combination thereof. The data network may be any local area network (LAN), metropolitan area network (MAN), wide area network (WAN), a public data network (e.g., the Internet), short range wireless network, or any other suitable packet-switched network, such as a commercially owned, proprietary packet-switched network, e.g., a proprietary cable or fiber-optic network, and the like, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks, code division multiple access (CDMA), wideband code division multiple access (WCDMA), wireless fidelity (WiFi), wireless LAN (WLAN), Bluetooth^{®}, Internet Protocol (IP) data casting, satellite, mobile ad-hoc network (MANET), and the like, or any combination thereof.

The processor 230 is any type of microcontroller, or any processor in a mobile terminal, fixed terminal, or portable terminal including a mobile handset, station, unit, device, multimedia computer, multimedia tablet, Internet node, cloud computer, communicator, desktop computer, laptop computer, notebook computer, netbook computer, tablet computer, personal communication system (PCS) device, personal navigation device, personal digital assistants (PDAs), audio/video player, digital camera/camcorder, positioning device, television receiver, radio broadcast receiver, electronic book device, game device, the accessories and peripherals of these devices, or any combination thereof.

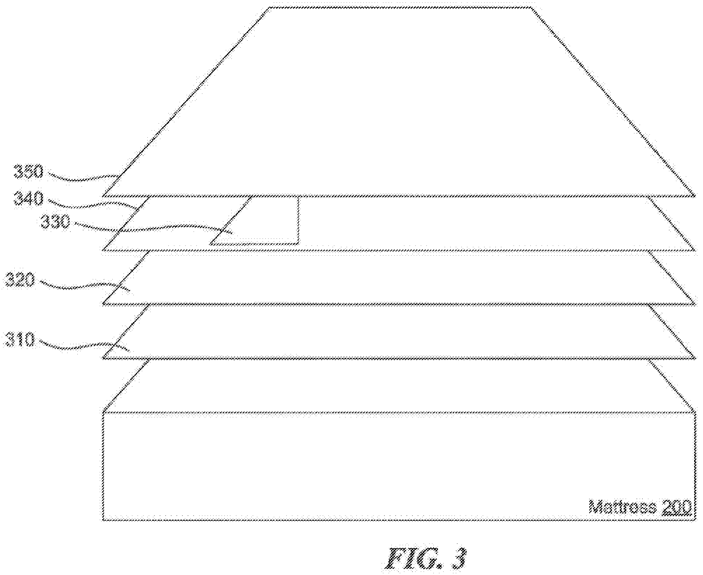

FIG. 3 illustrates an example of at least a portion of the components (e.g., layers) of the article of furniture (e.g., the bed pad device) of FIG. 1, according to one embodiment. In some embodiments, the bed pad device 120 is a pad that can be placed on top of the mattress. Bed pad device 120 comprises a plurality of portions (e.g., a plurality of layers). A top portion (e.g., a top layer) 350 comprises fabric. Another portion (e.g., another layer) 340 comprises a matrix (e.g., a batting) and a sensor (e.g., a sensor strip) 330. A different portion (e.g., a different layer) 320 may be at least a portion of a temperature control device. In an example, the layer 320 comprises coils for cooling or heating the bed device. Alternatively, the layer 320 may comprise a fluid in a fluid flow channel for cooling or heating the article of furniture. A layer 310 comprises waterproof material.

According to another embodiment, the layer 320 comprises a material (e.g., solid, semi-solid, gel, liquid, or a combination thereof) that can be heated or cooled from about 0.5 degrees Celsius (°C) to about 50°C. In some cases, the material may be heated or cooled from about 0.5 °C to about 50 °C without changing the materials properties such as the state of matter. Alternatively, the materials properties may change during heating or cooling, and such materials properties may be reversible. In some cases, the material can be cooled from about 10 °C to about 50 °C without changing the materials properties such as the state of matter. An example of such materials can be air, water, argon, a synthetic material such as polymers, carbon nanotubes, etc. According to one embodiment, the layer 320 is connected to an external thermal regulator which heats or cools the material, based on the signal received from the processor 230. The material of the layer 320 may be heated or cooled to a temperature in a range between about 10°C to about 50°C. A temperature of such material that may be adjusted by at least about 0.1°C, 0.2°C, 0.3°C, 0.4°C, 0.5°C, 0.6°C, 0.7°C, 0.8°C, 0.9°C, 1°C, 2°C, 3°C, 4°C, 5°C, 6°C, 7°C, 8°C, 9°C, 10°C, 11°C, 12°C, 13°C, 14°C, 15°C, 20°C, 25°C, 30°C, 35°C, 40°C, 45°C, 50°C, or more. The temperature of such material that may be adjusted by at most about 50°C, 49°C, 48°C, 47°C, 46°C, 45°C, 40°C, 35°C, 30°C, 25°C, 20°C, 15°C, 14°C, 13°C, 12°C, 11°C, 10°C, 9°C, 8°C, 7°C, 6°C, 5°C, 4°C, 3°C, 2°C, 1°C, 0.9°C, 0.8°C, 0.7°C, 0.6°C, 0.5°C, 0.4°C, 0.3°C, 0.2°C, 0.1°C, or less. The external thermal regulator may be a part of a temperature control device that is operatively coupled to the article of furniture.

According to another embodiment, the layer 320 comprising the material is integrated into the mattress, the bed sheets, the bed cover, the bed frame, etc. The layer 320 comprising the material can also be integrated with any piece of furniture.

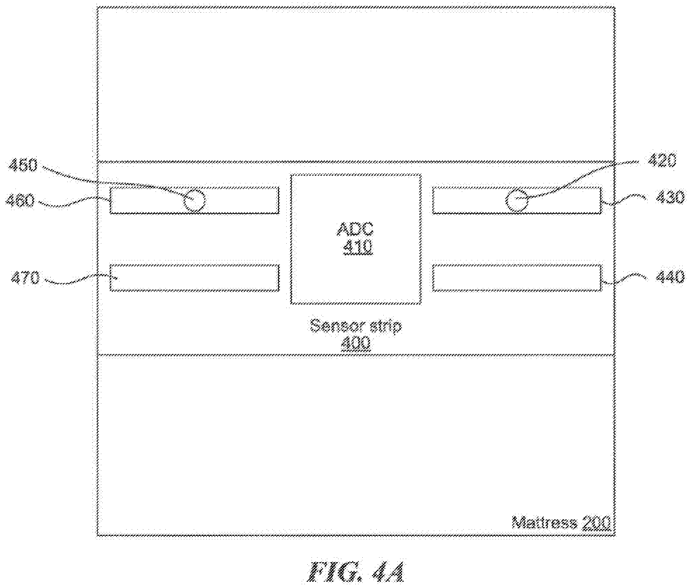

FIG. 4A illustrates a user sensor 420, 440, 450, 470 placed on a sensor 400, according to one embodiment. In some embodiments, the user sensors 420, 440, 450, 470 can be similar to or part of the sensor 210 of FIG. 2. Sensors 470 and 440 comprise a piezo sensor, which can measure a bio signal associated with a user, such as the heart rate and the respiration rate. Sensors 450 and 420 comprise a temperature sensor. According to one embodiment, sensors 450, and 470 measure the bio signals associated with one user, while sensors 420, 440 measure the bio signals associated with another user. Analog-to-digital converter 410 converts the analog sensor signals into digital signals to be communicated to a processor. Computer bus 430 and 460, such as the I2C bus, communicates the digitized bio signals to a processor.

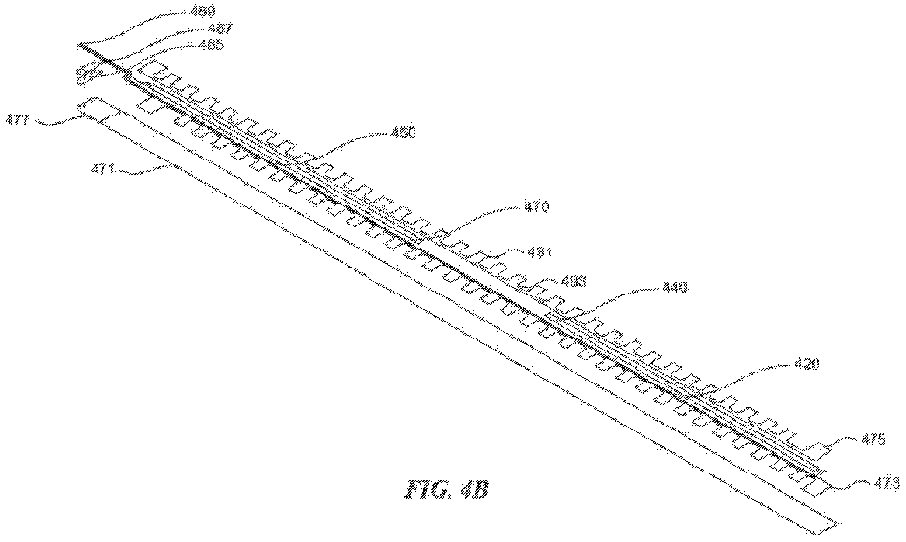

FIG. 4B is the sensor (e.g., sensor strip) 400, according to one embodiment. The sensor 400 comprises several layers, such as a fabric layer 471, a foam layer 473, 475, a piezo sensor 470, 440, a stiffener (e.g., a polymer stiffener, such as a polycarbonate stiffener) 485, a stiffener foam 487, and a temperature sensor 450, 420. Region 477 of the fabric layer 471 is the tail region of the sensor 400. Wire leads 489 associated with piezo sensor 470, 440, and temperature sensor 450, 420 are placed on top of the tail region 477. The fabric layer 471 includes two short edges and two long edges. The length of the short edge varies from 40-70 mm. The fabric layer 471 has at least one coated surface. The foam layer 473, 475 also has two short edges and two long edges. One of the long edges includes multiple protrusions 491, and multiple gaps 493, between the multiple protrusions 491.

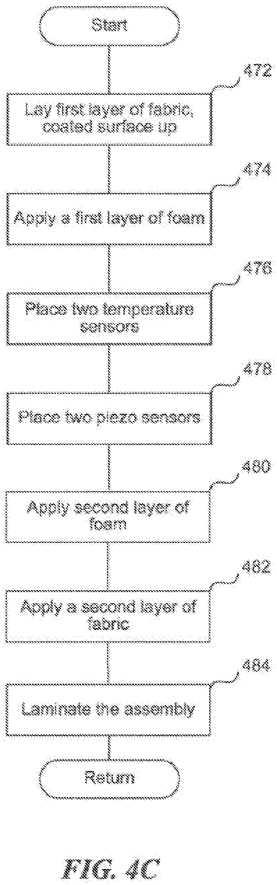

FIG. 4C is a flowchart of a process to manufacture the body of the sensor 400, according to one embodiment. In step 472, the fabric layer 471 is laid out with the coated surface pointing up. In step 474, a first foam layer is applied to the fabric layer 471. In one embodiment, the first foam layer 473 is centered on the fabric layer 471, with a margin of 10 mm from the first short edge and a margin of 5 mm from the long edges. The margin to the second short edge of the fabric layer 471 is greater than the margin to the first short edge. In one embodiment, the margin to the second short edge is at least twice as big than the margin to the first short edge. The margin to the second short edge of the fabric layer 471 is considered a tail part of the sensor 400, comprising the tail region 477 of the fabric layer 471. In step 476, two temperature sensors 450, 420 are placed on the first foam layer 473. In one embodiment, the temperature sensors are placed 17 mm from a long edge of the fabric layer 471. In step 478, two piezo sensors 470, 440 are placed on the first foam layer 473. In one embodiment, the piezo sensors are centered on the fabric layer 471. In step 480, a second foam layer 475 is applied on top of the piezo sensors. In one embodiment, the second foam layer 475 is centered on the fabric layer 471, with a margin of 10 mm from the short edges, and 5 mm from the long edges. Further, the second foam layer 475 is placed as a mirror image of the first foam layer 473, and is interlaced with the first foam layer 473. In step 482, a second fabric layer is applied on top of the second foam layer 475. In step 484, the whole assembly, comprising all the layers, is laminated.

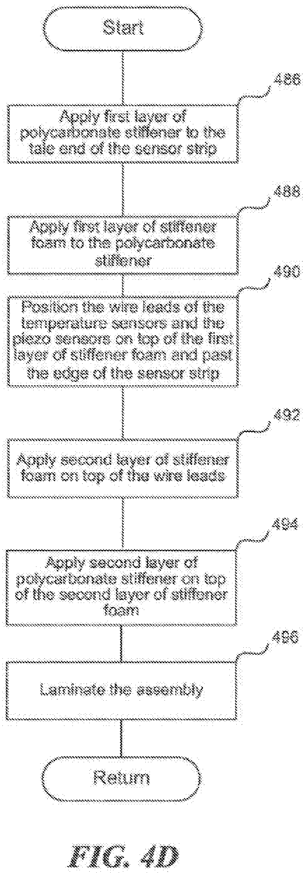

FIG. 4D is a flowchart of a process to manufacture the tail part of the sensor (e.g., the sensor strip) 400, according to one embodiment. In step 486, first polycarbonate stiffener layer 485 is placed on top of the tail region 477 of the fabric layer 471. In one embodiment, the dimensions of the polycarbonate stiffener layer 485 are 40-70 mm by 5-25 mm. The 40-70 mm edge matches the length of the 40-70 mm edge of the sensor 400. In step 488, the first stiffener foam layer 487 is applied on top of the polycarbonate stiffener layer 485. In step 490, the wire leads 489 of the piezo sensors 470, 440, and the wire leads 489 of the temperature sensors 450, 420 are placed on top of the first stiffener foam layer 487, and past the tail region 477 of the fabric layer 471. In step 492, the second stiffener foam layer is applied on top of the wire leads 489. The dimensions of the second stiffener foam layer are identical to the first stiffener foam layer 487. In step 494, the second polycarbonate stiffener layer is applied on top of the second stiffener foam layer. The dimensions of the second polycarbonate stiffener layer are identical to the dimensions of the first polycarbonate stiffener layer 485. In step 496, the whole tail part assembly is laminated.

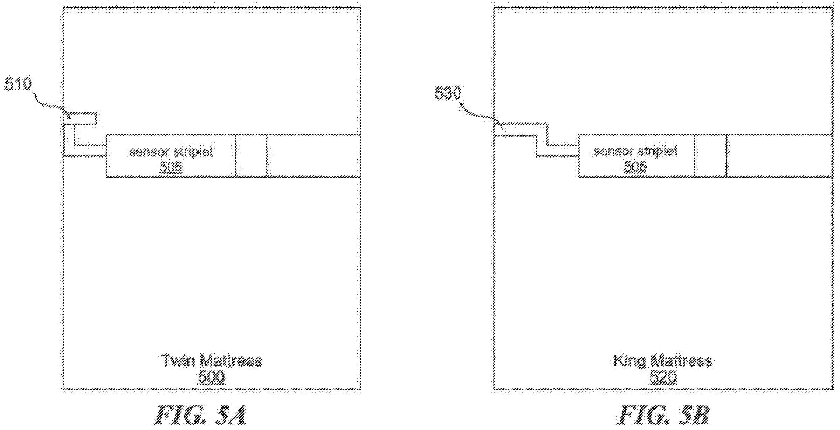

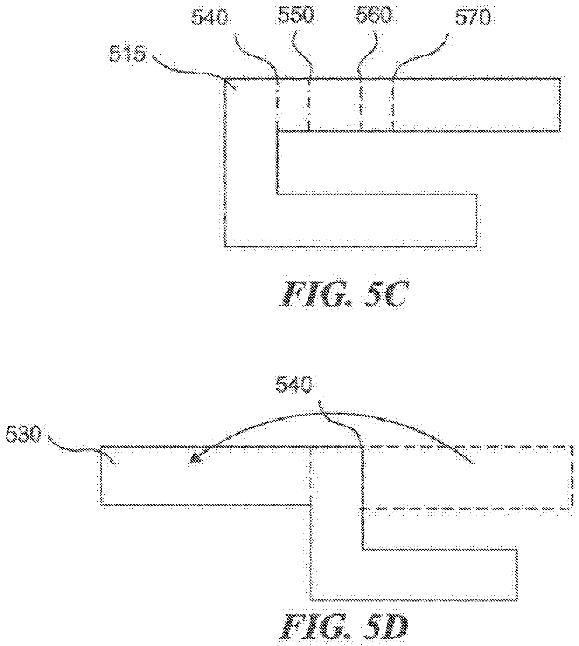

FIGs. 5A and 5B show different configurations of the sensor (e.g., the sensor strip), to fit different size beds (e.g., different size mattresses), according to one embodiment. FIGs. 5C and 5D show how such different configurations of the sensor can be achieved. Specifically, sensor 400 comprises a computer bus 510, 530, and a sensor striplet 505. The computer bus 510, 530 can be bent at predetermined locations 540, 550, 560, 570. Bending the computer bus 515 at location 540 produces the maximum total length of the computer bus 530. Computer bus 530 combined with a sensor striplet 505, fits a king size mattress 520. Bending the computer bus 515 at location 570 produces the smallest total length of the computer bus, 510. Computer bus 510 combined with a sensor striplet 505, fits a twin size mattress 500. Bending the computer bus 515 at location 560, enables the sensor 400 to fit a full-size bed. Bending the computer bus 515 at location 550 enables the sensor 400 to fit a queen-size bed. In some embodiments, twin mattress 500, or king mattress 520 can be similar to the mattress 200 of FIG. 2.

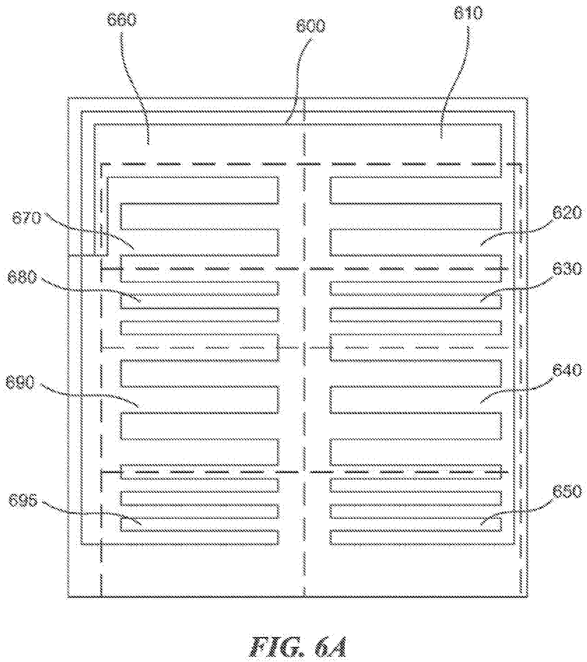

FIG. 6A illustrates the division of the heating coil 600 into zones and subzones, according to one embodiment. Specifically, the heating coil 600 is divided into two zones 660 and 610, each corresponding to one user of the bed. Each zone 660 and 610 can be heated or cooled independently of the other zone in response to the user's needs. To achieve independent heating of the two zones 660 and 610, the power supply associated with the heating coil 600 is divided into two zones, each power supply zone corresponding to a single user zone 660, 610. Further, each zone 660 and 610 is further subdivided into subzones. Zone 660 is divided into subzones 670, 680, 690, and 695. Zone 610 is divided into subzones 620, 630, 640, and 650. The distribution of coils in each subzone is configured so that the subzone is uniformly heated. However, the subzones may differ among themselves in the density of coils. For example, the data associated with the user subzone 670 has lower density of coils than subzone 680. This will result in subzone 670 having lower temperature than subzone 680, when the coils are heated. Similarly, when the coils are used for cooling, subzones 670 will have higher temperature than subzone 680. According to one embodiment, subzones 680 and 630 with highest coil density correspond to the user's lower back; and subzones 695 and 650 with highest coil density correspond to user's feet. According to one embodiment, even if the users switch sides of the bed, the system will correctly identify which user is sleeping in which zone by identifying the user based on any of the following signals alone, or in combination: heart rate, respiration rate, body motion, or body temperature associated with the user.

In another embodiment, the power supply associated with the heating coil 600 is divided into a plurality of zones, each power supply zone corresponding to a subzone 620, 630, 640, 650, 670, 680, 690, 695. The user can control the temperature of each subzone 620, 630, 640, 650, 670, 680, 690, 695 independently. Further, each user can independently specify the temperature preferences for each of the subzones. Even if the users switch sides of the bed, the system will correctly identify the user, and the preferences associated with the user by identifying the user based on any of the following signals alone, or in combination: heart rate, respiration rate, body motion, or body temperature associated with the user.

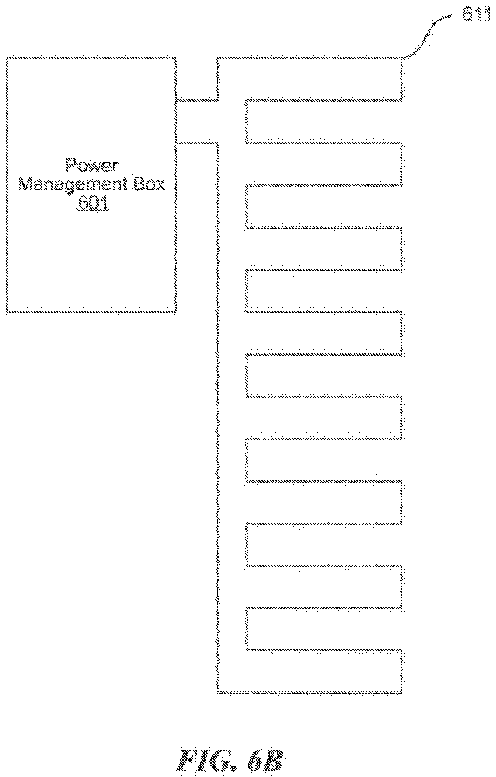

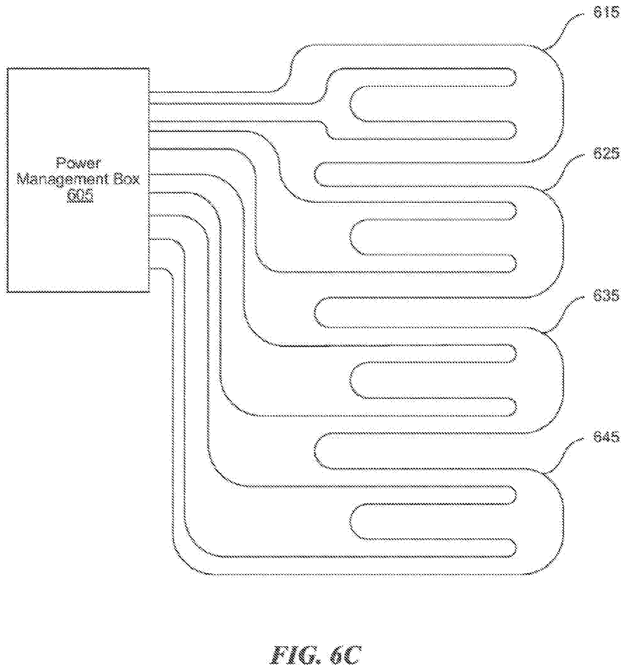

FIGs. 6B and 6C illustrate the independent control of the different subzones in each zone 610, 660, according to one embodiment. Set of uniform coils 611, connected to power management box 601, uniformly heats or cools the bed. Another set of coils, targeting specific areas of the body such as the neck, the back, the legs, or the feet, is layered on top of the uniform coils 611. Subzone 615 heats or cools the neck. Subzone 625 heats or cools the back. Subzone 635 heats or cools the legs, and subzone 645 heats or cools the feet. Power is distributed to the coils via duty cycling of the power supply 605. Contiguous sets of coils can be heated or cooled at different levels by assigning the power supply duty cycle to each set of coils. The user can control the temperature of each subzone independently.

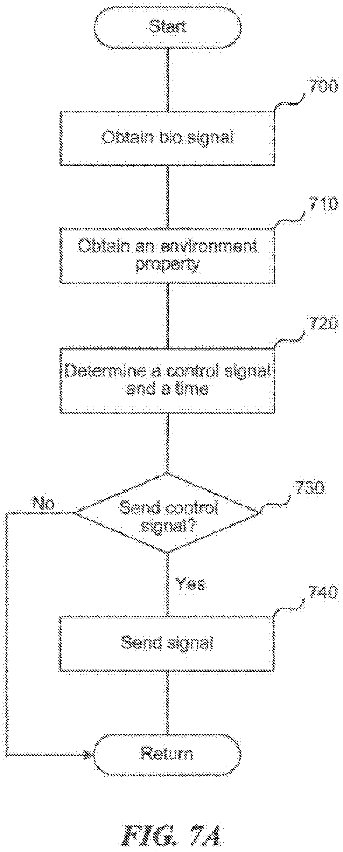

FIG. 7A is a flowchart of the process for deciding when to heat or cool the bed device, according to one embodiment. At block 700, the process obtains a biological signal associated with a user, such as presence in bed, motion, respiration rate, heart rate, or a temperature. The process obtains the biological signal from a sensor associated with a user. Further, at block 710, the process obtains environment property, such as the amount of ambient light and the bed temperature. The process obtains environment property from and environment sensor associated with the bed device. If the user is in bed, the bed temperature is low, and the ambient light is low, the process sends a control signal to the bed device. The control signal comprises an instruction to heat the bed device to the average nightly temperature associated with the user. According to another embodiment, the control signal comprises an instruction to heat the bed device to a user-specified temperature. Similarly, if the user is in bed, the bed temperature is high, and the ambient light is low, the process sends a control signal to the bed device to cool the bed device to the average nightly temperature associated with the user. According to another embodiment, the control signal comprises an instruction to cool the bed device to a user-specified temperature.

In another embodiment, in addition to obtaining the biological signal associated with the user, and the environment property, the process obtains a history of biological signals associated with the user. The history of biological signals can be stored in a database associated with the bed device, or in a database associated with a user. The history of biological signals comprises the average bedtime the user went to sleep for each day of the week; that is, the history of biological signals comprises the average bedtime associated with the user on Monday, the average bedtime associated with the user on Tuesday, etc. For a given day of the week, the process determines the average bedtime associated with the user for that day of the week, and sends the control signal to the bed device, allowing enough time for the bed to reach the desired temperature, before the average bedtime associated with the user. The control signal comprises an instruction to heat, or cool the bed to a desired temperature. The desired temperature may be automatically determined, such as by averaging the historical nightly temperature associated with a user, or the desired temperature may be specified by the user.

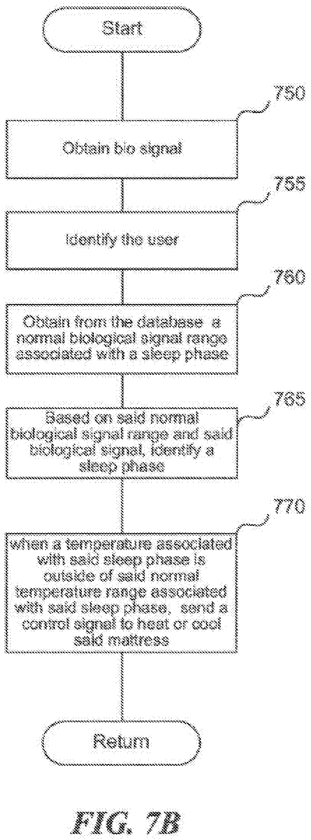

FIG. 7B is a flowchart of the process for cooling or heating a bed device, according to another embodiment. In step 750, processor 230 obtains the biological signal associated with the user, wherein the biological signal comprises a respiration rate associated with the user, a heart rate associated with the user, a motion associated with the user, or a temperature associated with the user. In step 755, the processor 230 identifies the user based on at least one of: the heart rate associated with the user, the respiration rate associated with the user, the motion associated with the user, or the temperature associated with the user. In step 760, based on the user identification, the processor 230 obtains from the database 180 a normal biological signal range associated with a sleep phase in a plurality of sleep phases associated with the user, wherein the normal biological signal range comprises a normal temperature range associated with the user. In step 765, based on the normal biological signal range and the biological signal, the processor 230 identifies a sleep phase in a plurality of sleep phases associated with the user. The plurality of sleep phases includes the sleep phase comprising a wakefulness phase, a light sleep phase, a deep sleep phase, or a rapid eye movement sleep phase. In step 770, when the temperature associated with the sleep phase is outside of the normal temperature range associated with the sleep phase, the processor 230 sends a control signal to a temperature control device coupled to the mattress, the control signal comprising an instruction to heat or cool the mattress to a temperature within the normal temperature range.

According to one embodiment, the processor 230 obtains the biological signal associated with a user from the sensor 210 coupled to the mattress, where the sensor 210 measures the biological signal associated with the user. In another embodiment, the processor 230 obtains the biological signal associated with the user from a wearable device coupled to the user, which measures the users biological signals, such as a fitbit bracelet. The processor 230 can also store the biological signals into the database 180.

According to another embodiment, the processor 230 determines a current time. The processor 230 identifies the user based on at least one of: the heart rate associated with the user, the respiration rate associated with the user, the motion associated with the user, or the temperature associated with the user. Based on the user identification, the processor 230 obtains a wake-up time associated with the user. When the current time is at most 3 hours before the wake-up time, the processor 230 sends the control signal to the temperature control device coupled to the mattress, the control signal comprising an instruction to turn off.

The processor 230 can detect a sleep phase by detecting a slowdown in the heart rate, a drop in the temperature, and a regular respiration rate. The processor 230 can also detect the sleep phase by detecting an end to preceding sleep phase. For example, a healthy user normally cycles between light sleep, deep sleep and REM sleep, in sequence, throughout the night. When the REM sleep phase ends, the light sleep phase begins, followed by a deep sleep phase.

According to another embodiment, the processor 230 obtains perspiration associated with the user from a perspiration sensor built into the sensor 210. When the user is perspiring, the processor sends a control signal to cool the temperature control device by a fraction of a degree Celsius, until the user stops perspiring. The processor 230 maintains the temperature at which the user is not perspiring. The fraction of a degree Celsius can be 1/10, 1/5, 1/4, 1/2, 1, etc. According to another embodiment, based on the total amount of perspiration from the user during the sleep, the processor 230 recommends an amount of liquid, such as water or electrolytes, that the user should consume upon waking up.

According to another embodiment, the processor 230 sends a control signal to cool or heat the temperature control device of a fraction of a degree Celsius, and monitors the quality of users sleep. For example, the processor 230 monitors if the user goes through the sleep cycles in order, and if the sleep cycles last a normal amount of time. Once the user sleep cycles becomes irregular, or do not last a normal amount of time, the processor records the last temperature, at which the user slept soundly. The last temperature at which the user slept soundly is the limit of the comfortable temperature range associated with that user. The limit can be a high temperature limit, or a low temperature limit. The fraction of a degree Celsius can be 1/10, 1/5, 1/4, 1/2, 1, etc. The processor 230 stores the comfortable temperature range associated with the user, comprising a high temperature limit, and a low temperature limit, and heats or cools the bed to temperature within the comfortable temperature range.

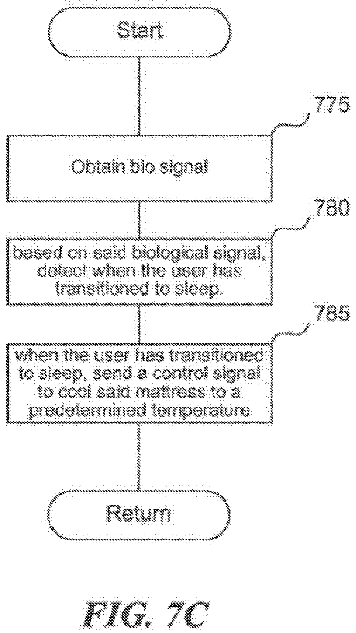

FIG. 7C is a flowchart of the process for cooling or heating a bed device, according to yet another embodiment. In step 775, the processor 230 obtains the biological signal associated with the user, wherein the biological signal comprises a respiration rate associated with the user, a heart rate associated with the user, a motion associated with the user, or a temperature associated with the user. In step 780, based on the biological signal, the processor 230 detects when the user has transitioned to sleep. The processor 230 detects transition to sleep by detecting a slowdown in the heart rate, a regular heart rate, a drop in the temperature, and/or a regular respiration rate. In step 785, when the user has transitioned to sleep, the processor 230 sends a control signal to a temperature control device coupled to the mattress, the control signal comprising an instruction to cool the mattress to a predetermined temperature. The predetermined temperature can be the average nightly temperature associated with the user, the predetermined temperature can be in the range 27 to 35°C, or the temperature can be user-specified. The biological signal can be measured by the sensor 210, or by any other sensing device, such as a wearable sensor, e.g. a fitbit bracelet.

According to another embodiment, the processor 230 obtains an ambient temperature surrounding the user. The environment sensor 220 can supply the processor 230 with the ambient temperature. When the ambient temperature is outside of a 35°C to 36°C range, the processor 230 sends the control signal to the temperature control device coupled to the mattress, the control signal comprising an instruction to adjust the mattress to a temperature within 27°C to 35°C range, a user-specified temperature, or a user-related temperature. The user-related temperature may be a set point pre-determined by using historical data of the user. The historical data of the user may comprise a plurality of bodily temperatures of the user over a set time period (e.g., over a period of at least 1, 2, 3, 4, 5, 6, 7, 8, 9, 10, or more days). The historical data of the user may comprise an average of the plurality of bodily temperatures of the user over the set time period.

According to another embodiment, the processor 230 identifies the user based on at least one of: the heart rate associated with the user, the respiration rate associated with the user, the temperature associated with the user, or the motion associated with the user. Based on the user identification, the processor 230 determines an average bedtime associated with the user. The average bedtime can be the same for every day of the week, or can comprise an average Monday bedtime, an average Tuesday bedtime, an average Wednesday bedtime, an average Thursday bedtime, an average Friday bedtime, an average Saturday bedtime, or an average Sunday bedtime. At the average bedtime associated with the user, the processor 230 sends the control signal to the temperature control device coupled to the mattress, wherein the control signal comprises one of an instruction to heat the temperature control device to a temperature in a 27°C to 35°C range, or an instruction to cool the temperature control device to the temperature in the 37°C to 35°C range. The temperature can be a user-specified temperature.

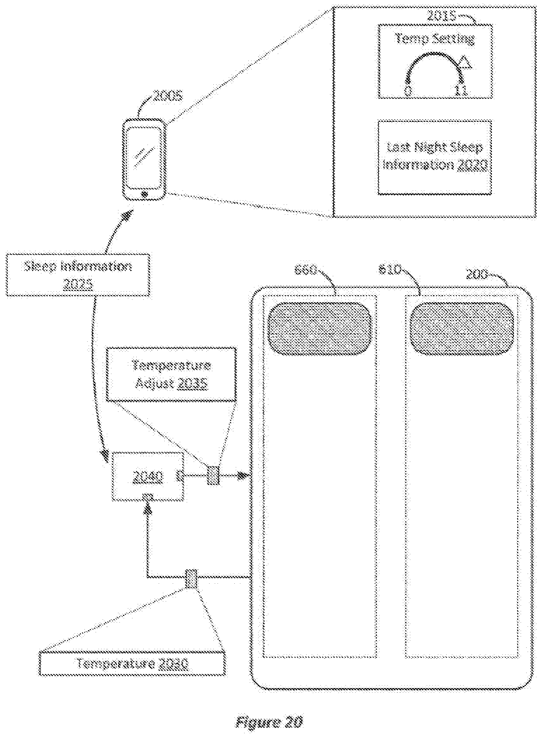

FIG. 20 is another example of adjusting a temperature of a bed. In FIG. 20, a user intending to sleep upon mattress 200 can use computing device 2005 to select a temperature setting 2015 indicating some preference to the cooling and/or heating and view last night sleep information 2020 to obtain and review information related to how the user slept. For example, hub 2040 (e.g., a temperature control device or circuit) can be a device that includes processor 230 that receives the various data disclosed herein such as the temperature, biological signals, and other types of information regarding the user's sleep and generates temperature adjustment 2035 for mattress 200. This can cause the mattress to heat or cool, improving the sleep experience for the user. Temperature sensors can provide back temperature 2030 indicating the current temperature of mattress 200. As the temperature changes, temperature 2030 provided to hub 2040 can change, and if the temperature as indicated by temperature 2030 is too hot (e.g., above a threshold temperature) or too cold (e.g., below a threshold temperature), then hub 2040 can generate temperature adjust 2035 that can allow for mattress 200 to change in temperature in response to the current conditions. Thus, a feedback loop can be implemented in which the temperature of mattress 200 is adjusted many times throughout the night as the user sleeps. As discussed later herein, temperature adjust 2035 can include data or a signal that can be used to adjust the temperature of mattress 200, for example, a signal providing a particular current used to generate a voltage across thermoelectric elements to heat or cool mattress 200 appropriately.

In some cases, mattress 200 can include different zones 660 and 610, as previously discussed. This can allow for two different people (or users) sleeping upon mattress 200 to have different heating or cooling performed throughout the users' sleeping experiences. For example, one person sleeping upon zone 660 (e.g., the left side of the bed) might result in zone 660 to be heated while another person sleeping upon zone 610 (e.g., the right side of the bed) might result in zone 610 to be cooled. Thus, different portions of mattress 200 can be heated and/or cooled differently. In another example, both zones 660 and 610 might be heated, but one zone might be heated to a higher temperature than the other zone. Likewise, both zones 660 and 610 might be cooled, but one zone might be cooled to a lower temperature than the other zone.

Hub 2040 can manage the different sleeping experiences for the different zones 660 and 610. For example, two different computing devices (e.g., mobile phones, tablets, smart watches, laptop computers, etc.) can be communicatively coupled with hub 2040, for example, via a wireless network such as the Institute of Electrical and Electronics Engineers (IEEE) 802.11 wireless local area network (WLAN) standards, Bluetooth, Zigbee, Z-Wave, etc. This can allow for the different computing devices to receive and provide different sleep information 2025, for example, different temperature settings 2015 and different last night sleep information 2020. For example, one computing device can be set or indicated by hub 2040 as being the computing device for a user sleeping upon zone 660. A different computing device can be set or indicated by hub 2040 as being the computing device for a user sleeping upon zone 610. Thus, when data is received from the computing device, it can be determined which device provided that data and the zone associated with that computing device can be operated accordingly (e.g., heated to a particular temperature later at night). When data is to be provided to a computing device (e.g., last night sleep information 2020) then hub 2040 can provide the computing device with the information related to the zone associated with that computing device such that different users sleeping upon the same mattress 200 would receive different information.

A variety of heating or cooling mechanisms other than the coils previously discussed can also be used with the techniques described herein. For example, forced directional gas (e.g., air) cooling (or heating), liquid (e.g., water) cooling (or heating), thermoelectric cooling (or heating), modifications thereof, or combinations thereof can be used for the article of furniture, such as the mattress or the mattress pad of the bed.

Regarding forced directional air cooling, hub 2040 or mattress 200 can include a directional fan or blower that can direct air into mattress 200. For example, one or more channels (e.g., baffles) can be integrated within a layer of mattress 200 (e.g., under a surface that a user sleeps upon) to provide a cavity for air to be pushed through. In some cases, the channel(s) may be a continuous network of channels. The channel(s) can include hollow portions throughout mattress 200 that allow for the propagation or flow of fluid (e.g., liquids or gas). In some cases, the gas may comprise air. The channel(s) can be concentrated upon the areas of mattress 200 where high-temperature areas of the user sleeps, for example, parts of mattress 200 that would be underneath a user's back, shoulders, and hips. Other areas, for example near the user's legs, can include less baffling or no baffling because those areas might not be areas where heating or cooling are as useful. Thus, different portions of mattress 200 can have different concentrations of channel(s) to promote air flow, with some portions even having no channel. Thus, air can be blown into an entry of the channel(s) integrated into mattress 200. In some cases, air can be blow into the entry and out of an exit of the channel(s) such that the air is circulated through mattress 200.

In some cases, if cooling is desired, then air at a temperature colder than what is indicated by temperature 2030 can be provided (e.g., blowing air into the entry of the channel(s) of mattress 200). If heating is desired, then air at a temperature hotter than what is indicated by temperature 2030 can be provided. Thus, temperature adjust 2035 can be generated by hub 2040 to adjust the forced directional air cooling mechanism (e.g., fans, air conditioning units, etc.) to provide the proper temperature.

Regarding liquid cooling, a liquid (e.g., water) can be pumped into one or more channels (e.g., baffles). The temperature of the water can be adjusted in a similar manner as the air blown into the baffle structure. The liquid can be circulated from outside of mattress 200, into the channel(s) of mattress 200, absorb heat, and then pumped back out of mattress 200. This can allow for the liquid to transport the heat outside of mattress 200 and cool off outside of mattress 200. Thus, the liquid can transfer heat away from mattress 200 and circulated outside of the mattress such that the heat is distributed away from mattress 200. This can result in a cooling (e.g., reduce the temperature) of mattress 200.

Thermoelectric temperature regulation (e.g., heating and/or cooling) can be implemented using an electric-based system (e.g., by a thermoelectric engine). The thermoelectric engine can be configured to convert electrical energy into a heat flux (or a temperature difference), or convert the heat flux into electrical energy. The thermoelectric engine can be a solid-state device.

In some embodiments, the article of furniture (e.g., the bed) can comprise the thermoelectric engine in the article of furniture (e.g., in the mattress or mattress pad) as a mechanism to regulate temperature of the article of furniture. Such thermoelectric engine may or may not have moving parts (e.g., fans, pumping parts, etc.), and may be quieter than liquid or air cooling. For example, a thermoelectric engine to adjust the temperature of mattress 200 can comprise thermoelectric elements integrated upon printed circuit boards (PCBs) embedded within mattress 200 or a cover upon mattress 200. When current (e.g., electrical current such as the flow of electric charge in amperes) is provided to a thermoelectric element and a voltage is generated across the thermoelectric element, a heat flux can be generated, resulting in a separation of hot temperature and cold temperature across the thermoelectric element. That is, the heat can be separated to one side of the thermoelectric element of the thermoelectric engine, resulting in one side being hotter than the other side (which is cooler than the hotter side). Thus, heat (or energy) can be distributed away from a user sleeping upon mattress 200. The thermoelectric elements can also be concentrated upon the areas of mattress 200 where high-temperature areas of the user sleeps, for example, parts of mattress 200 that would be underneath a user's back, shoulders, and hips similar to the baffling as described above. As a result, other areas, for example near the user's legs, can include fewer thermoelectric elements, or even no thermoelectric elements, because those areas might not be areas where heating or cooling are as useful. Thus, different portions of mattress 200 can have different concentrations of thermoelectric elements to promote heat transfer.

In some embodiments, the temperature regulation mechanism of the article of furniture can comprise a combination of the thermoelectric temperature regulation and the fluid (e.g., liquid or gas). In such a case, the fluid may flow in and out of the channel(s) of the article of furniture, and a thermoelectric temperature regulator may regulate a temperature of the fluid (e.g., water), to thereby regulate a temperature of the article of furniture. The fluid at a regulated temperature may flow through the channel(s) of the article of furniture (e.g., the bed) to (i) maintain a temperature of the user of the article of furniture, (ii) supply heat to the user of the article of furniture, or (iii) take heat from the user (or cool the user) of the article of furniture. The thermoelectric temperature regulator may or may not be part of the article of furniture. The thermoelectric temperature regulator may comprise a thermoelectric engine for regulating the temperature of the fluid, and a reservoir for containing the fluid. The thermoelectric engine may be separated from the reservoir, and in fluid communication with the reservoir. In some cases, the reservoir may regulate the temperature of teh fluid. Alternatively, the reservoir may not be configured to regulate a temperature of the fluid contained in the reservoir. In such a case, the fluid that is contained in the reservoir may not be heated or cooled inside the reservoir. In such a case, the fluid is that outside the reservoir and flowing through or adjacent to the thermoelectric engine (e.g., through one or more channels of the thermoelectric engine, through one or more channels directly adjacent to the thermoelectric engine, etc) may be heated or cooled by the thermoelectric engine.

The thermoelectric engine can comprise at least one thermoelectric unit. The thermoelectric engine can comprise at least about 1, 2, 3, 4, 5, 6, 7, 8, 9, 10, or more thermoelectric units. The thermoelectric engine can comprise at most about 10, 9, 8, 7, 6, 5, 4, 3, 2, or 1 thermoelectric unit. Each thermoelectric unit may be configured to regulate the temperature of the fluid that is flowing through or adjacent to each thermoelectric unit.

For each thermoelectric unit, a first direction of electrical current through the thermoelectric unit may increase a temperature of a side of the thermoelectric unit, thereby to increase a temperature of the fluid (e.g., water) flowing through or adjacent to the side of the thermoelectric unit. A second direction, opposite the first direction, of electrical current through the thermoelectric unit may decrease a temperature of the side of the thermoelectric unit, thereby to decrease a temperature of the fluid flowing through or adjacent to the side of the thermoelectric unit. In some cases, the first direction may be a positive electrical current, and the second direction may be a negative electrical current. In some cases, the first direction may be a negative electrical current, and the second direction may be a positive electrical current.

In some cases, phase change material can also be used to promote the transfer of heat between the user and the article of furniture, such as, for example, between the user and the mattress 200. For example, if a thermoelectric engine (e.g., in the absence or in combination with the fluid) is implemented to adjust the temperature of mattress 200, then a phase change material can be used to transfer the heat away from the side of the thermoelectric element such that it is distributed farther away from where the user sleeps (e.g., another area of mattress 200 such as below where the user sleeps, off to the side, etc.). That is, phase change material can be distributed upon or within mattress 200 such that it transports the heat from the side of the thermoelectric element that is hotter than the other, colder side away from those sleeping upon mattress 200.

The phase change material can include an organic material, such as, for example carbohydrates or lipids. Examples of the organic phase change material include Lauric acid, TME(63%) / H₂O(37%), Paraffin 14-Carbons, Paraffin 15-Carbons, Paraffin 16-Carbons, Paraffin 17-Carbons, Paraffin 18-Carbons, Paraffin 19-Carbons, Paraffin 20-Carbons, Paraffin 21-Carbons, Paraffin 22-Carbons, Paraffin 23-Carbons, Paraffin 24-Carbons, Paraffin 25-Carbons, Paraffin 26-Carbons, Paraffin 27-Carbons, Paraffin 28-Carbons, Paraffin 29-Carbons, Paraffin 30-Carbons, Paraffin 31-Carbons, Paraffin 32-Carbons, Paraffin 33-Carbons, Paraffin 34-Carbons, Formic acid, Caprilic acid, Glycerin, p-Lattic acid, Methyl palmitate, Camphenilone, Docasyl bromide, Caprylone, Phenol, Heptadecanone, 1-Cyclohexylooctadecane, 4-Heptadacanone, p-Joluidine, Cyanamide, Methyl eicosanate, 3-Heptadecanone, 2-Heptadecanone, Hydrocinnamic acid, Cetyl acid, a-Nepthylamine, Camphene, O-Nitroaniline, 9-Heptadecanone, Thymol, Methyl behenate, Diphenyl amine, p-Dichlorobenzene, Oxolate, Hypophosphoric acid, O-Xylene dichloride, β-Chloroacetic acid, Chloroacetic acid, Nitro napthalene, Trimyristin, Heptaudecanoic acid, α-Chloroacetic acid, Bees wax, Glyolic acid, Glycolic acid, p-Bromophenol, Azobenzene, Acrylic acid, Dinto toluent (2,4), Phenylacetic acid, Thiosinamine, Bromcamphor, Durene, Methly brombenzoate, Alpha napthol, Glautaric acid, p-Xylene dichloride, Catechol, Quinone, Actanilide, Succinic anhydride, Benzoic acid, Stibene, Benzamide, Acetic acid, Polyethylene glycol 600, Capric acid, Eladic acid, Pentadecanoic acid, Tristearin, Myristic acid, Palmatic acid, Stearic acid, Acetamide, Methyl fumarate, modifications thereof, or combinations thereof. Alternatively or in addition to, the phase change material can include inorganic materials, such as, for example, salts (e.g., salt hydrates), inorganic eutectics, or hygroscopic materials. Examples of the inorganic phase change material include water, sodium sulfate (Na₂SO₄·10H₂O), NaCl·Na₂SO₄·10H₂O, Mn(NO₃)₂·6H₂O / MnCl₂·4H₂O(4%), Na₂SiO₃·5H₂O, Aluminium, Copper, Gold, Iron, Lead, Lithium, Silver, Titanium, Zinc, NaNO₃, NaNO₂, NaOH, KNO₃, KOH, NaOH / Na₂CO₃(7.2%), NaCl(26.8%) / NaOH, NaCl / KCL(32.4%) / LiCl(32.8%), NaCl(5.7%) / NaNO₃(85.5%) / Na₂SO₄, NaCl / NaNO₃(5.0%), NaCl(5.0%) / NaNO₃, NaCl(42.5%) / KCl(20.5%) / MgCl₂, KNO₃(10%) / NaNO₃, KNO₃ / KCl(4.5%), KNO₃ / KBr(4.7%) / KCl(7.3%), modifications thereof, or combinations thereof. In some cases, the phase change material (e.g., paraffin) can be used for thermal energy storage and, therefore, can be used to store heat away from a user's body while sleeping upon mattress 200. The phase change material can be embedded within a memory foam (e.g., polyurethane) material that mattress 200 is composed of. In an example, paraffin can be "sprinkled" throughout the memory foam such that mattress 200 includes a layer of memory foam impregnated with paraffin as the phase change material. In some cases, a bladder or enclosure (e.g., made of rubber, plastic, etc.) of the phase change material (e.g., paraffin) can be integrated within mattress 200. In an example, the bladder can contain the paraffix wax such that it can be isolated into a particular layer of mattress 200. This can provide a layer of paraffin wax as a phase change material within mattress 200, resulting in a greater temperature regulation (e.g., cooling effect) than if paraffin was embedded throughout the memory foam. In such a case, more heat can be transported away from a user.

In some cases, the enclosure of the phase change material (e.g., paraffin wax) can be beneath a layer of memory foam upon which a user sleeps. For example, mattress 200 can include a layer of memory foam (e.g., a layer closer to the person sleeping upon mattress 200) and beneath that memory foam can be a layer of thermoelectric elements. Beneath that layer of thermoelectric elements, the enclosure of the phase change material (e.g., paraffin wax) can be positioned such that the heat separated by the thermoelectric elements can be distributed downward and away from the other side of the memory foam (e.g., the layer of memory foam upon which the user sleeps that is opposite from the side that is closest to the thermoelectric elements). Thus, these three layers can be positioned adjacent to each other as described above to distribute heat towards or away from the person sleeping upon mattress 200.

In some cases, the phase change material can also be concentrated in the portions of mattress 200 that are expected to be underneath a user's back, shoulders, and hips. Other portions of mattress 200, such as the areas underneath where a user's legs would be while sleeping, can have a lower concentration of the phase change material, or no phase change material.

Computing device 2005 can also be used to provide additional temperature settings. In some cases, a user may want a warming or cooling effect within a certain time period. In some cases, the user may want a plurality of time periods to be set with different temperature set points. In some cases, some users might only want the warming or cooling effect to be provided from 10:00 P.M. to 1:00 A.M.. This time period might include the general time period that the user tends to sleep and, therefore, only providing the warming or cooling effect during that time period can aid the user to fall asleep, but also prevent the usage of the system while the user is asleep later throughout the night. This can be helpful to reduce electricity costs of operating the system. Hub 2040 can also provide information related to the adjusting of the temperature of the mattress to computing device 2005 via a wireless network (e.g., a WLAN network as previously discussed).

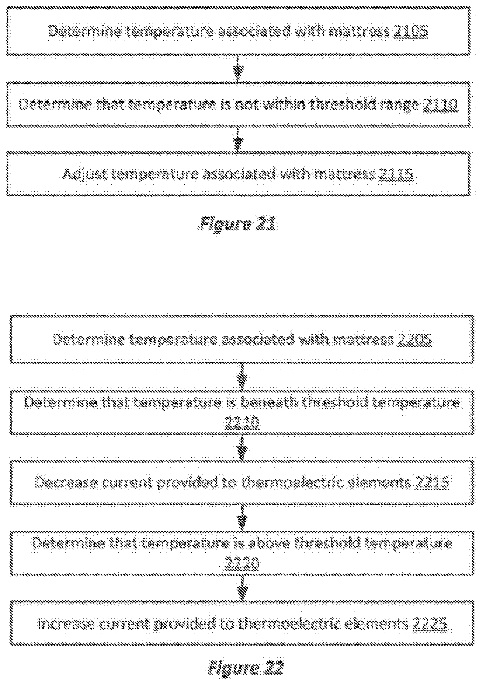

FIG. 21 is another example of a block diagram for adjusting a temperature of an article of furniture (e.g., a bed). In FIG. 21, at block 2105, a temperature associated with the bed (e.g., a mattress of the bed) can be determined. For example, in FIG. 20, the temperature of mattress 200 can be determined using one or more sensors (e.g., one or more temperature sensors) in a portion of the mattress 200, integrated within mattress 200, placed upon mattress 200, integrated within a cover that is placed upon mattress 200, etc. Such sensor(s) may measure one or more temperatures indicative of a user's body temperature. In some cases, the temperature can be the temperature of the user sleeping upon mattress 200. In some cases, the user might be wearing an activity tracker, smart watch, etc., which activity tracker can be used as a sensor for determining the user's body temperature. In some cases, the temperature might be an ambient temperature adjacent to the bed device (e.g., the mattress 200) or within the sheets or comforter of the mattress 200 (e.g., the temperature above mattress 200 but below sheets that the person is sleeping under) that may or may not be indicative of the user's bodily temperature.

At block 2110, the temperature can be determined to be outside of a threshold range. For example, hub 2040 in FIG. 20 can receive a temperature 2030 from the sensors (e.g., the temperature sensors). Hub 2040 might try to regulate the temperature of mattress 200 to be within a certain range. If temperature 2030 is below that range, then that might mean that the person sleeping upon mattress 200 is cold. If temperature 2030 is above that range, then that might mean that the person sleeping upon mattress 200 is hot.

Thus, at block 2115, the temperature associated with the mattress can be adjusted. For example, in FIG. 20, hub 2040 can generate temperature adjust 2035. Temperature adjust 2035 can be an analog signal providing an amount of current supplied to thermoelectric elements of mattress 200 such that heat can be distributed away from the person sleeping upon mattress 200 using the thermoelectric elements, as previously discussed. Alternatively or in addition to, temperature adjust 2035 can be a computer implemented instruction to instruct the thermoelectric temperature regulator to regulate (i) a temperature of the fluid (e.g., water) flowing between the thermoelectric temperature regulator and the channel(s) of the article of furniture (e.g., the bed), and (ii) a flow of such fluid through the channel(s) of the article of furniture, thereby to adjust a temperature of at least a portion of the article of furniture. In some cases, temperature adjust 2035 can include digital data, for example, instructions for the thermoelectric temperature regulator, fans, pumps, etc. to provide the heating or cooling of the fluid. In some cases, analog signals as described can also be provided to the thermoelectric temperature regulator, fans, pumps, etc.

FIG. 22 is an example of a block diagram for adjusting current provided to one or more thermoelectric elements of the thermoelectric regulator for adjusting a temperature of an article of furniture (e.g., a bed). In FIG. 22, at block 2205, the temperature associated with the bed (e.g., a mattress of the bed) can be determined. For example, in FIG. 20, temperature 2030 provided by the sensor(s) (e.g., temperature sensor(s)) can be received by hub 2040. Temperature 2030 can provide temperature readings from the sensor(s) of mattress 200. At block 2210, it can be determined that the temperature is beneath a threshold temperature. The threshold temperature may be a pre-determined temperature (e.g., a temperature suggested by physician, an average temperature of the user while using the article of furniture, etc.). The threshold temperature may be a pre-assigned temperature by the user. For example, hub 2040 can determine that the temperature 2030 is beneath the threshold temperature range, meaning that the person sleeping upon mattress 200 is too cold. Thus, the current provided to the thermoelectric elements can be reduced at block 2115. For example, hub 2040 can provide temperature adjust 2035 by providing a lower current than what it was providing before. This can result in the current provided to the thermoelectric elements to be reduced, resulting in a lower voltage across those thermoelectric elements. This reduces the heat separation capabilities of the thermoelectric elements, as previously discussed, and therefore less heat can be distributed away from the person sleeping upon mattress 200. That is, the difference in temperature between the two sides of the thermoelectric element can be reduced, reducing the heat distribution. This can allow for the temperature to increase within the threshold range such that the person is no longer cold. Such a method may be implemented when the thermoelectric regulator (i) directly regulate a temperature of the article of furniture, or (ii) regulates a temperature of a fluid that flows through the channel(s) of the article of furniture, thereby to adjust the heat distribution in the article of furniture.

At block 2220, it can be determined that the temperature is above a threshold temperature. For example, if the temperature increases such that it is now above the high temperature of the threshold temperature range, then this might indicate that the person sleeping upon mattress 200 is too hot. Thus, at block 2225, the current provided to the thermoelectric elements can be increased. This results in the thermoelectric elements having a higher voltage across them, improving the heat separation capabilities. This allows for the temperature difference across the thermoelectric element to increase due to the concentration of heat towards one end. The concentrated heat can then be distributed away using the phase change material, as previously discussed. This allows for the temperature to lower. Thus, a feedback loop can be implemented such that, in FIG. 20, hub 2040 is continuously or periodically (e.g., every second, every minute, every ten minutes, every time a motion upon mattress 200 is detected, every time snoring is heard, etc.) receiving and analyzing temperature 2030 and adjusting temperature adjust 2035 to heat or cool mattress 200 to provide a better sleeping experience.

In some cases, the thermoelectric element(s) of the thermoelectric engine(s) can be used for both cooling and heating an article of furniture (e.g., a bed or a mattress of the bed). For example, by changing the direction of the current of the signal provided to the thermoelectric elements, the operational mode can switch from cooling to heating, or heating to cooling.

### Thermal alarm

In one aspect, the present disclosure provides a system for regulating a temperature of a portion of an article of furniture (e.g., to wake up a user of the article of furniture). The system may comprise a sensor. The sensor may be a part of the article of furniture. Alternatively, the sensor may not be a part of the article of furniture, but operatively coupled to the article of furniture. The sensor may be configured to detect a biological signal of the user of the article of furniture. In some cases, the user may be one of a plurality of users of the article of furniture, and the sensor may be configured to detect a biological signal of each individual of the plurality of users. The system may comprise a temperature control device operatively coupled to the article of furniture, and the temperature control device may be configured to regulate the temperature of the article of furniture. The temperature control device may be thermally coupled to the article of furniture. The temperature control device may be coupled to (e.g., in contact with) the article of furniture. The system may comprise a processor communicatively coupled to the sensor and the temperature control device, and the processor may be configured to designate, while the user is asleep on the article of furniture, a time for the article of furniture to wake up the user based on the biological signal of the user that is detected by the sensor while the user is using the article of furniture. The processor may further be configured to regulate (e.g., change) the temperature of the portion of the article of furniture by the temperature control device prior to the time. The processor may be a part of the article of furniture. Alternatively, the processor may not be a part of the article of furniture, and communicatively and operatively linked to the article of furniture and one or more components of the article of furniture. In some cases, the processor may be configured to designate the time in absence of a user input to the processor (e.g., via a physical sensor or graphical user interface (GUI) of a computer system that is operatively coupled to the processor).

The system may comprise at least 1, 2, 3, 4, 5, 6, 7, 8, 9, 10, or more sensors. The system may comprise at most 10, 9, 8, 7, 6, 5, 4, 3, 2, or 1 sensor(s). An individual sensor may be configured to detect a biological signal of at least one user. In an example, an individual sensor may be capable of detecting one or more biological signals of a plurality of users of the article of furniture. In some cases, a plurality of sensors may be operatively in communication with one another. The system may comprise at least 1, 2, 3, 4, 5, or more temperature control devices. The system may comprise at most 5, 4, 3, 2, or 1 temperature control device(s). In some cases, a plurality of temperature control devices may be operatively in communication with one another.

In some cases, the processor may be further configured to designate the time based at least in part on the detected biological signal of the user and a history of biological signal data of the user, and regulate the temperature of the portion of the article of furniture prior to the time, thereby waking up the user of the article of furniture. The history of the biological signal data of the user may comprise one or more measurements of the user's biological signal while using the article of furniture.

In some cases, the history of the biological signal data of the user may comprise measurements of the user's biological signal during a current use of the article of furniture by the user (e.g., during the current sleep of the user). The history of biological signal data may comprise data measured from at least about the past 1 minute, 2 minutes, 3 minutes, 4 minutes, 5 minutes, 10 minutes, 20 minutes, 30 minutes, 40 minutes, 50 minutes, 60 minutes, 1.5 hours, 2 hours, 3 hours, 4 hours, 5 hours, 6 hours, 7 hours, 8 hours, 9 hours, 10 hours, 11 hours, 12 hours, or more. The history of biological signal data may comprise data measured from at most about the past 12 hours, 11 hours, 10 hours, 9 hours, 8 hours, 7 hours, 6 hours, 5 hours, 4 hours, 3 hours, 2 hours, 1.5 hours, 60 minutes, 50 minutes, 40 minutes, 30 minutes, 20 minutes, 10 minutes, 5 minutes, 4 minutes, 3 minutes, 2 minutes, 1 minute, or less.

The current use of the article of furniture by the user may range from about 0.1 hours to about 16 hours. The current use of the article of furniture by the user may range from at least about 0.1 hours. The current use of the article of furniture by the user may range from at most about 16 hours. The current use of the article of furniture by the user may range from about 0.1 hours to about 0.5 hours, about 0.1 hours to about 1 hour, about 0.1 hours to about 2 hours, about 0.1 hours to about 3 hours, about 0.1 hours to about 4 hours, about 0.1 hours to about 6 hours, about 0.1 hours to about 8 hours, about 0.1 hours to about 10 hours, about 0.1 hours to about 12 hours, about 0.1 hours to about 14 hours, about 0.1 hours to about 16 hours, about 0.5 hours to about 1 hour, about 0.5 hours to about 2 hours, about 0.5 hours to about 3 hours, about 0.5 hours to about 4 hours, about 0.5 hours to about 6 hours, about 0.5 hours to about 8 hours, about 0.5 hours to about 10 hours, about 0.5 hours to about 12 hours, about 0.5 hours to about 14 hours, about 0.5 hours to about 16 hours, about 1 hour to about 2 hours, about 1 hour to about 3 hours, about 1 hour to about 4 hours, about 1 hour to about 6 hours, about 1 hour to about 8 hours, about 1 hour to about 10 hours, about 1 hour to about 12 hours, about 1 hour to about 14 hours, about 1 hour to about 16 hours, about 2 hours to about 3 hours, about 2 hours to about 4 hours, about 2 hours to about 6 hours, about 2 hours to about 8 hours, about 2 hours to about 10 hours, about 2 hours to about 12 hours, about 2 hours to about 14 hours, about 2 hours to about 16 hours, about 3 hours to about 4 hours, about 3 hours to about 6 hours, about 3 hours to about 8 hours, about 3 hours to about 10 hours, about 3 hours to about 12 hours, about 3 hours to about 14 hours, about 3 hours to about 16 hours, about 4 hours to about 6 hours, about 4 hours to about 8 hours, about 4 hours to about 10 hours, about 4 hours to about 12 hours, about 4 hours to about 14 hours, about 4 hours to about 16 hours, about 6 hours to about 8 hours, about 6 hours to about 10 hours, about 6 hours to about 12 hours, about 6 hours to about 14 hours, about 6 hours to about 16 hours, about 8 hours to about 10 hours, about 8 hours to about 12 hours, about 8 hours to about 14 hours, about 8 hours to about 16 hours, about 10 hours to about 12 hours, about 10 hours to about 14 hours, about 10 hours to about 16 hours, about 12 hours to about 14 hours, about 12 hours to about 16 hours, or about 14 hours to about 16 hours. The current use may range from about 0.1 hours, about 0.5 hours, about 1 hour, about 2 hours, about 3 hours, about 4 hours, about 6 hours, about 8 hours, about 10 hours, about 12 hours, about 14 hours, or about 16 hours.

In some cases, the history of the biological signal data of the user may comprise measurements of the user's biological signal during one or more previous uses of the article of furniture by the user (e.g., one or more previous sleeps of the user on the article of furniture). The previous uses may comprise at least 1 day, 2 days, 3 days, 4 days, 5 days, 6 days, 7 days, 2 weeks, 3 weeks, 4 weeks, 2 months, 3 months, 4 months, 5 months, 6 months, 7 months, 8 months, 9 months, 10 months, 11 months, 12 months, 2 years, 3 years, 4 years, 5 years, or more. The previous uses may comprise at most about the past 5 years, 4 years, 3 years, 2 years, 12 months, 11 months, 10 months, 9 months, 8 months, 7 months, 6 months, 5 months, 4 months, 3 months, 2 months, 4 weeks, 3 weeks, 2 weeks, 7 days, 6 days, 5 days, 4 days, 3 days, 2 days, or 1 day.

In some cases, the one or more previous uses may have occurred at least about 1 day to 1 year prior to the time. In some cases, the one or more previous uses may have occurred at least about 1 day to 10 months year prior to the time. In some cases, the one or more previous uses may have occurred at least about 1 day to 8 months year prior to the time. In some cases, the one or more previous uses may have occurred at least about 1 day to 6 months year prior to the time. In some cases, the one or more previous uses may have occurred at least about 1 day to 4 months year prior to the time. In some cases, the one or more previous uses may have occurred at least about 1 day to 2 months year prior to the time. In some cases, the one or more previous uses may have occurred at least about 1 day to 1 month year prior to the time. In some cases, the one or more previous uses may have occurred at least about 1 day to 3 weeks year prior to the time. In some cases, the one or more previous uses may have occurred at least about 1 day to 2 weeks prior to the time. In some cases, the one or more previous uses may have occurred at least about 1 day to 1 week prior to the time. In some cases, the one or more previous uses may have occurred at least about 1 day to 6 days prior to the time. In some cases, the one or more previous uses may have occurred at least about 1 day to 5 days prior to the time. In some cases, the one or more previous uses may have occurred at least about 1 day to 4 days prior to the time. In some cases, the one or more previous uses may have occurred at least about 1 day to 3 days prior to the time. In some cases, the one or more previous uses may have occurred at least about 1 day to 2 days prior to the time.

In some cases, the processor may be communicatively coupled to at least one database, wherein the at least one database comprises a database associated with the article of furniture or a database associated with the user. In some cases, the processor may be configured to obtain the history (e.g., current history, previous history, or both) of biological signal data of the user from the at least one database.

In some cases, the processor may be further configured to identify the user from a plurality of users of the article of furniture based at least in part on the detected biological signal of the user. In some cases, the processor may be further configured to obtain the history of biological signal data of the user from the plurality of users based at least in part on the identity of the user.

In some cases, the biological signal of the user may comprise a heart signal, a respiration signal, a motion, a temperature, and/or perspiration. In some cases, the biological signal of the user may comprise two or more of: a heart signal, a respiration signal, a motion, a temperature, and perspiration. In some examples, the biological signal of the user may comprise a temperature and at least one of: a heart signal and a respiration signal. In some cases, the biological signal of the user may comprise three or more of: a heart signal, a respiration signal, a motion, a temperature, and perspiration. In some examples, the biological signal of the user may comprise a temperature, a heart signal, and a respiration signal.

In some cases, the processor may identify the user from the plurality of users based on a heart signal (e.g., amplitude and/or frequency of the heart signal) and/or a respiration signal (e.g., amplitude and/or frequency of the respiration signal). In some cases, the processor may use a piezo sensor to detect the heart signal and/or the respiration signal. The detected heart signal and/or the respiration signal may be compared to a plurality of historical data of the heart signal and/or the respiration signal of the plurality of users to identify the user from the plurality of users of said article of furniture. The plurality of historical data of the heart signal and/or the respiration signal may be stored in one or more databases that are operatively in communication with the processor of the article of furniture. In some cases, the processor may use detect and/or confirm a presence of a user based on a temperature of a surface of the article of furniture detected by the sensor. In some cases, the processor may use a temperature sensor to detect the temperature of the surface of the article of furniture. In an example, if the processor detects a sudden change in the temperature of the surface of the article of furniture, such data may indicate a start or end of a use of the article of furniture by one or more users.

In some cases, the article of furniture may comprise both the piezo sensor and the temperature sensor, wherein the piezo sensor and the temperature sensor are disposed on opposite sides of a layer of the article of furniture (e.g., on opposite surfaces of a layer of the bed device).

In some cases, the temperature control device may comprise a temperature regulatable mat and a controller to regulate a temperature of the mat. The controller may or may not be a part of the article of furniture. The temperature regulatable mat may be a part of the article of furniture. In some cases, the temperature regulatable mat may be disposed at a distance away from the temperature sensor, such that the temperature sensor does not read a temperature of the temperature regulatable mat. In some cases, the temperature regulatable mat may be on or adjacent to the layer comprising the piezo sensor and the temperature sensor, wherein the temperature sensor and the temperature regulatable mat may be on opposite sides of the layer. In some cases, the temperature sensor and the temperature regulatable mat may be a same side of the layer, but with sufficient spacing and/or insulation in between.

In some cases, the processor may be further configured to identify the user from a plurality of users of the article of furniture based at least in part on the detected biological signal of the user. In some cases, the processor may be further configured to designate the time to wake up the user based at least in part on the identity of the user, and regulate the temperature of the portion of the article of furniture prior to the time, thereby waking up the user of the article of furniture.

In some cases, the at least one sensor of the article of furniture may be configured to detect a first biological signal and a second biological signal of the user. The first biological and the second biological signal of the user may be different types of biological signals of the user. In some cases, the processor may be configured to (i) determine a presence of the user on the article of furniture based on the first biological signal, (ii) identify the user from a plurality of users of the article of furniture based on the second biological signal, and (iii) designate the time for the article of furniture to wake up the user based on the user's identity. In some examples, the first biological signal may be a temperature of the user. In some examples, the second biological signal may be a heart signal of the user. In some examples, the second biological signal may be a breathing signal of the user.

In some cases, the at least one sensor of said article of furniture may be configured to detect a first biological signal of a first user of said article of furniture and a second biological signal of a second user of said article of furniture. In such cases, the processor may be configured to (i) identify the first user from the first user and the second user based on the first biological signal, and designate a first time for the article of furniture to wake up the first user based on said first user's identity, and (ii) identify the second user from the first user and the second user based on the second biological signal, and designate a second time for the article of furniture to wake up the second user based on the second user's identity. The first time and the second time may be the same or different.

In some cases, the identity of the user may comprise a circadian rhythm associated with the user. In some cases, the processor may be further configured to designate the time based at least in part on the circadian rhythm of the user, and regulate the temperature of the portion of the article of furniture prior to the time, thereby waking up the user of the article of furniture. The circadian rhythm of the user may comprise a pattern of sleeping and/or waking up of the user from one or more time periods (e.g., one or more 24-hour periods or cycles). The one or more time periods may comprise at least about 1 day, 2 days, 3 days, 4 days, 5 days, 6 days, 7 days, 2 weeks, 3 weeks, 4 weeks, 2 months, 3 months, 4 months, 5 months, or more. The one or more time periods may be at most about 5 months, 4 months, 3 months, 2 months, 4 weeks, 3 weeks, 2 weeks, 7 days, 6 days, 5 days, 4 days, 3 days, 2 days, or 1 day(s). In some cases, each of the one or more time periods may be a portion of a 24-hour cycle, such as at least 3 hours, 4 hours, 5 hours, 6 hours, 7 hours, 8 hours, 9 hours, 10 hours, 11 hours, 12 hours of the 24-hour cycle.

In some cases, the circadian rhythm of the user may be generated by the article of furniture (e.g., by the processor of the article of furniture) by using (i) one or more sensors (e.g., the at least one sensor of the article of furniture) to detect one or more biological signals of the user, and/or (ii) one or more additional sensors (e.g., a wearable sensor) associated with the user. In some cases, the wearable sensor may comprise a smart watch.

In some cases, the identity of the user may comprise a plurality of sleep phases associated with the user. In some cases, the processor may be further configured to identify a sleep phase of the user from the plurality of sleep phases. In some cases, the processor may be further configured to designate the time based at least in part on the identified sleep phase of the user, and regulate the temperature of the portion of the article of furniture prior to the time, thereby waking up the user of the article of furniture. In some cases, the user may be in or about to enter a sleep phase that is optimal for waking up, and the processor may designate the time based at least in part on the identified sleep phase of the user, and regulate the temperature of the portion of the article of furniture prior to the time. In some cases, the user may be in a sleep phase that is undesired, and the processor may designate the time based at least in part on the identified sleep phase of the user, and regulate the temperature of the portion of the article of furniture prior to the time.

In some cases, the identity of the user may comprise an activity data of the user. The activity data may comprise an exercise pattern and/or a food consumption data of the user. Examples of the exercise pattern may comprise duration and/or frequency of walking, running, swimming, basketball, baseball, hockey, tennis, gymnastics, standing for duration of time, etc. Examples of the food consumption data may comprise types of foods consumed by the user (e.g., elementary foods, pre-packaged meals, home-cooked meals, fruits, vegetables, etc.), amounts of foods consumed by the user, frequency of food consumption by the user, and/or time of the food consumption within the day. In some cases, the processor may be further configured to designate the time based at least in part on the activity data of the user, and regulate the temperature of the article of furniture prior to the time, thereby waking up the user of the article of furniture. In some cases, the processor may allow the user to wake up faster or slower, in comparison to an article of furniture without such processor, based on the exercise pattern and/or the food consumption data of the user. In an example, the processor may regulate metabolism of the user by delaying the time at which to regulate the temperature of the article of furniture to wake up the user, thereby giving the user more time to metabolize food and its nutrients while sleeping.

In some cases, the identity of the user may comprise a predetermined wake-up time of the user. In some cases, the processor may be configured to retrieve the predetermined wake-up time of the user, and regulate the temperature of the article of furniture prior to the predetermined wake-up time of the user, thereby waking up the user of the article of furniture. In an example, the user may provide a preferred wake-up time that may or may not be specific for the day of the week. In such a case, the processor may obtain such preferred wake-up time of the user from the identity of the user (e.g., a digital profile of the user) and regulate the temperature of the article of furniture to wake up the user at or about the preferred wake-up time of the user.

In some cases, the identity of the user may comprise a history of wake-up time(s) of the user while using the article of furniture. In some cases, the processor may be further configured to designate the time based at least in part on the history of wake-up time(s) of the user, and regulate the temperature of the article of furniture prior to the time, thereby waking up the user of the article of furniture. The article of furniture (e.g., one or more sensors of the article of furniture) may be able to detect movement, presence, and/or absence of the user on the article of furniture. The detected movement, presence, and/or absence of the user on the article of furniture may be used to (i) determine when (e.g., time) the user is awake from sleep, and (ii) generate the history of wake-up time(s) of the user.

In some cases, the processor may be further configured to designate the time based at least in part on an average wake-up time of the user from the history of wake-up time(s) of the user, and regulate the temperature of the article of furniture prior to the time, thereby waking up the user of the article of furniture. The processor may obtain the history of wake-up time(s) of the user, and generate (e.g., calculate) the average wake-up time of the user. As such, the processor may regulate the temperature of the article of furniture at a specific time, such that the user may wake up at or around the average wake-up time of the user.

In some cases, the identity of the user may comprise a predetermined biological signal level the user. Examples of the predetermined biological signal level of the user may comprise a predetermined heart signal level, predetermined respiration signal level, predetermined motion level, predetermined temperature level, and/or predetermined perspiration level. In some cases, the processor may be further configured to designate the time based at least in part on the predetermined biological signal level of the user, and regulate the temperature of the article of furniture prior to the time, thereby waking up the user of the article of furniture. In some cases, the processor may designate the time once the predetermined biological signal is reached (e,g,, detected by one or more sensors of the article of furniture) at least 1 time, 2 times, 3 times, 4 times, 5 times, or more. In some cases, the processor may regulate the temperature of the article of furniture to wake up the user when the predetermined biological signal is reached (e,g,, detected by one or more sensors of the article of furniture) at most 5 times, 4 times, 3 times, 2 times, or 1 time. Alternatively or in addition to, the processor may be configured to designate the time when the detected biological signal of the user is expected (or projected) to reach the predetermined biological signal for at least 1 time, 2 times, 3 times, 4 times, 5 times, or more (or at most 5 times, 4 times, 3 times, 2 times, or 1 time). In some cases, the processor may designate the time when a current biological signal of the user is within a range (e.g., a predetermined range) away from the predetermined biological signal level of the user. Alternatively or in addition to, the processor may be configured to designate the time to be when a current biological of the user is expected (or projected) to be within a range away from the predetermined biological signal level of the user.

In an example, the user may be suspected of a health condition (e.g., heart condition), and it may be beneficial for the user to wake up (or be woken up by the article of furniture) prior to, during, and/or subsequent to reaching a predetermined heart signal during sleep. In another example, the user may be suspected of having a cold or flu, and it may be beneficial for the user to wake up prior to, during, and/or subsequent to reaching a predetermined temperature (e.g., 102°F) during sleep. Other examples of a health condition of the user can include, but are not limited to sleep disorders, neurological disorders, mental conditions (e.g., post-traumatic stress disorder), blood disorders, cancers, metabolic disorders, eye disorders, organ disorders, musculoskeletal disorders, cardiac disease, addictions (e.g., drug additions), and the like.

In some cases, the identity of the user may comprise one or more future events of the user. In some cases, the processor may be further configured to regulate the temperature of the article of furniture based at least in part on the one or more future events of the user, thereby waking up the user of the article of furniture. The future event(s) of the user may comprise a time and/or location of the future event(s). In some cases, the future event(s) may occur on the same day that the user is sleeping. In some cases, the processor may be operatively linked to a digital profile or the user comprising a digital calendar of the user. In some cases, the processor may be operatively linked to one or more personal devices (e.g., a mobile device, a computer, etc.) of the user to gain access to the digital calendar of the user. In some cases, information on the future event(s) may be provided as input data to the processor of the article of furniture by the user. In some cases, the processor may determine a wake-up time that provides the user sufficient time to prepare (e.g., shower, get dressed, transport to the event, etc.) for the future event(s) after waking up.

In some cases, the identity of the user may comprise a geolocation of the user while using the article of furniture. In some cases, the processor may be further configured to regulate the temperature of the article of furniture based at least in part on the geolocation of the user, thereby waking up the user of the article of furniture. Examples of the geolocation of the user may include continent, country, town, city, longitude, and/or latitude of the user while using the article of furniture. The processor of the article of furniture may be in digital communication with one or more databases (e.g., via internet) to obtain such data related the geolocation of the user. The processor of the article of furniture may be in digital communication with one or more personal devices of the user to obtain such data related to the geolocation of the user. In some cases, the geolocation may be provided by the user.

In some cases, the processor may be further configured to regulate the temperature of the article of furniture based at least in part on weather condition (e.g., snow, rain, earthquake, hurricane, etc.) of the geolocation, thereby waking up the user of the article of furniture.

In some cases, the processor may be further configured to obtain a current and/or projected traffic condition at or adjacent to the geolocation. In some cases, the processor may be further configured to regulate the temperature of the article of furniture based at least in part on the current and/or projected traffic condition, thereby waking up the user of the article of furniture. In some examples, using the geolocation of the user while using the article of furniture, the processor may adjust a wake-up time of the user depending on how heavy or light the traffic condition may be in the morning. In an example, if the traffic condition is projected to be bad from 7 A.M. to 9 A.M., the processor may regulate the temperature of the article of furniture to wake up the user before 7 A.M.

In some cases, the processor may comprise or may be operatively coupled to a global positioning system (GPS) to retrieve data with respect to the geolocation of the article of furniture and/or the user of the article of furniture. The processor may be coupled to the GPS via a wireless signal (e.g., near-field communication (NFC), Bluetooth, Wi-Fi, etc.) or a cable connection (e.g., USB 2.0, USC-C, micro-USB, etc.). In some cases, the processor may be operatively coupled to a user device (e.g., via a wireless signal or a cable connection). Examples of the user device may include, but are not limited to, a tablet computer, a mobile phone, a smart phone, a smart watch, a smart glass, etc. The user device may comprise or may be operatively coupled to the GPS, and the processor may retrieve data with respect to the geolocation of the article of furniture and/or the user through the user device. Additionally, the processor, the GPS, and/or the user device may be operatively coupled to (1) a weather database (e.g., National Weather Service, AccuWeather, Weather Underground, WeatherBug, etc.) to retrieve past, current, and/or forecasted weather conditions of the geolocation, and/or (2) a traffic database (e.g., Department of Transportation, Google Maps, Waze, Apple Maps, Sygic, MapQuest, INRIX Traffic, HERE WeGo, inRoute, Glob, Scout, ETA, etc.) to retrieve past, current, and/or forecasted ground (e.g., cars, buses, subways, trains, rental bikes, rental scooters, etc.) and/or air transportation traffic conditions at or near the geolocation.

In some cases, the processor may retrieve data with respect to one or more future events (or one or more planned events) of the user through the user device (e.g., from a calendar or scheduling application that is operatively coupled to the user device).

In some cases, the processor may be further configured to determine a wake-up time of the user of the article of furniture based at least in part on the detected biological signal of the user. In some cases, the processor may be further configured to regulate (e.g., change) the temperature of the article of furniture before the determined wake-up time of the user, thereby waking up the user of the article of furniture at or around the determined wake-up time of the user.

To wake up the user, the processor may initiate changing the temperature of the article of furniture at least 1 minute, 2 minutes, 3 minutes, 4 minutes 5 minutes, 10 minutes, 15 minutes, 20 minutes, 25 minutes, 30 minutes, 35 minutes, 40 minutes, 45 minutes, 50 minutes, 55 minutes, 60 minutes, or more prior to the determined wake-up time of the user. To wake up the user, the processor may initiate changing the temperature of the article of furniture at most 60 minutes, 55 minutes, 50 minutes, 45 minutes, 40 minutes, 35 minutes, 30 minutes, 25 minutes, 20 minutes, 15 minutes, 10 minutes, 5 minutes, 4 minutes, 3 minutes, 2 minutes or less prior to the determined wake-up time of the user. In an example, to wake up the user, the processor may initiate changing the temperature of the article of furniture at about 30 minutes prior to the determined wake-up time of the user.

To wake up the user, the processor may regulate the temperature of the article of furniture at a rate of at least about 0.1°F/hour, 0.2°F/hour, 0.3°F/hour, 0.4°F/hour, 0.5°F/hour, 0.6°F/hour, 0.7°F/hour, 0.8°F/hour, 0.9°F/hour, 1°F/hour, 2°F/hour, 3°F/hour, 4°F/hour, 5°F/hour, 6°F/hour, 7°F/hour, 8°F/hour, 9°F/hour, 10°F/hour, 11°F/hour, 12°F/hour, 13°F/hour, 14°F/hour, 15°F/hour, 16°F/hour, 17°F/hour, 18°F/hour, 19°F/hour, 20°F/hour, 25°F/hour, 30°F/hour, 35°F/hour, 40°F/hour, or more. To wake up the user, the processor may regulate the temperature of the article of furniture at a rate of at most about 40°F/hour, 35°F/hour, 30°F/hour, 25°F/hour, 20°F/hour, 19°F/hour, 18°F/hour, 17°F/hour, 16°F/hour, 15°F/hour, 14°F/hour, 13°F/hour, 12°F/hour, 11°F/hour, 10°F/hour, 9°F/hour, 8°F/hour, 7°F/hour, 6°F/hour, 5°F/hour, 4°F/hour, 3°F/hour, 2°F/hour, 1 °F/hour, 0.9°F/hour, 0.8°F/hour, 0.7°F/hour, 0.6°F/hour, 0.5°F/hour, 0.4°F/hour, 0.3°F/hour, 0.2°F/hour, 0.1°F/hour, or less. In an example, the processor may regulate the temperature of the article of furniture at a rate of about 10 °F/hour (or 5 °F/30 minutes) to wake up the user. In some cases, the processor may be configured to determine (e.g., automatically determine) the rate at which the temperature control device is to regulate (e.g., increase or decrease) the temperature of the portion of the article of furniture. In an example, a different sensor may be configured to measure a temperature of the portion of the article of furniture (e.g., a temperature of a portion of a mattress or a mattress pad), and the processor may be configured to determine the rate based at least in part of the temperature of the portion of the article of furniture.

To wake up the user, the processor may direct the temperature control device to change (e.g., increase or decrease) the temperature of the article of furniture by at least about 0.1°F, 0.2°F, 0.3°F, 0.4°F, 0.6°F, 0.7°F, 0.8°F, 0.9°F, 1°F, 2°F, 3°F, 4°F, 5°F, 6°F, 7°F, 8°F, 9°F, 10°F, 11°F, 12°F, 13°F, 14°F, 15 °F, 16 °F, 17 °F, 18 °F, 19 °F, 20 °F, 25°F, 30°F, 35°F, 40°F, 45°F, 50°F, or more. In some cases, to wake up the user, the processor may increase and/or decrease the temperature of the article of furniture by at most about 50°F, 45°F, 40°F, 35°F, 30°F, 25°F, 20°F, 19 °F, 18 °F, 17 °F, 16 °F, 15°F, 14°F, 13°F, 12°F, 11°F, 10°F, 9°F, 8°F, 7°F, 6°F, 5°F, 4°F, 3°F, 2°F, 1 °F, 0.9°F, 0.8°F, 0.7°F, 0.6°F, 0.5°F, 0.4°F, 0.3°F, 0.2°F, 0.1°F, or less.

In some embodiments, prior to changing the temperature of the portion of the article of furniture, the processor may be configured to designate a target temperature to which the temperature of the portion of the article of furniture is to be changed to. In some cases, a target temperature of the article of furniture to wake up the user may depend on the user (e.g., a temperature of the user during a current sleep), the environment of the article of furniture, the geolocation and weather condition around the user and the article of furniture, etc.

In some cases, the target temperature to wake up the user may be designated (e.g., by the processor) based at least in part on a temperature of the user detected during a current sleep on the article of furniture. In some examples, the target temperature may be based at least in part on a current temperature of the user. The current temperature may be a temperature of the user measured at a predetermined time, e.g., at about 6 P.M., about 6:30 P.M., about 7 P.M., about 7:30 P.M., about 8 P.M., about 8:30 P.M., about 9 P.M., about 9:30 P.M., about 10 P.M., about 10:30 P.M., about 11 P.M., about 11:30 P.M., about 12 A.M., about 12:30 A.M., about 1 A.M., about 1:30 A.M., about 2 A.M., about 2:30 A.M., about 3 A.M., about 3:30 A.M., about 4 A.M., about 4:30 A.M., about 5 A.M., about 5:30 A.M., about 6 A.M., about 6:30 A.M., about 7 A.M., 7:30 A.M., about 8 A.M., about 8:30 A.M., about 9 A.M, etc. Alternatively, the current temperature may be an average or median temperature of the user during the current sleep of the user, a highest temperature of the user measured during the current sleep of the user, or a lowest temperature of the user measured during the current sleep of the user.

In some cases, a different between the target temperature to wake up the user and the current temperature of the user may be at least about 0.1°F, 0.2°F, 0.3°F, 0.4°F, 0.5°F, 0.6°F, 0.7°F, 0.8°F, 0.9°F, 1°F, 1.1°F, 1.2°F, 1.3°F, 1.4°F, 1.5°F, 1.6°F, 1.7°F, 1.8°F, 1.9°F, 2°F, 2.1°F, 2.2°F, 2.3°F, 2.4°F, 2.5°F, 2.6°F, 2.7°F, 2.8°F, 2.9°F, 3°F, 3.1°F, 3.2°F, 3.3°F, 3.4°F, 3.5°F, 3.6°F, 3.7°F, 3.8°F, 3.9°F, 4°F, 4.5°F, 5°F, 6°F, 7°F, 8°F, 9°F, 10°F, 15°F, 20°F, 25°F, 30°F, or more. In some cases, a different between the target temperature to wake up the user and the current temperature of the user may be at most about 30°F, 25°F, 20°F, 15°F, 10°F, 9°F, 8°F, 7°F, 6°F, 5°F, 4.5°F, 4°F, 3.9°F, 3.8°F, 3.7°F, 3.6°F, 3.5°F, 3.4°F, 3.3°F, 3.2°F, 3.1°F, 3°F, 2.9°F, 2.8°F, 2.7°F, 2.6°F, 2.5°F, 2.4°F, 2.3°F, 2.2°F, 2.1°F, 2°F, 1.9°F, 1.8°F, 1.7°F, 1.6°F, 1.5°F, 1.4°F, 1.3°F, 1.2°F, 1°F, 0.9°F, 0.8°F, 0.7°F, 0.6°F, 0.5°F, 0.4°F, 0.3°F, 0.2°F, 0.1°F, or less.

Alternatively or in addition to, the target temperature to wake up the user may be designated (e.g., by the processor) based at least in part on a temperature of the user detected during a previous sleep on the article of furniture.

In some cases, the target temperature to wake up the user may be designated (e.g., by the processor) based at least in part on a temperature of the article of furniture during the current sleep of the user. In some examples, the target temperature may be based at least in part on a current temperature of a portion of the article of furniture. The current temperature may be a temperature of the portion of the article of furniture measured at a predetermined time, e.g., at about 6 P.M., about 6:30 P.M., about 7 P.M., about 7:30 P.M., about 8 P.M., about 8:30 P.M., about 9 P.M., about 9:30 P.M., about 10 P.M., about 10:30 P.M., about 11 P.M., about 11:30 P.M., about 12 A.M., about 12:30 A.M., about 1 A.M., about 1:30 A.M., about 2 A.M., about 2:30 A.M., about 3 A.M., about 3:30 A.M., about 4 A.M., about 4:30 A.M., about 5 A.M., about 5:30 A.M., about 6 A.M., about 6:30 A.M., about 7 A.M., 7:30 A.M., about 8 A.M., about 8:30 A.M., about 9 A.M, etc. Alternatively, the current temperature may be an average or median temperature of the portion of the article of furniture during the current sleep of the user, a highest temperature of the portion of the article of furniture measured during the current sleep of the user, or a lowest temperature of the portion of the article of furniture measured during the current sleep of the user.

In some cases, a different between the target temperature to wake up the user and the current temperature of the portion of the article of furniture may be at least about 0.1°F, 0.2°F, 0.3°F, 0.4°F, 0.5°F, 0.6°F, 0.7°F, 0.8°F, 0.9°F, 1°F, 1.1°F, 1.2°F, 1.3°F, 1.4°F, 1.5°F, 1.6°F, 1.7°F, 1.8°F, 1.9°F, 2°F, 2.1°F, 2.2°F, 2.3°F, 2.4°F, 2.5°F, 2.6°F, 2.7°F, 2.8°F, 2.9°F, 3°F, 3.1°F, 3.2°F, 3.3°F, 3.4°F, 3.5°F, 3.6°F, 3.7°F, 3.8°F, 3.9°F, 4°F, 4.5°F, 5°F, 6°F, 7°F, 8°F, 9°F, 10°F, 15°F, 20°F, 25°F, 30°F, or more. In some cases, a different between the target temperature to wake up the user and the current temperature of the portion of the article of furniture may be at most about 30°F, 25°F, 20°F, 15°F, 10°F, 9°F, 8°F, 7°F, 6°F, 5°F, 4.5°F, 4°F, 3.9°F, 3.8°F, 3.7°F, 3.6°F, 3.5°F, 3.4°F, 3.3°F, 3.2°F, 3.1°F, 3°F, 2.9°F, 2.8°F, 2.7°F, 2.6°F, 2.5°F, 2.4°F, 2.3°F, 2.2°F, 2.1°F, 2°F, 1.9°F, 1.8°F, 1.7°F, 1.6°F, 1.5°F, 1.4°F, 1.3°F, 1.2°F, 1°F, 0.9°F, 0.8°F, 0.7°F, 0.6°F, 0.5°F, 0.4°F, 0.3°F, 0.2°F, 0.1°F, or less.

Alternatively or in addition to, the target temperature to wake up the user may be designated (e.g., by the processor) based at least in part on a temperature of at least a portion of the article of furniture detected during a previous sleep of the user on the article of furniture.

In some cases, the processor may use one or more environment sensors to detect one or more environment properties (e.g., ambient temperature, light, noise, humidity, etc.) surrounding the user, and determine (i) the wake-up time, (ii) a rate of change of temperature of the article of furniture to wake up the user, (iii) a target temperature of the article of furniture to wake up the user, and/or (iv) duration of the regulation of the temperature of the article of furniture based at least in part by the detected biological signal of the user and the one or more environment properties of the user.

In some cases, the target temperature to wake up the user may be designated (e.g., by the processor) based at least in part on an ambient temperature of an environment surrounding the article of furniture during the current sleep of the user. In some examples, the target temperature may be based at least in part on a current ambient temperature the environment surrounding the article of furniture. The current ambient temperature may be a temperature of the environment measured at a predetermined time, e.g., at about 6 P.M., about 6:30 P.M., about 7 P.M., about 7:30 P.M., about 8 P.M., about 8:30 P.M., about 9 P.M., about 9:30 P.M., about 10 P.M., about 10:30 P.M., about 11 P.M., about 11:30 P.M., about 12 A.M., about 12:30 A.M., about 1 A.M., about 1:30 A.M., about 2 A.M., about 2:30 A.M., about 3 A.M., about 3:30 A.M., about 4 A.M., about 4:30 A.M., about 5 A.M., about 5:30 A.M., about 6 A.M., about 6:30 A.M., about 7 A.M., 7:30 A.M., about 8 A.M., about 8:30 A.M., about 9 A.M, etc. Alternatively, the current ambient temperature may be an average or median temperature of the environment during the current sleep of the user, a highest temperature of the environment measured during the current sleep of the user, or a lowest temperature of the environment measured during the current sleep of the user.

In some cases, a different between the target temperature to wake up the user and the current temperature of environment surrounding the article of furniture may be at least about 0.1°F, 0.2°F, 0.3°F, 0.4°F, 0.5°F, 0.6°F, 0.7°F, 0.8°F, 0.9°F, 1°F, 1.1°F, 1.2°F, 1.3°F, 1.4°F, 1.5°F, 1.6°F, 1.7°F, 1.8°F, 1.9°F, 2°F, 2.1°F, 2.2°F, 2.3°F, 2.4°F, 2.5°F, 2.6°F, 2.7°F, 2.8°F, 2.9°F, 3°F, 3.1°F, 3.2°F, 3.3°F, 3.4°F, 3.5°F, 3.6°F, 3.7°F, 3.8°F, 3.9°F, 4°F, 4.5°F, 5°F, 6°F, 7°F, 8°F, 9°F, 10°F, 15°F, 20°F, 25°F, 30°F, or more. In some cases, a different between the target temperature to wake up the user and the current temperature of the environment surrounding the article of furniture may be at most about 30°F, 25°F, 20°F, 15°F, 10°F, 9°F, 8°F, 7°F, 6°F, 5°F, 4.5°F, 4°F, 3.9°F, 3.8°F, 3.7°F, 3.6°F, 3.5°F, 3.4°F, 3.3°F, 3.2°F, 3.1°F, 3°F, 2.9°F, 2.8°F, 2.7°F, 2.6°F, 2.5°F, 2.4°F, 2.3°F, 2.2°F, 2.1°F, 2°F, 1.9°F, 1.8°F, 1.7°F, 1.6°F, 1.5°F, 1.4°F, 1.3°F, 1.2°F, 1°F, 0.9°F, 0.8°F, 0.7°F, 0.6°F, 0.5°F, 0.4°F, 0.3°F, 0.2°F, 0.1°F, or less.

Alternatively or in addition to, the target temperature to wake up the user may be designated (e.g., by the processor) based at least in part on an ambient temperature of the environment surrounding the article of furniture detected during a previous sleep of the user on the article of furniture.

In some cases, to wake up the user, the regulation of the temperature of the article of furniture may comprise increasing and/or decreasing the temperature of the article of furniture. In some cases, to wake up the user, the regulation of the temperature of the article of furniture may only comprise increasing the temperature at one or more rates. In some cases, to wake up the user, the regulation of the temperature of the article of furniture may only comprise decreasing the temperature at one or more rates. In some cases, to wake up the user, the regulation of the temperature of the article of furniture may comprise a combination of both increasing and decreasing the temperature of the article of furniture. In an example, to wake up the user, the regulation of the temperature of the article of furniture may comprise one or more phases of increasing and decreasing (and/or vice versa) the temperature of the article of furniture, with or without intermittent pauses after each phase.

In some cases, the sensor may be a part of a first portion of the article of furniture, configured to detect a biological signal of the user of the first portion of the article of furniture. In some cases, the temperature control device may be coupled to a second portion of the article of furniture, configured to regulate a temperature of the second portion of the article of furniture. The first and second portions of the article of furniture may be the same or different. In an example, the first and second portions of the article of furniture may be different. In some cases, the processor may be communicatively coupled to the sensor and the temperature control device, and the processor may be configured to regulate the temperature of the second portion of the article of furniture based at least in part on the detected biological signal of the user on the first portion of the article of furniture, thereby waking up the user of the article of furniture. In some cases, the first portion and the second portion of the article of furniture may be two opposite sides of a component of the article of furniture (e.g., a top and bottom sides of a layer of a bed device).

In some cases, the temperature control device may be further configured to independently regulate a temperature of each of a plurality of zones of the second portion of the article of furniture. Each of the plurality of zones of the second portion of the article of furniture may be sufficient for a person to use (e.g., to sleep on).

In some cases, the processor may be further configured to (i) regulate (e.g., automatically regulate) a first temperature of a first zone of the plurality of zones of the second portion of the article of furniture based at least in part on a first detected biological signal of a first user on the first zone, thereby waking up the first user at a first time, and (ii) regulate (e.g., automatically regulate) a second temperature of a second zone of the plurality of zones of the second portion of the article of furniture based at least in part on a second detected biological signal of a second user on the second zone, thereby waking up the first user at a second time. The first and second times may be the same or different. In some cases, the first and second times may be different, and waking up the first user at an earlier time point may not disrupt sleep of the second user.

In some embodiments, the portion of the article of furniture may comprise a plurality of zones. The plurality of zones may comprise at least 2, 3, 4, 5, 6, 7, 8, 9, 10, or more zones. The plurality of zones may comprise at most 10, 9, 8, 7, 6, 5, 4, 3, or 2 zones. In some examples, the portion of the article of furniture comprises a first zone and a second zone, and the temperature control device may be configured to independently change a temperature of each of the first and second zones. In such cases, the processor may be configured to independently: (i) designate, while a first user is asleep on the first zone of the article of furniture, a first time for the article of furniture to wake up the first user based on a first biological of the first user detected by the at least one sensor, and change a temperature of the first zone of the article of furniture prior to the first time, and (ii) designate, while a second user is asleep on the second zone of the article of furniture, a second time for the article of furniture to wake up the second user based on a second biological of the second user detected by the at least one sensor, and change a temperature of the second zone of the article of furniture prior to the second time.

In some cases, the subject system for regulating a temperature of an article of furniture to wake up a user of the article of furniture may utilize any one of the subject articles of furniture (or any one of the subject bed device) of the present disclosure, e.g., as illustrated in FIGs. 1-4 and FIGs. 23-24.

In one aspect, the present disclosure provides a method of regulating a temperature of an article of furniture (e.g., a portion of the article of furniture) to wake up a user of the article of furniture. The method may comprise providing (i) at least one sensor that is a part of the article of furniture, wherein the at least one sensor is configured to detect a biological signal of a user of the article of furniture, (ii) a temperature control device coupled to the portion of the article of furniture, wherein the temperature control device is configured to change the temperature of the portion of the article of furniture, and (iii) a processor communicatively coupled to the at least one sensor and the temperature control device. The method may comprise, with aid of the at least one sensor, detecting the biological signal of the user of the article of furniture while the user is using the article of furniture. The method may comprise, with aid of the processor, designating, while the user is asleep on the article of furniture, a time for the article of furniture to wake up the user based at least in part on the detected biological signal of the user. The method may comprise, with the aid of the processor, changing the temperature of the portion of the article of furniture by the temperature control device prior to the time.

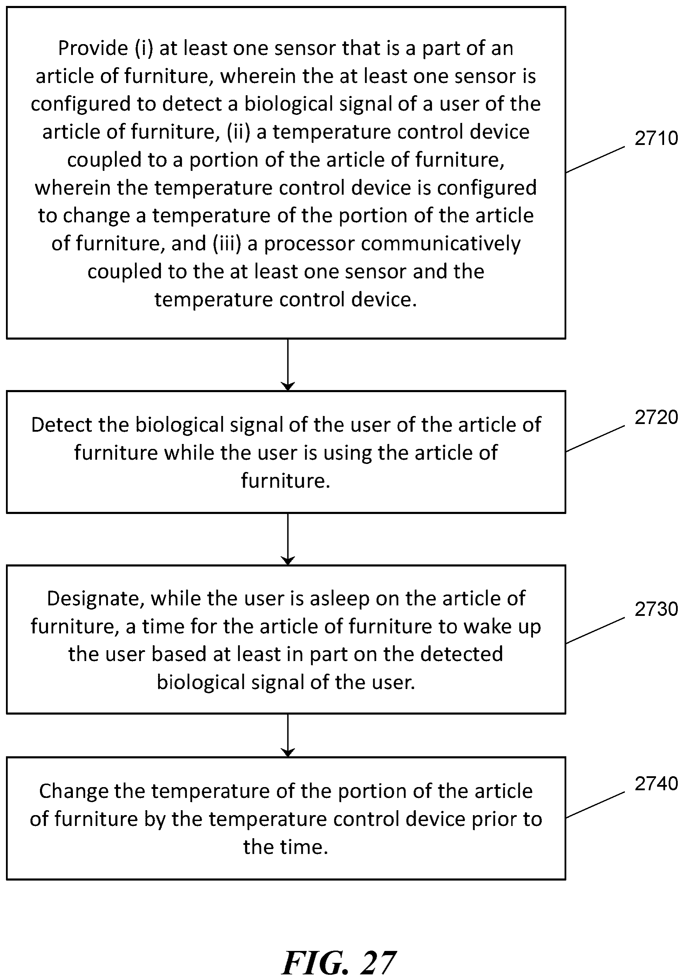

FIG. 27 illustrates an example of a method for regulating a temperature of a portion of an article of furniture. The method may comprise providing (i) at least one sensor that is a part of the article of furniture, wherein the at least one sensor is configured to detect a biological signal of a user of the article of furniture, (ii) a temperature control device coupled to the portion of the article of furniture, wherein the temperature control device is configured to change the temperature of the portion of the article of furniture, and (iii) a processor communicatively coupled to the at least one sensor and the temperature control device (process 2710). The method may comprise, with aid of the at least one sensor, detecting the biological signal of the user of the article of furniture while the user is using the article of furniture (process 2720). The method may comprise, with aid of the processor, designating, while the user is asleep on the article of furniture, a time for the article of furniture to wake up the user based at least in part on the detected biological signal of the user (process 2730). The method may comprise, with the aid of the processor, changing the temperature of the portion of the article of furniture by the temperature control device prior to the time (process 2740).

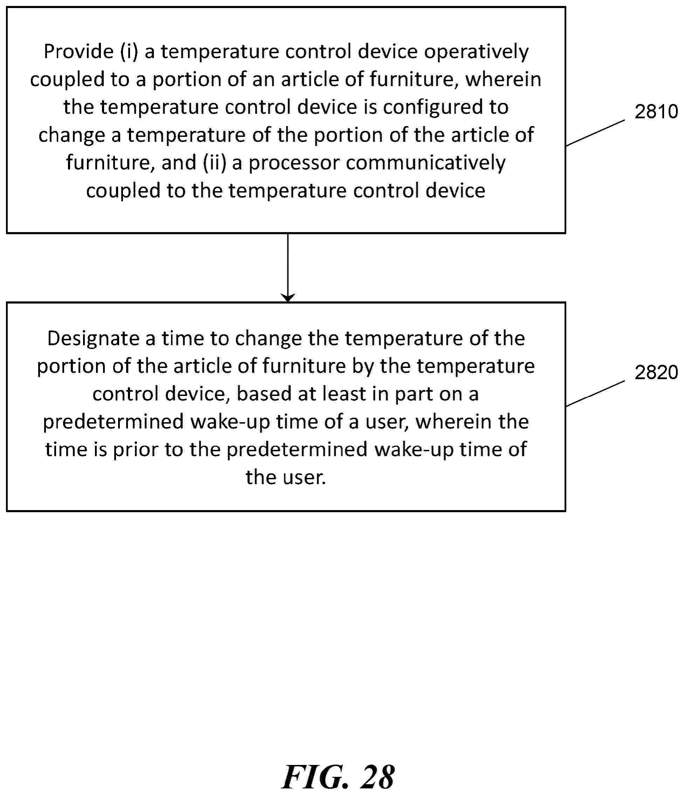

FIG. 28 illustrates an additional example of a method for regulating a temperature of a portion of an article of furniture. The method may comprise providing (i) a temperature control device operatively coupled to the portion of the article of furniture, configured to change the temperature of the portion of the article of furniture, and (ii) a processor communicatively coupled to the temperature control device (process 2810). The method may comprise with aid of the processor, designating a time to change the temperature of the portion of the article of furniture by the temperature control device based at least in part on a predetermined wake-up time of a user, wherein the time is prior to the predetermined wake-up time of the user (process 2820).

### Temperature control device

In one aspect, the present disclosure provides a system for regulating a temperature of an article of furniture, the system comprising: at least a portion of the article of furniture configured to hold a fluid; a reservoir in fluid communication with the at least the portion of the article of furniture, configured to contain the fluid; a temperature regulator in fluid communication with the at least the portion of the article of furniture and the reservoir, configured to modulate a temperature of the fluid; and a processor operatively coupled to the temperature regulator, programmed to control the temperature regulator to modulate the temperature of the fluid, thereby to regulate the temperature of the at least the portion of the article of furniture.

The article of furniture may comprise a bed or a seat. The bed may comprise a mattress, a mattress pad (i.e., a mattress cover), a blanket, a functional variant thereof, or a combination thereof. The mattress may be used alone or in combination with the mattress pad. The mattress pad may be used alone or in combination with the mattress. The mattress pad may cover at least a portion of the mattress. The mattress may be of different shapes (e.g., spherical, cylindrical, box, etc.). The mattress may have one or more sides (e.g., at least about 1, 2, 3, 4, 5, 6, 7, 8, 9, 10, or more sides). The mattress pad may be on or adjacent one or more sides of the mattress. In an example, the mattress pad may cover a top side of the mattress. In another example, the mattress pad may cover all sides of the mattress. The seat may be at least a portion (e.g., a portion of an area, a layer of a plurality of layers, etc.) of a larger article of furniture, such as, for example, a chair, loveseat, sofa, couch, stool, ottoman, bench, or modifications thereof.

The temperature of the at least the portion of the article of furniture may be regulated (e.g., by the fluid in the at least the portion of the article of furniture). Regulating the temperature of the at least the portion of the article of furniture may comprise maintaining at a pre-determined temperature or range of temperatures, increasing the temperature, and/or decreasing the temperature. The temperature of the at least the portion of the article of furniture may range between about 10°C to about 50°C. The temperature of the at least the portion of the article of furniture may be at least about 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 35°C, 40°C, 45°C, 50°C, or more. The temperature of the at least the portion of the article of furniture may be at most about 50°C, 45°C, 40°C, 35°C, 30°C, 29°C, 28°C, 27°C, 26°C, 25°C, 24°C, 23°C, 22°C, 21°C, 20°C, 19°C, 18°C, 17°C, 16°C, 15°C, 14°C, 13°C, 12°C, 11°C, 10°C, or less. In some cases, the temperature of the at least the portion of the article of furniture that is sensed (felt) by one or more users of the article of furniture may range between about 13°C to about 44°C. The temperature of the at least the portion of the article of furniture may increase and/or decrease by an increment of at least about 0.1°C, 0.2°C, 0.3°C, 0.4°C, 0.5°C, 0.6°C, 0.7°C, 0.8°C, 0.9°C, 1°C, 2°C, 3°C, 4°C, 5°C, or more. The temperature of the at least the portion of the article of furniture may increase and/or decrease by an increment of at most about 5°C, 4°C, 3°C, 2°C, 1°C, 0.9°C, 0.8°C, 0.7°C, 0.6°C, 0.5°C, 0.4°C, 0.3°C, 0.2°C, 0.1°C, or less.

In some cases, a pre-determined range of temperatures of the article of furniture suitable for an adult may range between about 14°C to about 20°C (e.g., teenagers or older). The pre-determined range of temperatures of the article of furniture suitable for the adult may be at least about 14°C, 14.5°C, 15°C, 15.5°C, 16°C, 16.5°C, 17°C, 17.5°C, 18°C, 18.5°C, 19°C, 19.5°C, 20°C, or more. The pre-determined range of temperatures of the article of furniture suitable for the adult may be at most about 20°C, 19.5°C, 19°C, 18.5°C, 18°C, 17.5°C, 17°C, 16.5°C, 16°C, 15.5°C, 15°C, 14.5°C, 14°C, or less. The pre-determined range of temperatures of the article of furniture suitable for a baby (e.g., 0 to 12 months old) or a toddler (e.g., 12 to 36 months old) may range between about 17°C to about 22°C. The pre-determined range of temperatures of the article of furniture suitable for the baby or toddler may be at least about 17°C, 17.5°C, 18°C, 18.5°C, 19°C, 19.5°C, 20°C, 20.5°C, 21°C, 21.5°C, 22°C, or more. The pre-determined range of temperatures of the article of furniture suitable for the baby or toddler may be at most about 22°C, 21.5°C, 21°C, 20.5°C, 20°C, 19.5°C, 19°C, 18.5°C, 18°C, 17.5°C, 17°C, or less. A pre-determined temperature and/or an average of a pre-determined range of temperatures for the baby or toddler may be the same, higher, or lower than the pre-determined temperature and/or the average of the pre-determined range of temperatures for the adult, respectively.

The at least the portion of the article of furniture may be configured to transfer (e.g., add or remove) heat between the at least the portion of the article of furniture and a user of the system that is on or adjacent to the at least the portion of the article of furniture. The user may be sitting, lying down, and/or sleeping on the article of furniture, such as, for example, the bed. The user may be sitting on the article of furniture, such as, for example, the seat. A temperature of a bodily surface or an internal temperature of the user of the article of furniture may be maintained, increased, or decreased to a pre-determined temperature (or range of temperatures) by the transferred heat.

The at least the portion of the article of furniture may be configured to hold the fluid. Alternatively or in addition to, the at least the portion of the article of furniture may be configured to permit flow of the fluid through, underneath, over, or adjacent to the at least the portion of the article of furniture. The fluid may be a liquid or gas. The liquid may comprise aqueous liquid (e.g., water) or non-aqueous liquid (e.g., oil). The gas may comprise air or argon. The fluid may be configured to be heated or cooled. A temperature of the fluid may be regulated (e.g., by the temperature regulator). The regulated temperature of the fluid may range between about 10°C to about 50°C. The regulated temperature of the fluid may be at least about 10°C, 11°C, 12°C, 13°C, 14°C, 15°C, 16°C, 17°C, 18°C, 19°C, 20°C, 25°C, 30°C, 35°C, 40°C, 45°C, 50°C, or more. The regulated temperature of the fluid may be at most about 50°C, 45°C, 40°C, 35°C, 30°C, 25°C, 20°C, 19°C, 18°C, 17°C, 16°C, 15°C, 14°C, 13°C, 12°C, 11°C, 10°C, or less.

The temperature of the fluid may increase and/or decrease (e.g., by the temperature regulator) by an increment of at least about 0.1°C, 0.2°C, 0.3°C, 0.4°C, 0.5°C, 0.6°C, 0.7°C, 0.8°C, 0.9°C, 1°C, 2°C, 3°C, 4°C, 5°C, or more. The temperature of the fluid may increase and/or decrease by an increment of at most about 5°C, 4°C, 3°C, 2°C, 1°C, 0.9°C, 0.8°C, 0.7°C, 0.6°C, 0.5°C, 0.4°C, 0.3°C, 0.2°C, 0.1°C, or less.

The temperature of the fluid may increase and/or decrease (e.g., by the temperature regulator) at a rate ranging between about 0.01°C per minute (°C/min) to about 5°C/min. The temperature of the fluid may increase and/or decrease at a rate of at least about 0.01°C/min, 0.02°C/min, 0.03°C/min, 0.04°C/min, 0.05°C/min, 0.06°C/min, 0.07°C/min, 0.08°C/min, 0.09°C/min, 0.1°C/min, 0.2°C/min, 0.3°C/min, 0.4°C/min, 0.5°C/min, 0.6°C/min, 0.7°C/min, 0.8°C/min, 0.9°C/min, 1°C/min, 2°C/min, 3°C/min, 4°C/min, 5°C/min, or more. The temperature of the fluid may increase and/or decrease at a rate of at most about 5°C/min, 4°C/min, 3°C/min, 2°C/min, 1°C/min, 0.9°C/min, 0.8°C/min, 0.7°C/min, 0.6°C/min, 0.5°C/min, 0.4°C/min, 0.3°C/min, 0.2 °C/min, 0.1°C/min, 0.09°C/min, 0.08°C/min, 0.07°C/min, 0.06°C/min, 0.05°C/min, 0.04°C/min, 0.03°C/min, 0.02°C/min, 0.01°C/min, or less.

The fluid may be capable of maintaining at a set temperature for about 0.1 hour to about 10 hours. The fluid may be capable of maintaining at a set temperature for at least about 0.1 hour, 0.2 hours, 0.3 hours, 0.4 hours, 0.5 hours, 0.6 hours, 0.7 hours, 0.8 hours, 0.9 hours, 1 hours, 1.5 hours, 2 hours, 3 hours, 4 hours, 5 hours, 6 hours, 7 hours, 8 hours, 9 hours, 10 hours, or more. The fluid may be capable of maintaining at a set temperature for at most about 10 hours, 9 hours, 8 hours, 7 hours, 6 hours, 5 hours, 4 hours, 3 hours, 2 hours, 1.5 hours, 1 hours, 0.9 hours, 0.8 hours, 0.7 hours, 0.6 hours, 0.5 hours, 0.4 hours, 0.3 hours, 0.2 hours, 0.1 hours, or less.

The temperature of the fluid being held and/or flowing through the portion of the article of furniture may be indicative of the temperature of the portion of the article of furniture. The temperature of the portion of the article of furniture may be the same or substantially the same as the temperature of the fluid being held and/or flowing through the portion of the article of furniture. The temperature of the portion of the article of furniture may equilibrate to the temperature of the fluid being held and/or flowing through the portion of the article of furniture, if initially different, within a range of about 0.1 min to about 60 min. The temperature of the portion of the article of furniture may equilibrate to the temperature of the fluid being held and/or flowing through the portion of the article of furniture within at least about 0.1 min, 0.2 min, 0.3 min, 0.4 min, 0.5 min, 0.6 min, 0.7 min, 0.8 min, 0.9 min, 1 min, 2 min, min, 4 min, 5 min, min, min, 8 min, 9 min, 10 min, 20 min, 30 min, 40 min, 50 min, 60 min, or more. The temperature of the portion of the article of furniture may equilibrate to the temperature of the fluid being held and/or flowing through the portion of the article of furniture within at most about 60 min, 50 min, 40 min, 30 min, 20 min, 10 min, 9 min, 8 min, 7 min, 6 min, 5 min, 4 min, 3 min, 2 min, 1 min, 0.9 min, 0.8 min, 0.7 min, 0.6 min, 0.5 min, 0.4 min, 0.3 min, 0.2 min, 0.1 min, or less.

The temperature regulator may not be part of the reservoir. The temperature regulator may not be inside the reservoir nor configured to be in physical contact with the reservoir. The temperature regulator may be configured to modulate the temperature of the fluid that is not contained (e.g., outside of) the reservoir. The temperature regulator may comprise at least about one channel (e.g., at least 1, 2, 3, 4, 5, 6, 7, 8, 9, 10 channels) configured to hold the fluid and/or permit flow of the fluid. The at least one channel of the temperature regulator may be connected to each other. The at least one channel of the temperature regulator may be a thermoelectric engine. Alternatively or in addition to, the at least one channel of the temperature generator may be disposed on or adjacent to (e.g., in contact with) at least one thermal device (e.g., at least one thermoelectric engine), such that the at least one thermal device modulates a temperature of the at least one channel of the temperature generator, thereby to modulate the temperature of the fluid in the at least one channel of the temperature generator. In some cases, at least two thermal devices may be disposed on top of each other (e.g., stacked), adjacent to each other (e.g., in parallel or perpendicular), or opposite of each other (e.g., on opposite ends of the at least one channel of the temperature generator). In some cases, the at least one thermal device may be at least one thermoelectric engine. The temperature regulator may comprise at least one thermoelectric engine configured to modulate the temperature of the fluid. The temperature regulator may comprise at least about 1, 2, 3, 4, 5, 6, 7, 8, 9, 10, or more thermoelectric engines configured to modulate the temperature of the fluid. The temperature regulator may comprise at most about 10, 9, 8, 7, 6, 5, 4, 3, 2, or 1 thermoelectric engine configured to modulate the temperature of the fluid. Alternatively or in addition to, the temperature regulator may be part of the reservoir.

The system may comprise at least about 1, 2, 3, 4, 5, 6, 7, 8, 9, 10, or more temperature regulators. The system may comprise at most about 10, 9, 8, 7, 6, 5, 4, 3, 2, or 1 temperature regulator. A plurality of temperature regulators may or may not be in communication with each other. In some cases, the temperature regulators may or may not be part of the article of furniture.

The system may comprise at least about 1, 2, 3, 4, 5, 6, 7, 8, 9, 10, or more reservoirs. The system may comprise at most about 10, 9, 8, 7, 6, 5, 4, 3, 2, or 1 reservoir.

The reservoir may be configured to modulate the temperature of the fluid. In an example, the reservoir may comprise at least one thermal device (e.g., at least 1, 2, 3, 4, 5, 6, 7, 8, 9, 10, or more thermal devices) configured to modulate the temperature of the fluid contained in the reservoir. The at least one thermal device may be inside the reservoir and/or outside the reservoir (e.g., on or adjacent to an outer side wall of the reservoir). Alternatively or in addition to, the at least one thermal device may be part of the at least one side wall of the reservoir.

The reservoir may not be configured to modulate the temperature of the fluid. In such a case, the fluid may be drawn out of the reservoir (e.g., by a gravitational force, by an external force, such as, for example, an external pump), and a temperature of the drawn out fluid may be modulated (e.g., by the temperature generator that is not part of the reservoir). The reservoir may comprise at least one exit orifice for the fluid to be drawn out of the reservoir. The at least one exit orifice may be in fluid communication with the reservoir and another device that controls or allows flow of the fluid, such as a gate (e.g., a valve) and/or a pump. The reservoir may comprise at least about 1, 2, 3, 4, 5, 6, 7, 8, 9, 10, or more exit orifices for the fluid to be drawn out. In some cases, the fluid that is drawn out of the reservoir (e.g., through the at least one exit orifice) may be configured to re-enter the reservoir. In some cases, the fluid that is drawn out of the reservoir may not be configured to re-enter the reservoir.

The reservoir may or may not be sealed. In some cases, the reservoir may be sealed, and thus the fluid contained in the reservoir may be sealed off from ambient air outside the reservoir. Such sealed reservoir may slow down or prevent escape of the fluid (e.g., evaporation of the liquid) out of the reservoir. The reservoir may comprise at least one container configured to contain the fluid. The container may or may not be removable from the reservoir. The container may be a vat. The container may or may not have a lid. The lid may or may not be removable from the container. In some cases, the container may be sealed, thereby to slow down or prevent escape of the fluid (e.g., evaporation of the liquid) out of the reservoir.

The reservoir may not leak. The reservoir may be located above or below the height of the article of furniture (e.g., the mattress of the bed). The reservoir may be located approximately at the height of the article of furniture.

The reservoir may comprise one or more sensors to detect an amount of the fluid contained in the reservoir (e.g., contained in the container of the reservoir). The reservoir may comprise at least 1, 2, 3, 4, 5, or more of such sensors. The reservoir may comprise at most 5, 4, 3, 2, or 1 of such sensor. The sensor may comprise an electromagnetic radiation (e.g., visible light, ultraviolet light, infrared light, etc.) sensor. The sensor may be a camera. The sensor may be a water sensor.

The system may further comprise at least one pump configured to retrieve the fluid from the reservoir. The system may comprise at least about 1, 2, 3, 4, 5, 6, 7, 8, 9, 10, or more pumps. The system may comprise at most 10, 9, 8, 7, 6, 5, 4, 3, 2, or 1 pump. Such pump may be configured to operate via one or more energy sources, e.g., manual operation, electricity, engine, wind power, etc. Such pump may include a positive displacement pump, gear pump, screw pump, progressing cavity pump, roots-type pump, peristaltic pump, plunger pump, compressed-air-powered double-diaphragm pump, hydraulic pump, velocity pump, radial flow pump, axial flow pump, eductor jet pump, gravity pump, steam pump, valveless pump, etc. The at least one pump may be in fluid communication with one or more reservoirs, a container from each of the reservoir(s), one or more temperature regulators, and/or one or more portions of the article of furniture. The at least one pump may be configured to direct flow of the fluid between the at least one pump and the reservoir. The at least one pump may be configured to direct flow of the fluid from the pump, through the temperature regulator, and to the pump. The at least one pump may be configured to prevent flow of the fluid from the at least one pump to the reservoir. Alternatively or in addition to, the at least one pump may be configured to allow flow of the fluid from the at least one pump to the reservoir. The pump may be configured to separate the fluid in the temperature regulator from the fluid contained in the reservoir. Alternatively or in addition to, the pump may be configured to allow passage of the fluid in the temperature regulator back into the reservoir. In some cases, the pump may be configured to direct flow of the fluid from the pump, through the temperature regulator, through the portion of the article of furniture, and to the pump. Alternatively or in addition to, the pump may be configured to direct flow of the fluid from the pump, through the portion of the article of furniture, through the temperature regulator, and to the pump.

The processor may be coupled to the at least one pump and programmed to control the at least one pump to retrieve the fluid from the reservoir. The processor may be further configured to control the at least one pump to direct flow of the fluid between the at least one pump and the reservoir. The processor may be further configured to control the at least one pump to direct flow of the fluid from the at least one pump, through the temperature regulator, and to the at least one pump.

The system may comprise at least one gate disposed between the reservoir and the temperature regulator. The system may comprise at least about 1, 2, 3, 4, 5, 6, 7, 8, 9, 10, or more gates. The system may comprise at most about 10, 9, 8, 7, 6, 5, 4, 3, 2, or 1 gate. The gate may be configured to control flow of the fluid between the reservoir and the temperature regulator. The gate may be configured to control flow of the fluid away from the reservoir and towards the temperature regulator. The gate may be configured to prevent flow of the fluid away from the temperature regulator and towards the reservoir. Alternatively or in addition to, the gate may be configured to allow flow of the fluid away from the temperature regulator and towards the reservoir. In some cases, the pump may be disposed between the reservoir and the temperature regulator, and the gate may be disposed between the reservoir and the pump. Such gate may be configured to control flow of the fluid between the reservoir and the pump. The gate may be configured to control flow of the fluid away from the reservoir and towards the pump. The gate may be configured to prevent flow of the fluid away from the pump and towards the reservoir. Alternatively or in addition to, the gate may be configured to allow flow of the fluid away from the pump and towards the reservoir. The gate may be in fluid communication with the reservoir(s), the pump(s), the temperature regulator(s), and/or the portion(s) of the article of furniture.

The gate may comprise at least about 1, 2, 3, 4, 5, or more orifices (e.g., ports) that allow flow of the fluid in and/or out of the gate. The gate may comprise at most about 5, 4, 3, 2, or 1 orifice. In some cases, the gate may be a one-way gate, two-way gate, three-way gate, or four-way gate. The gate may be a valve. The valve may be a check valve, clack valve, non-return valve, reflux valve, retention valve or one-way valve. In some cases, the gate may be a gravitational gate (e.g., a gravitational valve). The gravitational gate may use a force of gravity to draw the fluid away from the reservoir (e.g., out of the reservoir) and towards the pump and/or the temperature regulator.

The gate may further comprise an air purge orifice. The air purge orifice may be coupled to an air purge channel. The air purge orifice and/or the air purge channel may be configured to purge (or remove) air in the gate and/or any other components (e.g., one or more channels) of the system that is configured to hold or permit flow of the fluid. The air purge orifice and/or the air purge channel may prevent leakage of the fluid from the system. In some cases, the gate may be in fluid communication with (i) the air purge channel, (ii) a channel that allows fluid flow between the gate and the portion of the article of furniture, (iii) the channel that allows fluid flow between the gate and the reservoir, and (iv) the channel that allows fluid flow between the gate and the pump. In some cases, the abovementioned four channels may be coupled to the gate vertically, in a descending order (e.g., from top to bottom) of (i) the air purge channel, (ii) the gate-article of furniture channel, (iii) the gate-reservoir channel), and (iv) the gate-pump channel.

The portion of the article of furniture may comprise at least one channel configured to hold the fluid and/or permit flow of the fluid. The portion of the article of furniture may comprise at least 1, 2, 3, 4, 5, 6, 7, 8, 9, 10, or more channels. The portion of the article of furniture may comprise at most 10, 9, 8, 7, 6, 5, 4, 3, 2, or 1 channel. The channel(s) of the portion of the particle of furniture may comprise a plurality of interconnected channels configured to hold the fluid and/or permit flow of the fluid. The plurality of interconnected channels may be in a mesh (or porous) network structure, thereby to help the article of furniture to breathe. The channel(s) may be a fluid circulating mat (e.g., a water circulating mat).

The portion of the article may comprise an entry orifice for the fluid to enter flow the portion of the article (e.g., from the gate, pump, and/or the temperature regulator). The entry orifice may be in fluid communication with the gate, pump, and/or the temperature regulator. The portion of the article may comprise an exit orifice for the fluid to flow out of the portion of the article (e.g., towards the gate, pump, and/or the temperature regulator). The exit orifice may be in fluid communication with the gate, pump, and/or the temperature regulator. The entry orifice and/or the exit orifice may comprise a gate (e.g., a valve) to allow or prevent flow of the fluid.

The article of furniture may comprise at least about 1, 2, 3, 4, 5, 6, 7, 8, 9, 10, or more portions. The article of furniture may comprise at most about 10, 9, 8, 7, 6, 5, 4, 3, 2, or 1 portion. Each of a plurality of portions of the article of furniture may correspond to a zone for each user to seat, rest, or sleep on. Each of the plurality of portions of the article of furniture may correspond to different areas that would be in contact or adjacent to different portions of a user's body (e.g., feet, legs, butt, arms, back, neck, head, etc.). Temperatures of the plurality of portions of the article of furniture may be regulated independently or in unison. In an example, different zones of the bed may be set (e.g., by the processor) at different temperatures for different users. In another example, different zones of the bed may be set (e.g., by the processor) at different temperatures for different bodily parts of a user.

The system may further comprise an additional portion of the article of furniture configured to hold the fluid. The portion of the article of furniture and the additional portion of the article of furniture may be different. The additional portion of the article of furniture may be in fluid communication with the temperature regulator. Alternatively, the additional portion of the article of furniture may be in fluid communication with an additional temperature regulator configured to modulate the temperature of the fluid. The temperature regulator and the additional temperature regulator may be different. The temperature regulator and the additional temperature regulator may not be in fluid communication with each other. Alternatively or in addition to, the temperature regulator and the additional temperature regulator may be in fluid communication with each other. The additional temperature regulator may be in fluid communication with the reservoir. The temperature regulator and the additional temperature regulator may be in fluid communication with a common (or a same) reservoir.

The processor may be operatively coupled to the additional temperature regulator. The processor may be further programmed to control the additional temperature regulator to modulate the temperature of the fluid, thereby to regulate a temperature of the additional portion of the article of furniture. The processor may be further programmed to independently control the temperature regulator and the additional temperature regulator, thereby to independently regulate the temperature of the portion of the article of furniture and the temperature of the additional portion of the article of furniture. The processor may be further programmed to control the temperature regulator and the additional temperature regulator in union, thereby to regulate the temperature of the portion of the article of furniture and the temperature of the additional portion of the article of furniture in unison.

The system may further comprise a sensor to detect a property of the fluid. The sensor may be a temperature sensor. The sensor may be in direct or indirect contact with the fluid. The sensor may be part of the gate (e.g., the valve), the pump, the temperature regulator, the portion of the article of furniture, or one or more channels (e.g., a water loop) configured to hold and/or allow flow of the fluid.

The system may further comprise at least one heat sink configured to absorb heat from its surrounding. The at least one heat sink may be disposed on or adjacent to the temperature regulator (e.g., the thermoelectric engine). The system may comprise at least about 1, 2, 3, 4, 5, 6, 7, 8, 9, or 10 heat sinks. The system may comprise at most 10, 9, 8, 7, 6, 5, 4, 3, 2, or 1 heat sink. The heat sink(s) may be configured to absorb heat from the temperature regulator.

The system may further comprise at least one fan (e.g., a dual fan) configured to regulate temperature of one or more components of the system. The system may comprise at least 1, 2, 3, 4, 5, 6, 7, 8, 9, 10, or more fans. The system may comprise at most 10, 9, 8, 7, 6, 5, 4, 3, 2, or 1 fan. The fan(s) may be configured to blow or pull air across the heat sink(s) to regulate temperature of the heat sink(s). Operation of the fan(s) may not impact the operation of the temperature regulator to modulate the temperature of the fluid. Operation of the fan(s) may not impact the operation of the sensor (e.g., the temperature sensor) configured to detect the property (e.g., temperature) of the fluid.

The system may further comprise an additional portion of the article of furniture that includes at least one sensor that is (i) operatively coupled to the processor and (ii) configured to detect a biological signal of at least one user of the article of furniture. The biological signal comprises a heart signal (e.g., a heart rate), a respiration signal (e.g., a respiration rate), a motion, a temperature, and/or perspiration of the at least one user of the article of furniture. The processor may be configured to determine a shape of the heart signal, at least based in part on the amplitude and/or frequency of the heart signal. The processor may be configured to determine a shape of the respiration signal, at least based in part on the amplitude and/or frequency of the respiration signal.

The channel(s) disclosed herein (e.g., the channel(s) configured at least to hold the fluid and/or permit flow of the fluid) may comprise fluid-insoluble (e.g., water-insoluble) materials.
The channel(s) may comprise a polymeric material, metallic material, ceramic material, any functional modification thereof, or any combination thereof. Examples of the polymeric material include polyvinyl acetate, polyvinyl chloride, polyvinyl carbonate, ethyl cellulose, nitrocellulose, vinylidene chloride-acrylonitrile copolymer, acrylonitrile-styrene copolymer, ethylene vinyl acetate, cellulose acetate, cellulose acetate phthalate, cellulose acetate butyrate, copolymer of vinyl pyrrolidone, hydroxypropylmethylcellulose phthalate, methacrylic acid copolymer, methacrylate copolymer, any functional modification thereof, or any combination thereof.

The processor may be further programmed to control the temperature regulator to modulate the temperature of the fluid based on the detected biological signal of the at least one user. The processor may control the temperature regulator to modulate the temperature of the fluid, such that the temperature of the fluid (and/or the temperature of the portion of the article of furniture) may be the same, substantially the same, lower, and/or higher than a detected temperature of the at least one user. The processor may be further programmed to (i) identify the at least one user based on the detected biological signal of the at least one user, and/or (ii) control the temperature regulator to modulate the temperature of the fluid based on the at least one user's identity. The at least one user's identity may comprise age, gender, physical condition, geolocation, a predetermined temperature of the portion of the article of furniture, a predetermined temperature range of the portion of the article of furniture, or a history of the biological signal of the at least one user while using the article of furniture (e.g., an average temperature of the fluid while the user is sleeping on the bed, or an average temperature of the user while the user is sleeping on the bed).

The processor may be further configured to modulate the temperature of the fluid, thus the temperature of the portion of the article of furniture, based on the identity of the user. The processor may be programmed to determine that the same user has been using (e.g., sleeping on) the portion of the article of furniture for one or more days (e.g., at least about 1, 2, 3, 4, 5, 6, 7, 8, 9, 10, or more days). On a following day, the processor may be programmed to modulate the temperature of the fluid (e.g., by the temperature generator) prior to a predicted time of use by the same user (e.g., an average time that the user has started using the article of furniture for the past one or more days). The processor may pre-warm or pre-cool the fluid, thereby to pre-warm or pre-cool the portion of the article of furniture. Pre-warming or precooling the portion of the article of furniture may help equilibration between the temperature of the portion of the article of furniture and the temperature of the user. Alternatively or in addition to, the user may pre-set a desired temperature and a desired time for the control to pre-adjust the temperature of the portion of the article of furniture to the desired temperature at the desired time.

The system may comprise a sensor operatively coupled to the processor and configured to detect a biological signal of at least one user of the article of furniture. Such sensor may not be part of the article of furniture. The sensor may be a smart watch or a fitness tracker. The at least one user may be wearing the sensor. The processor may be further configured to modulate the temperature of the fluid based on the detected biological signal of the at least one user. In some cases, the biological signal may be a temperature of the at least one user, and the processor may be further configured to modulate (e.g., increase or decrease) a temperature difference between the temperature of the fluid (e.g., the that is being held or flowing through the portion of the article of furniture) and the temperature of the at least one user. In some cases, the processor may be further programmed to modulate the temperature of the fluid prior to usage of the article of furniture by the at least one user, thereby to pre-adjust the temperature of the portion of the article of furniture prior to use of the article of furniture by the at least one user.

The processor may be further configured to modulate the temperature of the fluid based on the detected biological signal of the at least one user, thereby to regulate duration of sleep (e.g., sleeping longer, or waking up faster) of the at least one user. The processor may be further programmed to modulate the temperature of the fluid based on the detected biological signal of the at least one user, thereby to regulate metabolism (e.g., help the user burn more fat while sleeping) of the at least one user. The processor may be further configured to apply a preset temperature setting (or temperature profile) to the temperature regulator, thereby to apply a preset temperature setting to the portion of the article of furniture. The preset temperature setting may be based on a bio feedback of the user. The biofeedback may be provided by the user or determined by the processor using the user's detected biological signal and/or identity. Examples of the bio feedback include pregnancy, menopause, fever, illness, fatigue, cancer, sleep disorder, heart conditions, or other physical conditions.

The processor may be further programmed to monitor (i) the biological signal of the at least one user, (ii) a sleep pattern of the at least one user based on the detected biological signal of the at least one user over a period of time, and/or (iii) a temperature setting of the portion of the article of furniture over the period of time. The processor may be further configured to compare the biological signal, the sleep pattern, and/or the temperature setting between two or more users. In an example, the processor may compare and identify two or more users with similar or approximately the same sleep pattern, and compare the temperature settings of the portion of the article of furniture (e.g., a record of the temperature of the fluid that was heated and cooled) of the two or more users. The processor may be further configured to start a group of two or more users based on the comparison of the biological signal, the sleep pattern, and/or the temperature setting. In an example, the processor may start a group of two or more users with a similar biological signal (e.g., a similar heart signal that indicates a heart condition, such as, for example, heart arrhythmia, atrial fibrillation, etc.). Within the created group, the processor may compare each user's sleep pattern and the temperature settings of the portion of the article of furniture, and determine which temperature setting seems to yield a most desirable biological signal (e.g., a more regular hear signal or respiration signal) and/or sleep pattern (e.g., falling asleep faster, moving less, sleeping longer, waking up fewer times. Subsequently, the processor may be programmed to apply (e.g., automatically apply) the temperature setting of the article of furniture of a user of the group to a temperature setting of the article of furniture of another user of the group. Alternatively or in addition to, the processor may suggest to a user such application of the temperature setting of a different user (e.g., for an improved sleep quality). The processor may utilize a user interface (e.g., a graphical user interface, or GUI) on the user's personal device (e.g., a mobile phone, smart phone, smart watch, smart glass, etc.) to allow the two or more users of the created group to communicate and share information (e.g., voice, text, images, videos, etc.). Such group may serve as a support group.

In some cases, the processor may be further configured to connect (i) the user and any data collected and/or created by the processor for the user and (ii) a physician. The physician may be able to use the user interface on the physician's personal device to evaluate (i) the biological signal of the at least one user, (ii) a sleep pattern of the at least one user based on the detected biological signal of the at least one user over a period of time, and/or (iii) a temperature setting of the portion of the article of furniture over the period of time. The processor may utilize the GUI on the user's personal device and the physician's personal device to allow the user and the physician to communicate and share information (e.g., voice, text, images, videos, etc.). Such GUI may reduce a time for the user to consult with a doctor to discuss the user's biological signal, sleep pattern, and/or physical condition.

The processor may be capable of employing artificial intelligence (e.g., one or more machine learning algorithms) to analyze a database comprising a plurality of biological signals, sleep patterns, and/or temperature settings of the article of furniture of a plurality of users. One or more machine learning algorithms of the artificial intelligence may be capable of comparing a plurality of data within the database, and creating a group of two or more users based on the comparison.

The processor may be operatively coupled to other components and their configurations described in the aforementioned system for regulating the temperature of the portion of the article of furniture.

One or more components described in the aforementioned system for regulating the temperature of the portion of the article of furniture may be enclosed in a temperature regulating tower. In some cases, the temperature regulating tower may comprise one or more reservoirs, one or more valve(s), one or more temperature regulators, one or more pumps, or a combination thereof. The components of the temperature regulating tower may be in fluid communication (directly or indirectly) with each other. The temperature regulating tower may be in fluid communication with the article of furniture, such as, for example, one or more portions of the article of furniture (e.g., at least 1, 2, 3, 4, 5, 6, 7, 8, 9, 10, or more portions of the article of furniture). In some cases, the temperature regulating tower may be in fluid communication with a plurality of articles of furniture (e.g., at least 2, 3, 4, 5, 6, 7, 8, 9, 10, or more beds). In an example, a common temperature regulating tower that comprises a common reservoir and two or more temperature regulators may be in fluid communication with two or more articles of furniture to regulate (independently or in unison) temperatures of the two or more articles of furniture. In another example, a common temperature regulating tower that comprises a common reservoir and a plurality of temperature regulators may be in fluid communication with a plurality of beds (e.g., a plurality of babyterms) to regulate (independently or in unison) temperatures of the plurality of beds. In some cases, an article of furniture may be in fluid communication with one or more temperature regulating towers.

In one aspect, the present disclosure provides a method for regulating a temperature of an article of furniture, the method comprising: (a) providing a temperature regulator in fluid communication with (i) the portion of the article of furniture capable of holding a fluid, and (ii) a reservoir capable of containing the fluid, wherein the temperature regulator is capable of modulating a temperature of the fluid; and (b) controlling, by a computer system, the temperature regulator to modulate the temperature of the fluid, thereby regulating the temperature of the portion of the article of furniture. The method disclosed herein may utilize all components, configurations, and uses described in the aforementioned systems for regulating the temperature of the article of furniture.

The method may further comprise controlling, by the computer system, the temperature regulator to modulate the temperature of the fluid that is not in the reservoir (or not in the container of the reservoir). The temperature of the fluid may or may not be modulated in the reservoir.

The computer system may comprise a computer program product comprising a non-transitory computer-readable medium having computer-executable code encoded therein, the computer-executable code adapted to be executed to implement the abovementioned method regulating the temperature of the article of furniture.

In one aspect, the present disclosure provides a system for regulating a temperature of an article of furniture, the system comprising: the article of furniture comprising a first portion and a second portion, wherein each of the first and second portions is configured to hold a fluid; a common temperature controller configured to modulate a temperature of the fluid, wherein the common temperature controller comprises (i) a first channel in fluid communication with the first portion of the article of furniture, and (ii) a second channel in fluid communication with the second portion of the article of furniture, wherein the first and second channels are configured to hold the fluid; and a processor operatively coupled to the common temperature controller, programmed to control the common temperature controller to modulate the temperature of the fluid, thereby to independently regulate a first temperature of the first portion of the article of furniture and a second temperature of the second portion of the article of furniture. The system disclosed herein may utilize all components, configurations, and uses described in the aforementioned systems and methods for regulating the temperature of the article of furniture.

The first and second portions of the article of furniture may be different. In use, the first and second portions of the article of furniture may be used (e.g., occupied) by a common user (or a same user). Alternatively or in addition to, in use, the first and second portions of the article of furniture may be used (e.g., occupied) by different users.

The common temperature controller may comprise a reservoir in fluid communication with the first and second channels of the common temperature controller, which reservoir may be configured to contain the fluid. The reservoir may or may not be configured to modulate the temperature of the fluid.

The common temperature controller may comprise (i) a first temperature regulator in fluid communication with the first channel and configured to modulate the temperature of the fluid, and/or (ii) a second temperature regulator in fluid communication with the second channel and configured to modulate the temperature of the fluid. The first and second temperature regulators may or may not be part of the reservoir. The first and/or second temperature regulator may be a thermoelectric engine. The first temperature generator and the second temperature generator may or may not be in fluid communication with each other.

The common temperature controller may comprise (i) a first pump in fluid communication with the first channel, configured to direct flow of the fluid between the first channel and the first portion of the article of furniture, and/or (ii) a second pump in fluid communication with the second channel, configured to direct flow of the fluid between the second channel and the second portion of the article of furniture. The first pump may be in fluid communication (e.g., via at least the first channel of the common temperature controller) with the reservoir, the first temperature regulator, and/or the first portion of the article of furniture. The second pump may be in fluid communication (e.g., via at least the second channel of the common temperature regulator) with the reservoir, the second temperature regulator, and/or the second portion of the article of furniture. The first pump and the second pump may or may not be in communication with each other.

The common temperature controller may comprise (i) a first gate disposed between the reservoir and the first temperature regulator, which first gate is configured to prevent flow of the fluid away from the first temperature regulator and towards the reservoir, and/or (ii) a second gate disposed between the reservoir and the second temperature regulator, which second gate is configured to prevent flow of the fluid away from the second temperature regulator and towards the reservoir. In some cases, the first gate may be disposed between the reservoir and the first pump, which first pump is disposed between the first pump and the first temperature regulator. In some cases, the second gate may be disposed between the reservoir and the second pump, which second pump is disposed between the second gate and the second temperature generator. The first gate may be in fluid communication (e.g., via at least the first channel of the common temperature controller) with the reservoir, the first pump, the first temperature generator, and/or the first portion of the article of furniture. The second gate may be in fluid communication (e.g., via at least the second channel of the common temperature regulator) with the reservoir, the second pump, the second temperature generator, and/or the second portion of the article of furniture. The first gate and the second gate may or may not be in communication with each other.

In one aspect, the present disclosure provides a method for regulating a temperature of an article of furniture, the method comprising: (a) providing a common temperature controller configured to modulate a temperature of a fluid, wherein the common temperature controller comprises (i) a first channel in fluid communication with a first portion of the article of furniture, and (ii) a second channel in fluid communication with a second portion of the article of furniture, wherein the first and second portions of the article of furniture are configured to hold a fluid, and wherein the first and second channels are configured to hold the fluid; and (b) controlling the common temperature controller to modulate the temperature of the fluid, thereby independently regulating a first temperature of the first portion of the article of furniture and a second temperature of the second portion of the article of furniture. The method disclosed herein may utilize all components, configurations, and uses described in the aforementioned systems and methods for regulating the temperature of the article of furniture.

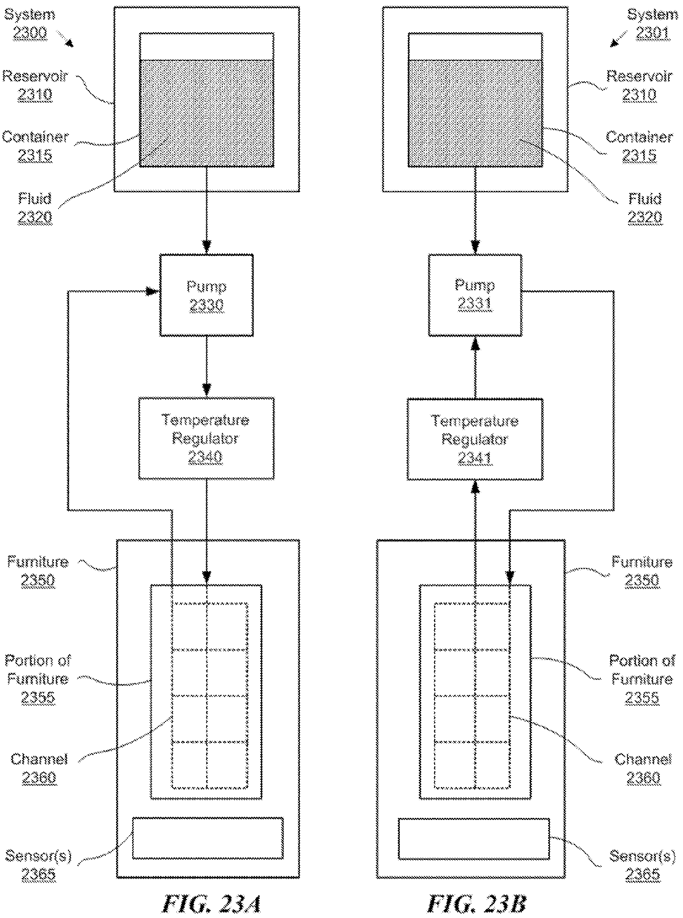

FIGs. 23A to 23H schematically illustrate examples of a system for regulating a temperature of an article of furniture (e.g., a bed, mattress, or mattress pad), which system comprises a fluid loop (e.g., one water loop). Referring to FIG. 23A, a system 2300 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2300 comprises a pump 2330 in fluid communication with the container 2315 of the reservoir 2310. The pump 2330 is configured to retrieve or receive the fluid 2320 from the container 2315 of the reservoir 2310. The pump 2330 is configured to prevent flow of the fluid 2320 away from the pump 2330 and back into the container 2315 of the reservoir 2310. The system 2300 comprises a temperature regulator 2340 that is in fluid communication with the pump 2330 (and thus, in indirect fluid communication with the container 2315 of the reservoir 2310). The temperature regulator 2340 is configured to modulate a temperature (e.g., maintain, increase, and/or decrease) of the fluid 2320. The temperature regulator 2340 may be a plurality of temperature regulators (or a plurality of temperature regulating units), wherein each of the plurality of temperature regulators is configured to modulate a temperature of the fluid 2320, in unison or independently of each other. The temperature regulator 2340 may comprise a thermoelectric engine. The pump 2330 is configured to (i) retrieve or receive the fluid 2320 from the container 2315 of the reservoir 2310, and (ii) direct flow of the fluid 2320 from the pump 2330 and to the temperature regulator 2340. The system 2300 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 is in fluid communication with the temperature regulator 2340 and the pump 2330. The pump 2330 is configured to direct flow of the fluid 2320 from the channel 2360 to the temperature regulator 2340. The fluid loop (e.g., the water loop) of the system 2300 comprises a flow of the fluid 2320 away from the pump 2330, to the temperature regulator 2340, to the channel 2360 of the portion of furniture 2355, and back to the pump 2330. The pump 2330 is configured to draw fluid 2320 out of the container 2315 of the reservoir 2310 and add the drawn fluid 2320 into the fluid loop. The pump 2330 separates (i) the fluid 2320 contained in the container 2315 of the reservoir 2310 from (ii) the fluid 2320 in, flowing through, and/or flowing adjacent to the temperature regulator 2340. The temperature regulator 2340 is not part of the reservoir 2310. The system 2300 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2300 may regulate the temperature of the portion of furniture 2355 based at least in part on the detected biological signal of the at least one user of the article of furniture 2350.

Referring to FIG. 23B, a system 2301 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2301 comprises a pump 2331 in fluid communication with the container 2315 of the reservoir 2310. The pump 2331 is configured to retrieve or receive the fluid 2320 from the container 2315 of the reservoir 2310. The pump 2331 is configured to prevent flow of the fluid 2320 away from the pump 2331 and back into the container 2315 of the reservoir 2310. The system 2301 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 is in fluid communication with the pump 2331. The pump 2331 is configured to (i) retrieve or receive the fluid 2320 from the container 2315 of the reservoir 2310, and (ii) direct flow of the fluid 2320 from the pump 2331 and to the channel 2360. The system 2301 comprises a temperature regulator 2341 that is in fluid communication with the channel 2360 and the pump 2331. The temperature regulator 2341 is configured to modulate a temperature (e.g., maintain, increase, and/or decrease) of the fluid 2320. The temperature regulator 2341 may be a plurality of temperature regulators (or a plurality of temperature regulating units), wherein each of the plurality of temperature regulators is configured to modulate a temperature of the fluid 2320, in unison or independently of each other. The temperature regulator 2341 may comprise a thermoelectric engine. The pump 2331 is configured to direct flow of the fluid 2320 from the temperature regulator 2341 to the channel 2360. The fluid loop (e.g., the water loop) of the system 2301 comprises a flow of the fluid 2320 away from the pump 2331, to the channel 2360 of the portion of furniture 2355, to the temperature regulator 2341, and back to the pump 2331. The pump 2331 is configured to draw fluid 2320 out of the container 2315 of the reservoir 2310 and add the drawn fluid 2320 into the fluid loop. The pump 2331 separates (i) the fluid 2320 contained in the container 2315 of the reservoir 2310 from (ii) the fluid 2320 in, flowing through, and/or flowing adjacent to the temperature regulator 2341. The temperature regulator 2341 is not part of the reservoir 2310. The system 2301 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2301 may regulate the temperature of the portion of furniture 2355 based at least in part on the detected biological signal of the at least one user of the article of furniture 2350.

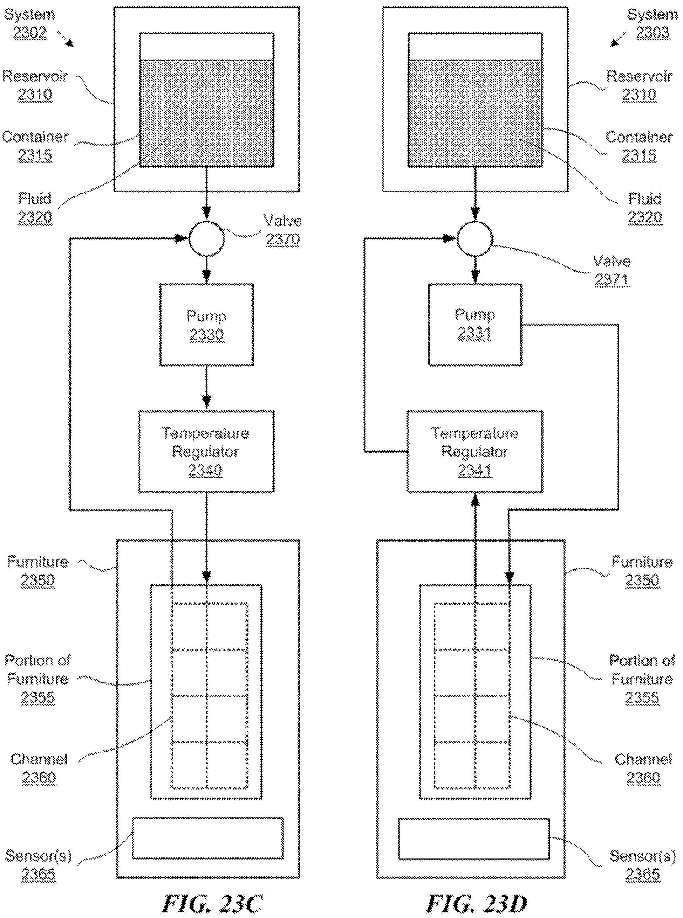

Referring to FIG. 23C, a system 2302 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2302 comprises a valve 2370 in fluid communication with the container 2315 of the reservoir 2310. The valve 2370 may be a gravitational valve that only allows a flow of the fluid 2320 in a direction away from the container 2315 of the reservoir 2310 and towards the valve 2370. The valve 2370 is configured to prevent flow of the fluid 2320 away from the valve 2370 and back into the container 2315 of the reservoir 2310. The system 2302 comprises a pump 2330 in fluid communication with the valve 2370. The pump 2330 is configured to retrieve or receive the fluid 2320 from the valve 2370. The system 2302 comprises a temperature regulator 2340 that is in fluid communication with the pump 2330. The temperature regulator 2340 is configured to modulate a temperature (e.g., maintain, increase, and/or decrease) of the fluid 2320. The temperature regulator 2340 may be a plurality of temperature regulators (or a plurality of temperature regulating units), wherein each of the plurality of temperature regulators is configured to modulate a temperature of the fluid 2320, in unison or independently of each other. The temperature regulator 2340 may comprise a thermoelectric engine. The pump 2330 is configured to (i) retrieve or receive the fluid 2320 from the valve 2370, and (ii) direct flow of the fluid 2320 from the pump 2330 and to the temperature regulator 2340. The system 2302 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 is in fluid communication with the temperature regulator 2340 and the valve 2370. The valve 2370 is configured to permit flow of the fluid 2320 from the channel 2360 and towards the pump 2330. The fluid loop (e.g., the water loop) of the system 2302 comprises a flow of the fluid 2320 away from the valve 2370, to the pump 2330, to the temperature regulator 2340, to the channel 2360 of the portion of furniture 2355, and back to the valve 2370. The valve 2370 is configured to receive (e.g., by gravitational force) fluid 2320 out of the container 2315 of the reservoir 2310 and add the received fluid 2320 into the fluid loop. The valve 2370 separates (i) the fluid 2320 contained in the container 2315 of the reservoir 2310 from (ii) the fluid 2320 in, flowing through, and/or flowing adjacent to the temperature regulator 2340. The temperature regulator 2340 is not part of the reservoir 2310. The system 2302 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2302 may regulate the temperature of the portion of furniture 2355 based at least in part on the detected biological signal of the at least one user of the article of furniture 2350.

Referring to FIG. 23D, a system 2303 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2303 comprises a valve 2371 in fluid communication with the container 2315 of the reservoir 2310. The valve 2371 may be a gravitational valve that only allows a flow of the fluid 2320 in a direction away from the container 2315 of the reservoir 2310 and towards the valve 2371. The valve 2371 is configured to prevent flow of the fluid 2320 away from the valve 2371 and back into the container 2315 of the reservoir 2310. The system 2303 comprises a pump 2331 in fluid communication with the valve 2371. The pump 2331 is configured to retrieve or receive the fluid 2320 from the valve 2371. The system 2303 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 is in fluid communication with the pump 2331. The pump 2331 is configured to (i) retrieve or receive the fluid 2320 from the valve 2371, and (ii) direct flow of the fluid 2320 from the pump 2331 and to the channel 2360. The system 2303 comprises a temperature regulator 2341 that is in fluid communication with the channel 2360 and the valve 2371. The temperature regulator 2341 is configured to modulate a temperature (e.g., maintain, increase, and/or decrease) of the fluid 2320. The temperature regulator 2341 may be a plurality of temperature regulators (or a plurality of temperature regulating units), wherein each of the plurality of temperature regulators is configured to modulate a temperature of the fluid 2320, in unison or independently of each other. The temperature regulator 2341 may comprise a thermoelectric engine. The valve 2371 is configured to permit flow of the fluid 2320 from the temperature regulator 2341 and towards the pump 2331. The fluid loop (e.g., the water loop) of the system 2303 comprises a flow of the fluid 2320 away from the valve 2371, to the pump 2331, to the channel 2360 of the portion of furniture 2355, to the temperature regulator 2341, and back to the valve 2371. The valve 2371 is configured to receive (e.g., by gravitational force) fluid 2320 out of the container 2315 of the reservoir 2310 and add the received fluid 2320 into the fluid loop. The valve 2371 separates (i) the fluid 2320 contained in the container 2315 of the reservoir 2310 from (ii) the fluid 2320 in, flowing through, and/or flowing adjacent to the temperature regulator 2341. The temperature regulator 2341 is not part of the reservoir 2310. The system 2303 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2303 may regulate the temperature of the portion of furniture 2355 based at least in part on the detected biological signal of the at least one user of the article of furniture 2350.

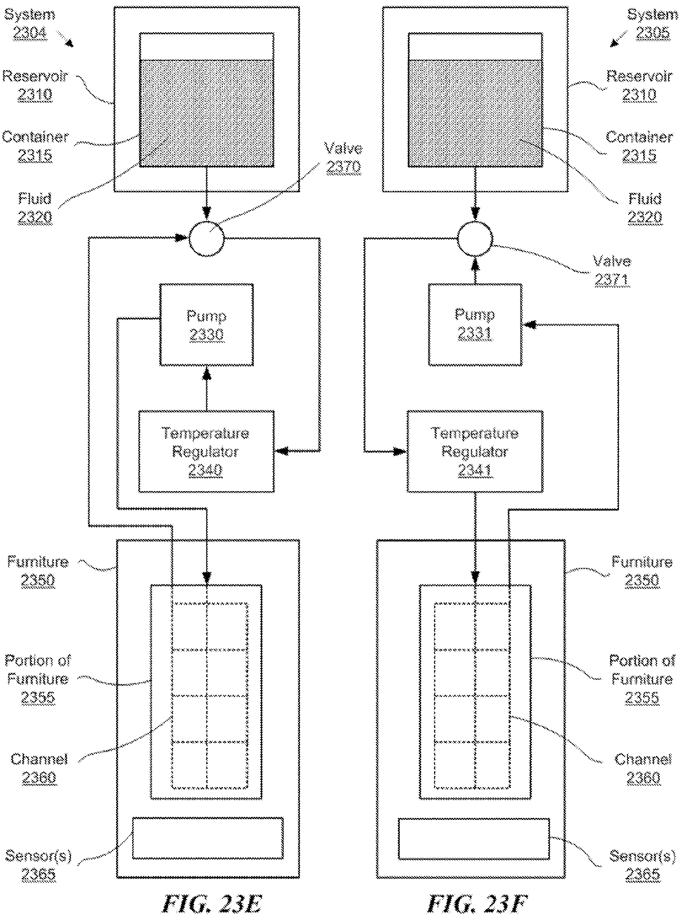

Referring to FIG. 23E, a system 2304 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2304 comprises a valve 2370 in fluid communication with the container 2315 of the reservoir 2310. The valve 2370 may be a gravitational valve that only allows a flow of the fluid 2320 in a direction away from the container 2315 of the reservoir 2310 and towards the valve 2370. The valve 2370 is configured to prevent flow of the fluid 2320 away from the valve 2370 and back into the container 2315 of the reservoir 2310. The system 2304 comprises a temperature regulator 2340 that is in fluid communication with the valve 2370. The temperature regulator 2340 is configured to retrieve or receive the fluid 2320 from the valve 2370. The temperature regulator 2340 is configured to modulate a temperature (e.g., maintain, increase, and/or decrease) of the fluid 2320. The temperature regulator 2340 may be a plurality of temperature regulators (or a plurality of temperature regulating units), wherein each of the plurality of temperature regulators is configured to modulate a temperature of the fluid 2320, in unison or independently of each other. The temperature regulator 2340 may comprise a thermoelectric engine. The system 2304 comprises a pump 2330 in fluid communication with the temperature regulator 2340. The pump 2330 is configured to (i) retrieve or receive the fluid 2320 from the temperature regulator 2340, and (ii) direct flow of the fluid 2320 from the pump 2330 and towards the article of furniture 2350. The system 2304 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 may be in fluid communication with the pump 2330 and the valve 2370. The valve 2370 is configured to permit flow of the fluid 2320 from the channel 2360 and towards the temperature regulator 2340. The fluid loop (e.g., the water loop) of the system 2304 comprises a flow of the fluid 2320 away from the valve 2370, to the temperature regulator 2340, to the pump 2330, to the channel 2360 of the portion of furniture 2355, and back to the valve 2370. The valve 2370 is configured to receive (e.g., by gravitational force) fluid 2320 out of the container 2315 of the reservoir 2310 and add the received fluid 2320 into the fluid loop. The valve 2370 separates (i) the fluid 2320 contained in the container 2315 of the reservoir 2310 from (ii) the fluid 2320 in, flowing through, and/or flowing adjacent to the temperature regulator 2340. The temperature regulator 2340 is not part of the reservoir 2310. The system 2304 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2304 may regulate the temperature of the portion of furniture 2355 based at least in part on the detected biological signal of the at least one user of the article of furniture 2350.

Referring to FIG. 23F, a system 2305 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2305 comprises a valve 2371 in fluid communication with the container 2315 of the reservoir 2310. The valve 2371 may be a gravitational valve that only allows a flow of the fluid 2320 in a direction away from the container 2315 of the reservoir 2310 and towards the valve 2371. The valve 2371 is configured to prevent flow of the fluid 2320 away from the valve 2371 and back into the container 2315 of the reservoir 2310. The system 2305 comprises a temperature regulator 2341 that is in fluid communication with the valve 2371. The temperature regulator 2341 is configured to retrieve or receive the fluid 2320 from the valve 2371. The temperature regulator 2341 is configured to modulate a temperature (e.g., maintain, increase, and/or decrease) of the fluid 2320. The temperature regulator 2341 may be a plurality of temperature regulators (or a plurality of temperature regulating units), wherein each of the plurality of temperature regulators is configured to modulate a temperature of the fluid 2320, in unison or independently of each other. The temperature regulator 2341 may comprise a thermoelectric engine. The system 2304 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 may be in fluid communication with the temperature regulator 2341. The system 2305 comprises a pump 2331 in fluid communication with the channel 2360. The pump 2331 is configured to (i) retrieve or receive the fluid 2320 from the channel 2360, and (ii) direct flow of the fluid 2320 from the pump 2331 and towards the valve 2371. The valve 2371 is configured to permit flow of the fluid 2320 from the pump 2331 and towards the temperature regulator 2341. The fluid loop (e.g., the water loop) of the system 2305 comprises a flow of the fluid 2320 away from the valve 2371, to the temperature regulator 2341, to the channel 2360 of the portion of furniture 2355, to the pump 2331, and back to the valve 2371. The valve 2371 is configured to receive (e.g., by gravitational force) fluid 2320 out of the container 2315 of the reservoir 2310 and add the received fluid 2320 into the fluid loop. The valve 2371 separates (i) the fluid 2320 contained in the container 2315 of the reservoir 2310 from (ii) the fluid 2320 in, flowing through, and/or flowing adjacent to the temperature regulator 2341. The temperature regulator 2341 is not part of the reservoir 2310. The system 2305 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2305 may regulate the temperature of the portion of furniture 2355 based at least in part on the detected biological signal of the at least one user of the article of furniture 2350.

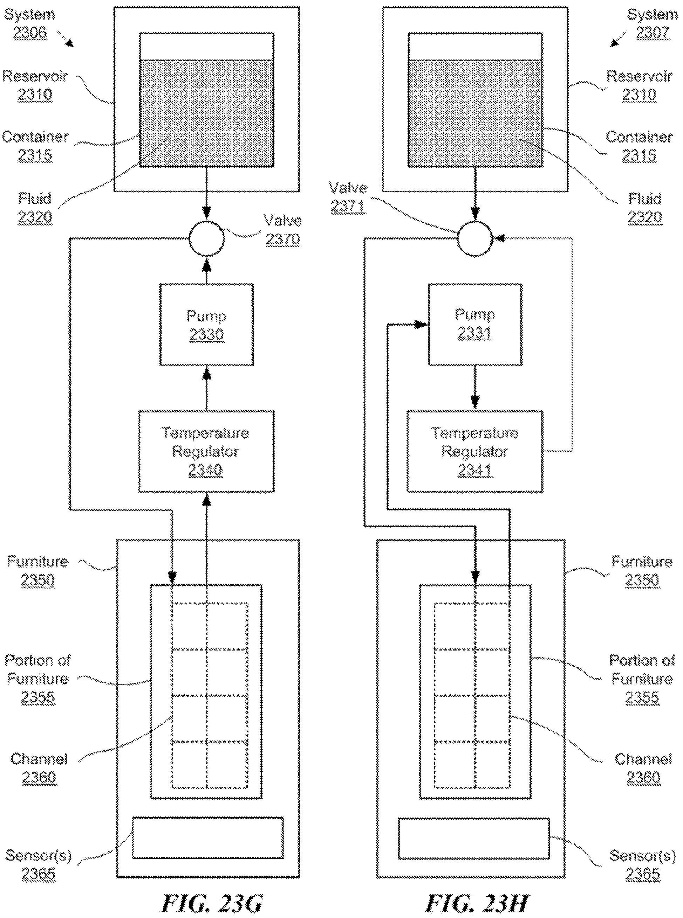

Referring to FIG. 23G, a system 2306 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2306 comprises a valve 2370 in fluid communication with the container 2315 of the reservoir 2310. The valve 2370 may be a gravitational valve that only allows a flow of the fluid 2320 in a direction away from the container 2315 of the reservoir 2310 and towards the valve 2370. The valve 2370 is configured to prevent flow of the fluid 2320 away from the valve 2370 and back into the container 2315 of the reservoir 2310. The system 2306 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 may be in fluid communication with the valve 2370. The valve 2370 is configured to permit flow of the fluid 2320 from the container 2315 and towards the channel 2360. The system 2306 comprises a temperature regulator 2340 that is in fluid communication with the channel 2360. The temperature regulator 2340 is configured to retrieve or receive the fluid 2320 from the channel 2360. The temperature regulator 2340 is configured to modulate a temperature (e.g., maintain, increase, and/or decrease) of the fluid 2320. The temperature regulator 2340 may be a plurality of temperature regulators (or a plurality of temperature regulating units), wherein each of the plurality of temperature regulators is configured to modulate a temperature of the fluid 2320, in unison or independently of each other. The temperature regulator 2340 may comprise a thermoelectric engine. The system 2306 comprises a pump 2330 in fluid communication with the temperature regulator 2340 and the valve 2370. The pump 2330 is configured to (i) retrieve or receive the fluid 2320 from the temperature regulator 2340, and (ii) direct flow of the fluid 2320 from the pump 2330 and towards the valve 2370. The fluid loop (e.g., the water loop) of the system 2306 comprises a flow of the fluid 2320 away from the valve 2370, to the channel 2360 of the portion of furniture 2355, to the temperature regulator 2340, to the pump 2330, and back to the valve 2370. The valve 2370 is configured to receive (e.g., by gravitational force) fluid 2320 out of the container 2315 of the reservoir 2310 and add the received fluid 2320 into the fluid loop. The valve 2370 separates (i) the fluid 2320 contained in the container 2315 of the reservoir 2310 from (ii) the fluid 2320 in, flowing through, and/or flowing adjacent to the temperature regulator 2340. The temperature regulator 2340 is not part of the reservoir 2310. The system 2306 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2306 may regulate the temperature of the portion of furniture 2355 based at least in part on the detected biological signal of the at least one user of the article of furniture 2350.

Referring to FIG. 23H, a system 2307 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2307 comprises a valve 2371 in fluid communication with the container 2315 of the reservoir 2310. The valve 2371 may be a gravitational valve that only allows a flow of the fluid 2320 in a direction away from the container 2315 of the reservoir 2310 and towards the valve 2371. The valve 2371 is configured to prevent flow of the fluid 2320 away from the valve 2371 and back into the container 2315 of the reservoir 2310. The system 2307 comprises a portion 2355 of the article of furniture 2350 configured to hold and permit flow of the fluid 2320. The portion of furniture 2355 comprises a channel 2360 (e.g., an interconnected network of a plurality of channels) configured to hold and permit flow of the fluid 2320. The fluid 2320 may be held in the channel 2360 and/or flow through the channel 2360 to modulate the temperature of the portion of furniture 2355. The channel 2360 may be in fluid communication with the valve 2371. The valve 2371 is configured to permit flow of the fluid 2320 from the container 2315 and towards the channel 2360. The system 2307 comprises a pump 2331 in fluid communication with the channel 2360. The pump 2331 is configured to (i) retrieve or receive the fluid 2320 from the channel 2360, and (ii) direct flow of the fluid 2320 from the pump 2331 and towards a temperature regulator 2341. The system 2307 comprises the temperature regulator 2341 that is in fluid communication with the pump 2331 and the valve 2371. The temperature regulator 2341 is configured to retrieve or receive the fluid 2320 from the pump 2331. The temperature regulator 2341 is configured to modulate a temperature (e.g., maintain, increase, and/or decrease) of the fluid 2320. The temperature regulator 2341 may be a plurality of temperature regulators (or a plurality of temperature regulating units), wherein each of the plurality of temperature regulators is configured to modulate a temperature of the fluid 2320, in unison or independently of each other. The temperature regulator 2341 may comprise a thermoelectric engine. The fluid loop (e.g., the water loop) of the system 2307 comprises a flow of the fluid 2320 away from the valve 2371, to the channel 2360 of the portion of furniture 2355, to the pump 2331, to the temperature regulator 2341, and back to the valve 2371. The valve 2371 is configured to receive (e.g., by gravitational force) fluid 2320 out of the container 2315 of the reservoir 2310 and add the received fluid 2320 into the fluid loop. The valve 2371 separates (i) the fluid 2320 contained in the container 2315 of the reservoir 2310 from (ii) the fluid 2320 in, flowing through, and/or flowing adjacent to the temperature regulator 2341. The temperature regulator 2341 is not part of the reservoir 2310. The system 2307 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365 and the portion of furniture 2355 may be in different parts of the article of furniture 2350. The system 2307 may regulate the temperature of the portion of furniture 2355 based at least in part on the detected biological signal of the at least one user of the article of furniture 2350.

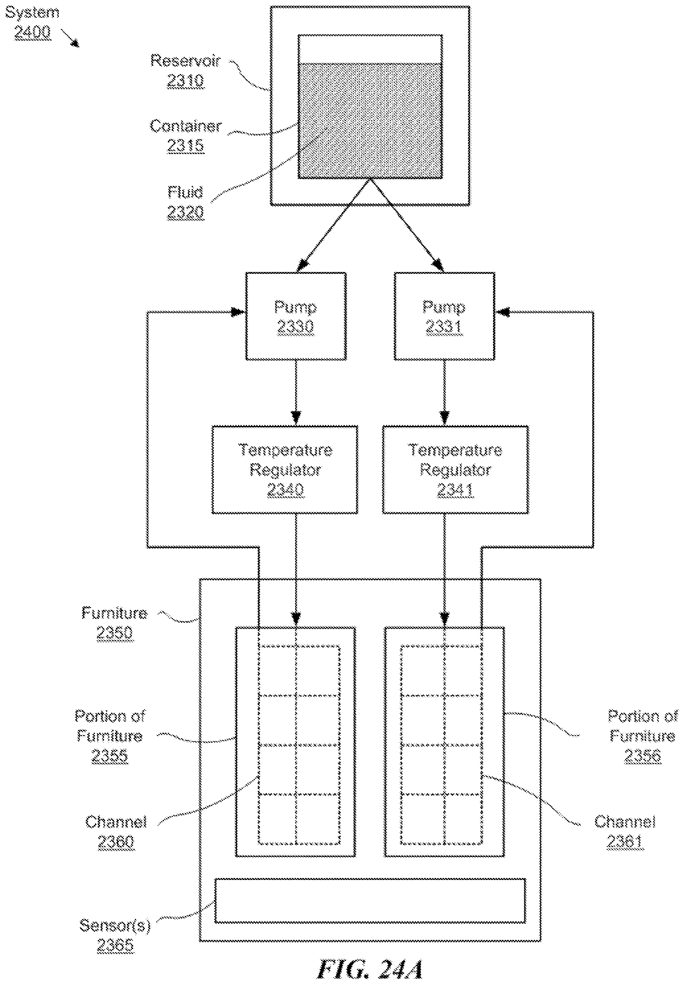

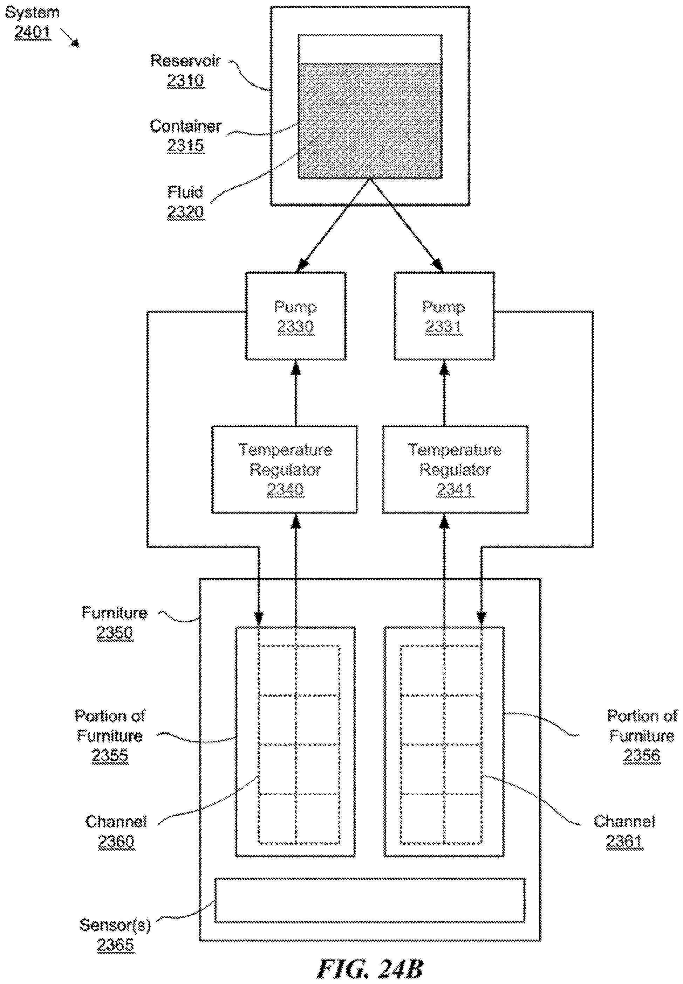

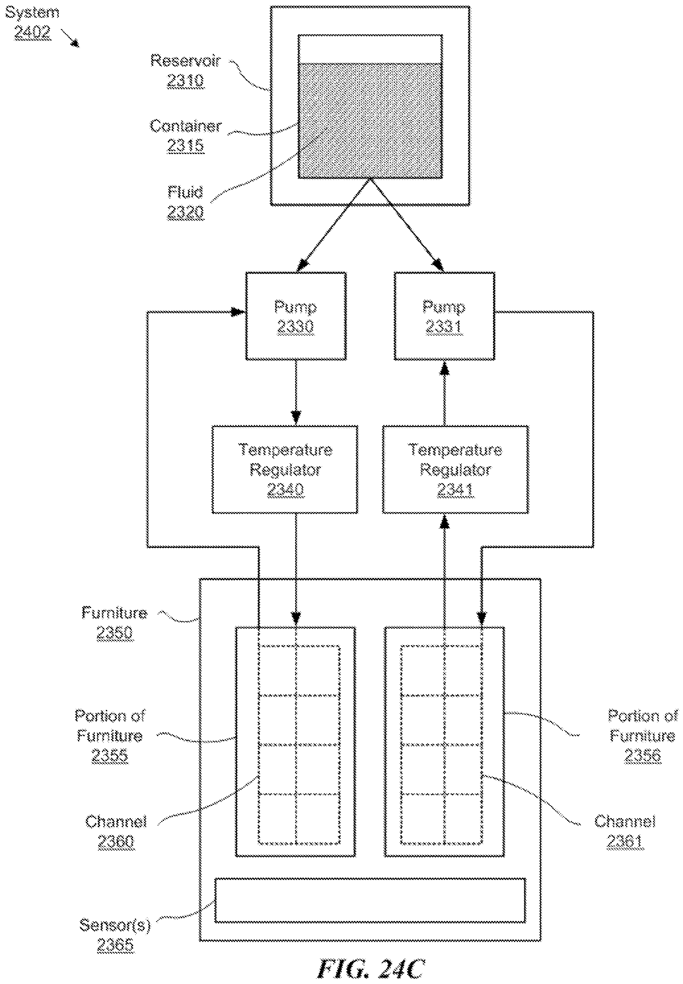

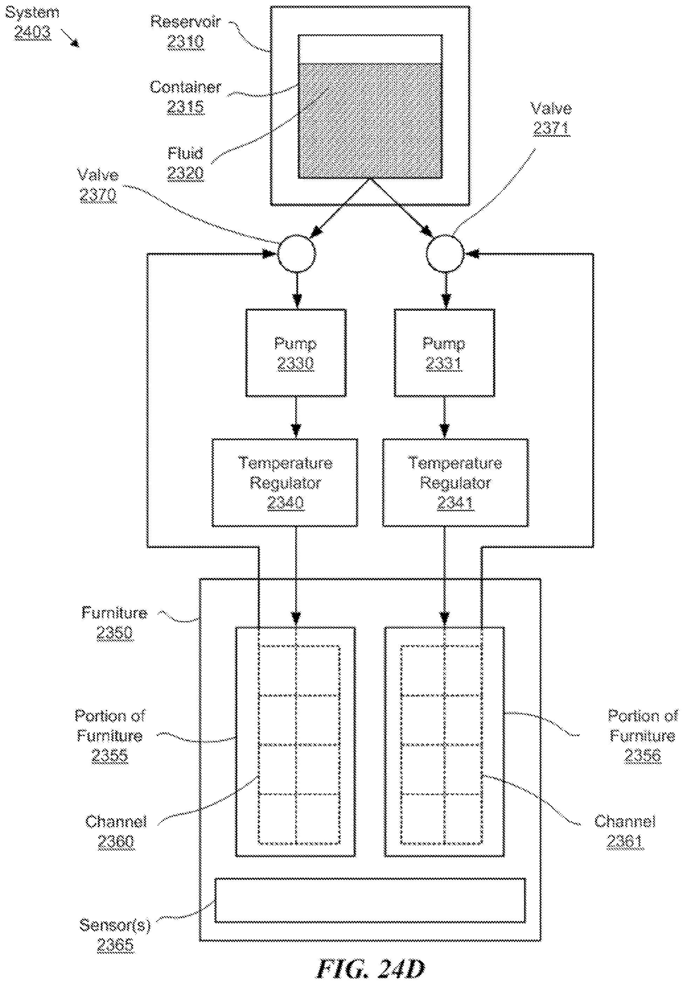

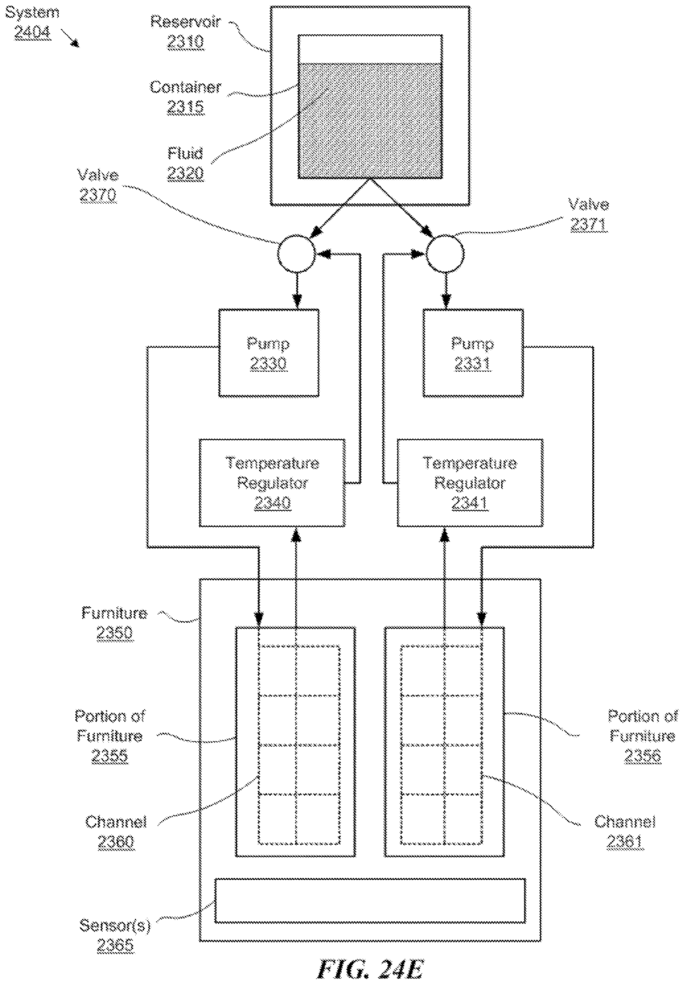

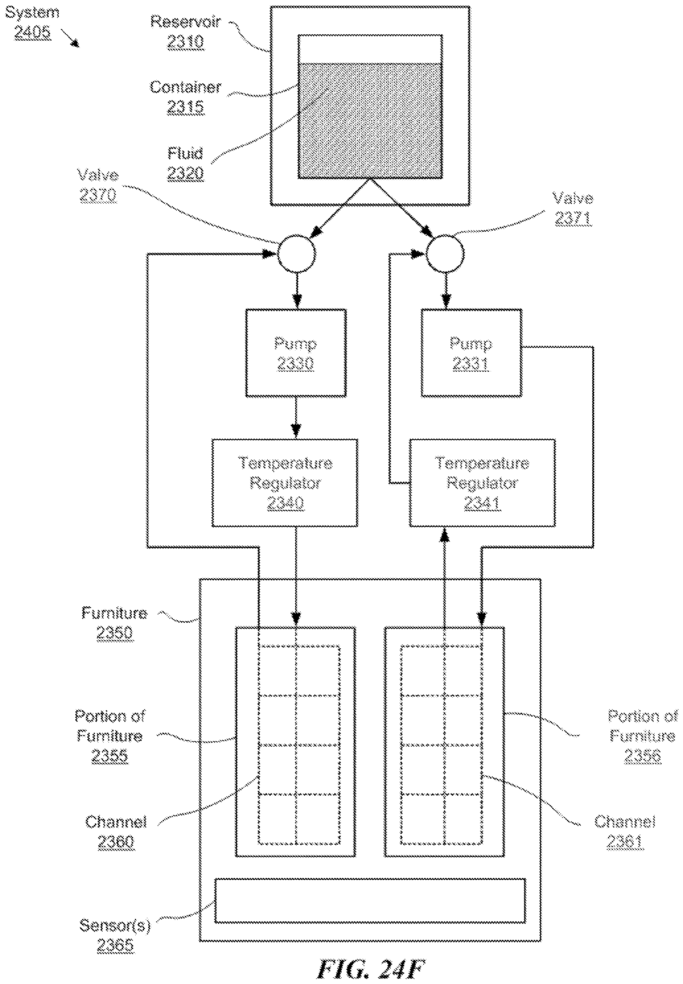

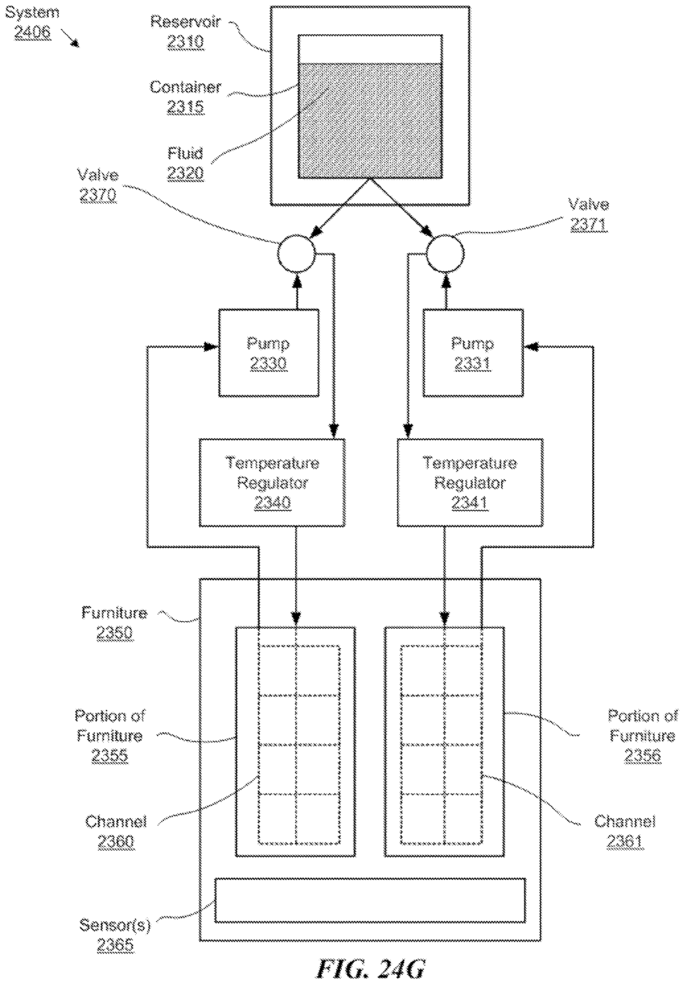

At least two of the fluid loops (e.g., at least about 2, 3, 4, 5, or more fluid loops) as illustrated in FIGs. 23A to 23F, or functional modifications thereof, may be combined into a common system, which common system comprises a common reservoir. The common system may comprise a common article of furniture (e.g., one bed). The at least two fluid loops may be in fluid communication with the common article of furniture (e.g., in fluid communication with at least two different portions of the common article of furniture). The at least two fluid loops may be in fluid communication with the common reservoir. A processor may be configured to control (independently or in unison) the at least two fluid loops to modulate the temperature of the fluid in each of the at least two fluid loops, thereby to regulate (independently or in unison) temperatures of the at least two different portions of the common article of furniture. Alternatively or in addition to, the common system may comprise at least two of articles of furniture (e.g., at least two beds). Each of the at least two fluid loops may be in fluid communication with each of the at least two articles of furniture. The processor may be configured to control (independently or in unison) the at least two fluid loops to modulate the temperature of the fluid in each of the at least two fluid loops, thereby to regulate (independently or in unison) temperatures of the at least two articles of furniture. The at least two fluid loops in fluid communication with the common reservoir may have a same direction or different directions of fluid flow. Examples of such system comprising the common reservoir and the at least two fluid loops are illustrated in FIG. 24.

FIGs. 24A to 24F schematically illustrate examples of a system for regulating temperatures of two portions of an article of furniture (e.g., a bed, mattress, or mattress pad), which system comprises two fluid loops (e.g., two water loops). Referring to FIG. 24A, the system 2400 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2400 comprises two fluid loops that are in fluid communication with the container 2315 of the reservoir 2310. The two fluid loops may or may not be in fluid communication with each other. The reservoir 2310 serves as a common reservoir for the two fluid loops of the system 2400. The first fluid loop comprises (i) the pump 2330, (ii) the temperature regulator 2340, and (iii) the channel 2360 of the portion 2355 of the article of furniture 2350. The pump 2330 is configured to retrieve or receive the fluid 2320 from the container 2315 of the reservoir 2310. The pump 2330 is configured to prevent flow of the fluid 2320 away from the pump 2330 and back into the container 2315 of the reservoir 2310. The pump 2330 is configured to direct flow of the fluid 2320 in the first fluid loop, from the pump 2330, to the temperature regulator 2340, to the channel 2360, and back to the pump 2330. The temperature regulator 2340 is configured to modulate a temperature of the fluid 2320 in the first fluid loop. The second loop may comprise features that may or may not be identical to the first loop. Referring to FIG. 24A, the second fluid loop comprises (i) the pump 2331, (ii) the temperature regulator 2341, and (iii) the channel 2361 of the portion 2356 of the article of furniture 2350. The pump 2331 is configured to retrieve or receive the fluid 2320 from the container 2315 of the reservoir 2310. The pump 2331 is configured to prevent flow of the fluid 2320 away from the pump 2331 and back into the container 2315 of the reservoir 2310. The pump 2331 is configured to direct flow of the fluid 2320 in the second fluid loop, from the pump 2331, to the temperature regulator 2341, to the channel 2361, and back to the pump 2331. The temperature regulator 2341 is configured to modulate a temperature of the fluid 2320 in the second fluid loop. The system 2400 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365, the portion of furniture 2355, and the portion of furniture 2356 may be in different parts of the article of furniture 2350. The system 2400 may regulate the temperature of the portion of furniture 2355 and/or the temperature of the portion of furniture 2356 based at least in part on the detected biological signal of the at least one user (e.g., one or two users) of the article of furniture 2350. The first fluid loop of the system 2400 may utilize all components and configurations described in the fluid loop of the system 2300, as illustrated in FIG. 23A. The second fluid loop of the system 2400 may utilize all components and configurations described in the fluid loop of the system 2300, as illustrated in FIG. 23A.

Referring to FIG. 24B, the system 2401 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2401 comprises two fluid loops that are in fluid communication with the container 2315 of the reservoir 2310. The two fluid loops may or may not be in fluid communication with each other. The reservoir 2310 serves as a common reservoir for the two fluid loops of the system 2401. The first fluid loop comprises (i) the pump 2330, (ii) the temperature regulator 2340, and (iii) the channel 2360 of the portion 2355 of the article of furniture 2350. The pump 2330 is configured to retrieve or receive the fluid 2320 from the container 2315 of the reservoir 2310. The pump 2330 is configured to prevent flow of the fluid 2320 away from the pump 2330 and back into the container 2315 of the reservoir 2310. The pump 2330 is configured to direct flow of the fluid 2320 in the first fluid loop, from the pump 2330, to the channel 2360, to the temperature regulator 2340, and back to the pump 2330. The temperature regulator 2340 is configured to modulate a temperature of the fluid 2320 in the first fluid loop. The second loop may comprise features that may or may not be identical to the first loop. Referring to FIG. 24B, the second fluid loop comprises (i) the pump 2331, (ii) the temperature regulator 2341, and (iii) the channel 2361 of the portion 2356 of the article of furniture 2350. The pump 2331 is configured to retrieve or receive the fluid 2320 from the container 2315 of the reservoir 2310. The pump 2331 is configured to prevent flow of the fluid 2320 away from the pump 2331 and back into the container 2315 of the reservoir 2310. The pump 2331 is configured to direct flow of the fluid 2320 in the second fluid loop, from the pump 2331, to the channel 2361, to the temperature regulator 2341, and back to the pump 2331. The temperature regulator 2341 is configured to modulate a temperature of the fluid 2320 in the second fluid loop. The system 2401 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365, the portion of furniture 2355, and the portion of furniture 2356 may be in different parts of the article of furniture 2350. The system 2401 may regulate the temperature of the portion of furniture 2355 and/or the temperature of the portion of furniture 2356 based at least in part on the detected biological signal of the at least one user (e.g., one or two users) of the article of furniture 2350. The first fluid loop of the system 2401 may utilize all components and configurations described in the fluid loop of the system 2301, as illustrated in FIG. 23B. The second fluid loop of the system 2401 may utilize all components and configurations described in the fluid loop of the system 2301, as illustrated in FIG. 23B.

Referring to FIG. 24C, the system 2402 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2402 comprises two fluid loops that are in fluid communication with the container 2315 of the reservoir 2310. The two fluid loops may or may not be in fluid communication with each other. The reservoir 2310 serves as a common reservoir for the two fluid loops of the system 2402. The first fluid loop comprises (i) the pump 2330, (ii) the temperature regulator 2340, and (iii) the channel 2360 of the portion 2355 of the article of furniture 2350. The pump 2330 is configured to retrieve or receive the fluid 2320 from the container 2315 of the reservoir 2310. The pump 2330 is configured to prevent flow of the fluid 2320 away from the pump 2330 and back into the container 2315 of the reservoir 2310. The pump 2330 is configured to direct flow of the fluid 2320 in the first fluid loop, from the pump 2330, to the temperature regulator 2340, to the channel 2360, and back to the pump 2330. The temperature regulator 2340 is configured to modulate a temperature of the fluid 2320 in the first fluid loop. The second loop may comprise features that may or may not be identical to the first loop. Referring to FIG. 24C, the second fluid loop comprises (i) the pump 2331, (ii) the temperature regulator 2341, and (iii) the channel 2361 of the portion 2356 of the article of furniture 2350. The pump 2331 is configured to retrieve or receive the fluid 2320 from the container 2315 of the reservoir 2310. The pump 2331 is configured to prevent flow of the fluid 2320 away from the pump 2331 and back into the container 2315 of the reservoir 2310. The pump 2331 is configured to direct flow of the fluid 2320 in the second fluid loop, from the pump 2331, to the channel 2361, to the temperature regulator 2341, and back to the pump 2331. The temperature regulator 2341 is configured to modulate a temperature of the fluid 2320 in the second fluid loop. The system 2402 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365, the portion of furniture 2355, and the portion of furniture 2356 may be in different parts of the article of furniture 2350. The system 2402 may regulate the temperature of the portion of furniture 2355 and/or the temperature of the portion of furniture 2356 based at least in part on the detected biological signal of the at least one user (e.g., one or two users) of the article of furniture 2350. The first fluid loop of the system 2402 may utilize all components and configurations described in the fluid loop of the system 2300, as illustrated in FIG. 23A. The second fluid loop of the system 2402 may utilize all components and configurations described in the fluid loop of the system 2301, as illustrated in FIG. 23B.

Referring to FIG. 24D, the system 2403 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2403 comprises two fluid loops that are in fluid communication with the container 2315 of the reservoir 2310. The two fluid loops may or may not be in fluid communication with each other. The reservoir 2310 serves as a common reservoir for the two fluid loops of the system 2403. The first fluid loop comprises (i) the valve 2370, (ii) the pump 2330, (iii) the temperature regulator 2340, and (iv) the channel 2360 of the portion 2355 of the article of furniture 2350. The valve 2370 is configured to receive (e.g., by the gravitational force) the fluid 2320 from the container 2315 of the reservoir 2310. The valve 2370 is configured to prevent flow of the fluid 2320 away from the valve 2370 and back into the container 2315 of the reservoir 2310. The pump 2330 is configured to direct flow of the fluid 2320 in the first fluid loop, from the valve 2370, to the pump 2330, to the temperature regulator 2340, to the channel 2360, and back to the valve 2370. The temperature regulator 2340 is configured to modulate a temperature of the fluid 2320 in the first fluid loop. The second loop may comprise features that may or may not be identical to the first loop. Referring to FIG. 24D, the second fluid loop comprises (i) the valve 2371, (ii) the pump 2331, (iii) the temperature regulator 2341, and (iv) the channel 2361 of the portion 2356 of the article of furniture 2350. The valve 2371 is configured to retrieve or receive (e.g., by the gravitational force) the fluid 2320 from the container 2315 of the reservoir 2310. The valve 2371 is configured to prevent flow of the fluid 2320 away from the valve 2371 and back into the container 2315 of the reservoir 2310. The pump 2331 is configured to direct flow of the fluid 2320 in the second fluid loop, from the valve 2371, to the pump 2331, to the temperature regulator 2341, to the channel 2361, and back to the valve 2371. The temperature regulator 2341 is configured to modulate a temperature of the fluid 2320 in the second fluid loop. The system 2403 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365, the portion of furniture 2355, and the portion of furniture 2356 may be in different parts of the article of furniture 2350. The system 2403 may regulate the temperature of the portion of furniture 2355 and/or the temperature of the portion of furniture 2356 based at least in part on the detected biological signal of the at least one user (e.g., one or two users) of the article of furniture 2350. The first fluid loop of the system 2403 may utilize all components and configurations described in the fluid loop of the system 2302, as illustrated in FIG. 23C. The second fluid loop of the system 2403 may utilize all components and configurations described in the fluid loop of the system 2302, as illustrated in FIG. 23C.

Referring to FIG. 24E, the system 2404 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2404 comprises two fluid loops that are in fluid communication with the container 2315 of the reservoir 2310. The two fluid loops may or may not be in fluid communication with each other. The reservoir 2310 serves as a common reservoir for the two fluid loops of the system 2403. The first fluid loop comprises (i) the valve 2370, (ii) the pump 2330, (iii) the temperature regulator 2340, and (iv) the channel 2360 of the portion 2355 of the article of furniture 2350. The valve 2370 is configured to receive (e.g., by the gravitational force) the fluid 2320 from the container 2315 of the reservoir 2310. The valve 2370 is configured to prevent flow of the fluid 2320 away from the valve 2370 and back into the container 2315 of the reservoir 2310. The pump 2330 is configured to direct flow of the fluid 2320 in the first fluid loop, from the valve 2370, to the pump 2330, to the channel 2360, to the temperature regulator 2340, and back to the valve 2370. The temperature regulator 2340 is configured to modulate a temperature of the fluid 2320 in the first fluid loop. The second loop may comprise features that may or may not be identical to the first loop. Referring to FIG. 24E, the second fluid loop comprises (i) the valve 2371, (ii) the pump 2331, (iii) the temperature regulator 2341, and (iv) the channel 2361 of the portion 2356 of the article of furniture 2350. The valve 2371 is configured to retrieve or receive (e.g., by the gravitational force) the fluid 2320 from the container 2315 of the reservoir 2310. The valve 2371 is configured to prevent flow of the fluid 2320 away from the valve 2371 and back into the container 2315 of the reservoir 2310. The pump 2331 is configured to direct flow of the fluid 2320 in the second fluid loop, from the valve 2371, to the pump 2331, to the channel 2361, to the temperature regulator 2341, and back to the valve 2371. The temperature regulator 2341 is configured to modulate a temperature of the fluid 2320 in the second fluid loop. The system 2404 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365, the portion of furniture 2355, and the portion of furniture 2356 may be in different parts of the article of furniture 2350. The system 2404 may regulate the temperature of the portion of furniture 2355 and/or the temperature of the portion of furniture 2356 based at least in part on the detected biological signal of the at least one user (e.g., one or two users) of the article of furniture 2350. The first fluid loop of the system 2404 may utilize all components and configurations described in the fluid loop of the system 2303, as illustrated in FIG. 23D. The second fluid loop of the system 2404 may utilize all components and configurations described in the fluid loop of the system 2303, as illustrated in FIG. 23D.

Referring to FIG. 24F, the system 2405 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2405 comprises two fluid loops that are in fluid communication with the container 2315 of the reservoir 2310. The two fluid loops may or may not be in fluid communication with each other. The reservoir 2310 serves as a common reservoir for the two fluid loops of the system 2405. The first fluid loop comprises (i) the valve 2370, (ii) the pump 2330, (iii) the temperature regulator 2340, and (iv) the channel 2360 of the portion 2355 of the article of furniture 2350. The valve 2370 is configured to receive (e.g., by the gravitational force) the fluid 2320 from the container 2315 of the reservoir 2310. The valve 2370 is configured to prevent flow of the fluid 2320 away from the valve 2370 and back into the container 2315 of the reservoir 2310. The pump 2330 is configured to direct flow of the fluid 2320 in the first fluid loop, from the valve 2370, to the pump 2330, to the temperature regulator 2340, to the channel 2360, and back to the valve 2370. The temperature regulator 2340 is configured to modulate a temperature of the fluid 2320 in the first fluid loop. The second loop may comprise features that may or may not be identical to the first loop. Referring to FIG. 24F, the second fluid loop comprises (i) the valve 2371, (ii) the pump 2331, (iii) the temperature regulator 2341, and (iv) the channel 2361 of the portion 2356 of the article of furniture 2350. The valve 2371 is configured to retrieve or receive (e.g., by the gravitational force) the fluid 2320 from the container 2315 of the reservoir 2310. The valve 2371 is configured to prevent flow of the fluid 2320 away from the valve 2371 and back into the container 2315 of the reservoir 2310. The pump 2331 is configured to direct flow of the fluid 2320 in the second fluid loop, from the valve 2371, to the pump 2331, to the channel 2361, to the temperature regulator 2341, and back to the valve 2371. The temperature regulator 2341 is configured to modulate a temperature of the fluid 2320 in the second fluid loop. The system 2405 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365, the portion of furniture 2355, and the portion of furniture 2356 may be in different parts of the article of furniture 2350. The system 2405 may regulate the temperature of the portion of furniture 2355 and/or the temperature of the portion of furniture 2356 based at least in part on the detected biological signal of the at least one user (e.g., one or two users) of the article of furniture 2350. The first fluid loop of the system 2405 may utilize all components and configurations described in the fluid loop of the system 2302, as illustrated in FIG. 23C. The second fluid loop of the system 2404 may utilize all components and configurations described in the fluid loop of the system 2303, as illustrated in FIG. 23D.

Referring to FIG. 24G, the system 2406 comprises a reservoir 2310 configured to contain the fluid 2320 (e.g., water). The reservoir comprises a container 2315 (e.g., a removable or non-removable container) configured to contain the fluid. Neither the reservoir 2310 nor the container 2315 is configured to modulate the temperature of the fluid that is contained in the container 2315. The system 2406 comprises two fluid loops that are in fluid communication with the container 2315 of the reservoir 2310. The two fluid loops may or may not be in fluid communication with each other. The reservoir 2310 serves as a common reservoir for the two fluid loops of the system 2406. The first fluid loop comprises (i) the valve 2370, (ii) the temperature regulator 2340, (iii) the channel 2360 of the portion 2355 of the article of furniture 2350, and (iv) the pump 2330. The valve 2370 is configured to receive (e.g., by the gravitational force) the fluid 2320 from the container 2315 of the reservoir 2310. The valve 2370 is configured to prevent flow of the fluid 2320 away from the valve 2370 and back into the container 2315 of the reservoir 2310. The temperature regulator 2340 is configured to modulate a temperature of the fluid 2320 in the first fluid loop. The pump 2330 is configured to direct flow of the fluid 2320 in the first fluid loop, from the valve 2370, to the temperature regulator 2340, to the channel 2360, to the pump 2330, and back to the valve 2370. The second loop may comprise features that may or may not be identical to the first loop. Referring to FIG. 24G, the second fluid loop comprises features that are identical to the first loop. The system 2406 further comprises one or more sensor(s) 2365 configured to detect a biological signal (e.g., a heart signal, a respiration signal, a motion, a temperature, and/or perspiration) of at least one user of the article of furniture 2350. The sensor(s) 2365 may be part of the article of furniture 2350. The sensor(s) 2365, the portion of furniture 2355, and the portion of furniture 2356 may be in different parts of the article of furniture 2350. The system 2406 may regulate the temperature of the portion of furniture 2355 and/or the temperature of the portion of furniture 2356 based at least in part on the detected biological signal of the at least one user (e.g., one or two users) of the article of furniture 2350. The first fluid loop of the system 2406 may utilize all components and configurations described in the fluid loop of the system 2305, as illustrated in FIG. 23F. The second fluid loop of the system 2406 may utilize all components and configurations described in the fluid loop of the system 2305, as illustrated in FIG. 23F.

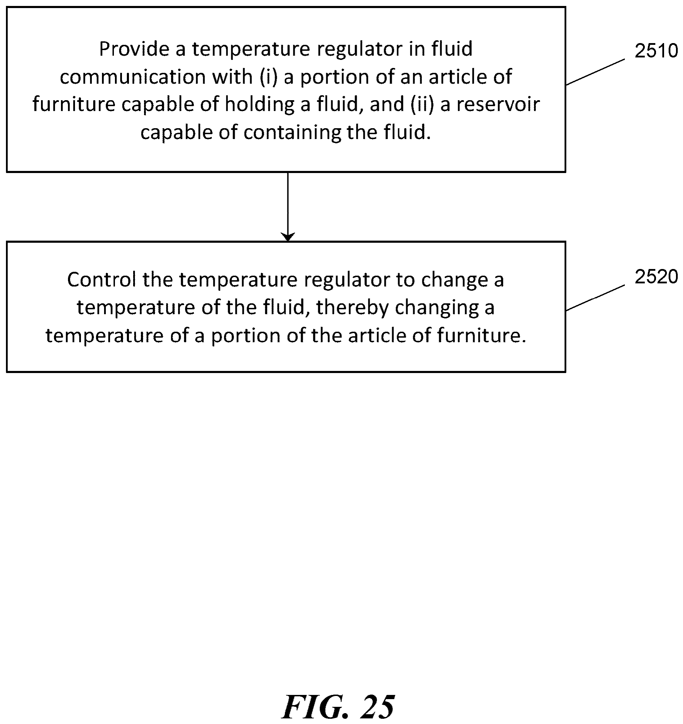

FIG. 25 illustrates an example of a method for regulating a temperature of an article of furniture. The method may comprise providing a temperature regulator in fluid communication with (i) the portion of the article of furniture capable of holding a fluid, and (ii) a reservoir capable of containing the fluid, wherein the temperature regulator is capable of modulating a temperature of the fluid when the fluid is not contained in the reservoir (process 2510). The method may comprise, controlling, by a computer system, the temperature regulator to modulate the temperature of the fluid, thereby regulating the temperature of the portion of the article of furniture (process 2520).

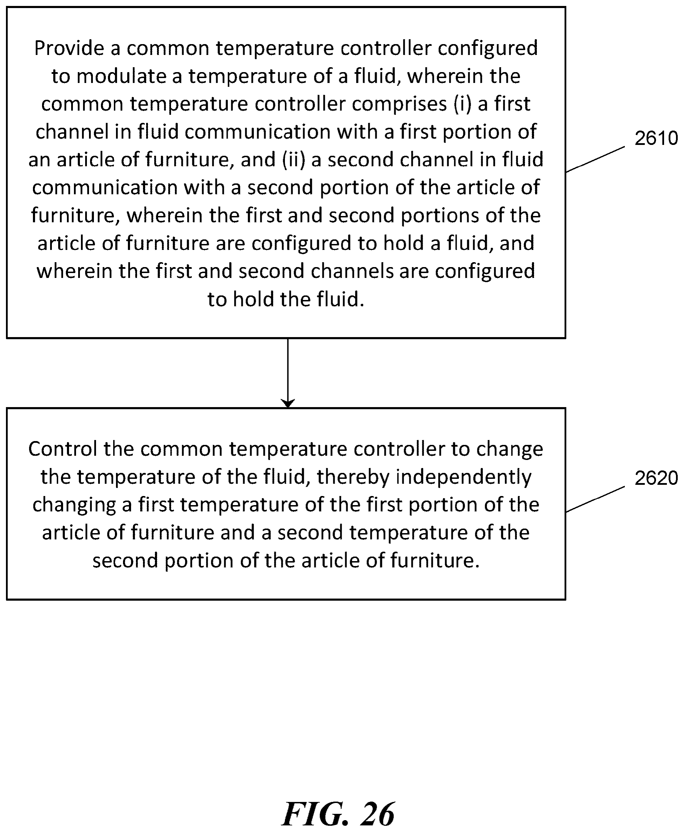

FIG. 26 illustrates an additional example of a method for regulating a temperature of an article of furniture. The method may comprise providing a common temperature controller configured to modulate a temperature of a fluid, wherein the common temperature controller comprises (i) a first channel in fluid communication with a first portion of the article of furniture, and (ii) a second channel in fluid communication with a second portion of the article of furniture, wherein the first and second portions of the article of furniture are configured to hold a fluid, and wherein the first and second channels are configured to hold the fluid (process 2610). The method may comprise controlling the common temperature controller to modulate the temperature of the fluid, thereby independently regulating a first temperature of the first portion of the article of furniture and a second temperature of the second portion of the article of furniture (process 2620).

### Biological signal processing

The technology disclosed here categorizes the sleep phase associated with a user as light sleep, deep sleep, or REM sleep. Light sleep comprises stage one and stage two sleep. The technology performs the categorization based on the respiration rate associated with the user, heart rate associated with the user, motion associated with the user, and body temperature associated with the user. Generally, when the user is awake the respiration is erratic. When the user is sleeping, the respiration becomes regular. The transition between being awake and sleeping is quick, and lasts less than 1 minute.

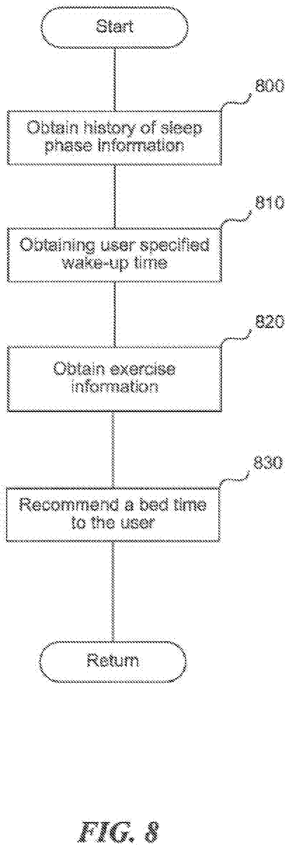

FIG. 8 is a flowchart of the process for recommending a bed time to the user, according to one embodiment. At block 800, the process obtains a history of sleep phase information associated with the user. The history of sleep phase information comprises an amount of time the user spent in each of the sleep phases, light sleep, deep sleep, or REM sleep. The history of sleep phase information can be stored in a database associated with the user. Based on this information, the process determines how much light sleep, deep sleep, and REM sleep, the user needs on average every day. In another embodiment, the history of sleep phase information comprises the average bedtime associated with the user for each day of the week (e.g. the average bedtime associated with the user on Monday, the average bedtime associated with the user on Tuesday, etc.). At block 810, the process obtains user-specified wake-up time, such as the alarm setting associated with the user. At block 820, the process obtains exercise information associated with the user, such as the distance the user ran that day, the amount of time the user exercised in the gym, or the amount of calories the user burned that day. According to one embodiment, the process obtains the exercise information from a user phone, a wearable device, a Titbit bracelet, or a database storing the exercise information. Based on all this information, at block 830, the process recommends a bedtime to the user. For example, if the user has not been getting enough deep and REM sleep in the last few days, the process recommends an earlier bedtime to the user. Also, if the user has exercised more than the average daily exercise, the process recommends an earlier bedtime to the user.

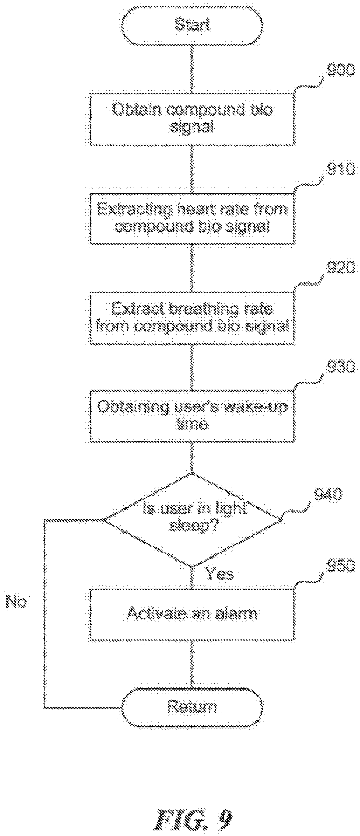

FIG. 9 is a flowchart of the process for activating a user's alarm, according to one embodiment. At block 900, the process obtains the compound bio signal associated with the user. The compound bio signal associated with the user comprises the heart rate associated with the user, and the respiration rate associated with the user. According to one embodiment, the process obtains the compound bio signal from a sensor associated with the user. At block 910, the process extracts the heart rate signal from the compound bio signal. For example, the process extracts the heart rate signal associated with the user by performing low-pass filtering on the compound bio signal. Also, at block 920, the process extracts the respiration rate signal from the compound bio signal. For example, the process extracts the respiration rate by performing bandpass filtering on the compound bio signal. The respiration rate signal includes breath duration, pauses between breaths, as well as breaths per minute. At block 930, the process obtains user's wake-up time, such as the alarm setting associated with the user. Based on the heart rate signal and the respiration rate signal, the process determines the sleep phase associated with the user, and if the user is in light sleep, and current time is at most one hour before the alarm time, at block 940, the process activates an alarm. Waking up the user during the deep sleep or REM sleep is detrimental to the user's health because the user will feel disoriented, groggy, and will suffer from impaired memory. Consequently, at block 950, the process activates an alarm, when the user is in light sleep and when the current time is at most one hour before the user specified wake-up time.

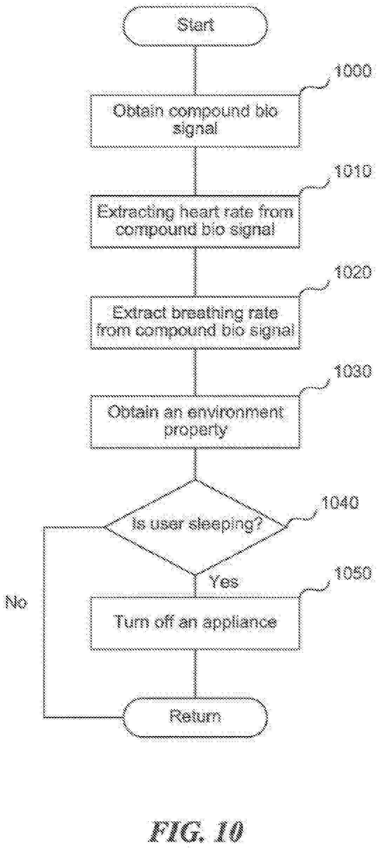

FIG. 10 is a flowchart of the process for turning off an appliance, according to one embodiment. At block 1000, the process obtains the compound bio signal associated with the user. The compound bio signal comprises the heart rate associated with the user, and the respiration rate associated with the user. According to one embodiment, the process obtains the compound bio signal from a sensor associated with the user. At block 1010, the process extracts the heart rate signal from the compound bio signal by, for example, performing low-pass filtering on the compound bio signal. Also, at block 1020, the process extracts the respiration rate signal from the compound bio signal by, for example, performing bandpass filtering on the compound bio signal. At block 1030, the process obtains an environment property, comprising temperature, humidity, light, sound from an environment sensor associated with the sensor strip. Based on the environment property and the sleep state associated with the user, at block 1040, the process determines whether the user is sleeping. If the user is sleeping, the process , at block 1050, turns an appliance off. For example, if the user is asleep and the environment temperature is above the average nightly temperature, the process turns off the thermostat. Further, if the user is asleep and the lights are on, the process turns off the lights. Similarly, if the user is asleep and the TV is on, the process turns off the TV.

### Smart home

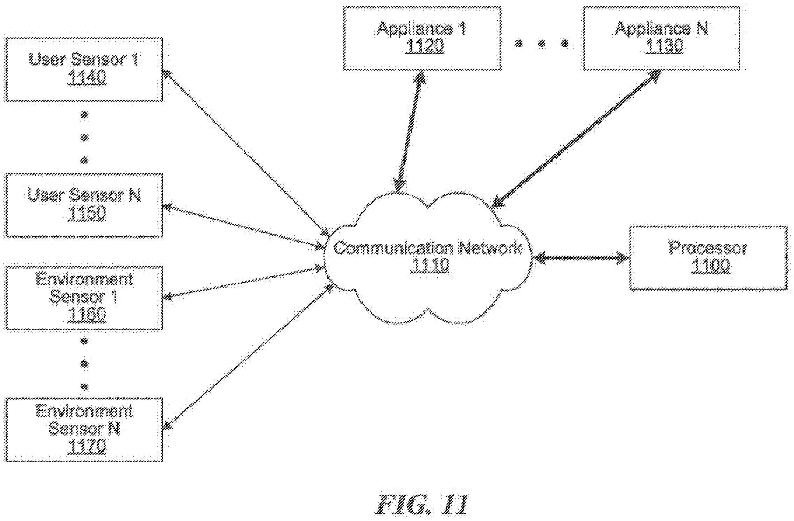

FIG. 11 is a diagram of a system capable of automating the control of the home appliances, according to one embodiment. Any number of user sensors 1140, 1150 monitor biological signals associated with the user, such as temperature, motion, presence, heart rate, or respiration rate. Any number of environment sensors 1160, 1170 monitor environment properties, such as temperature, sound, light, or humidity. According to one embodiment, the environment sensors 1160, 1170 are placed next to a bed. The user sensors 1140, 1150 and the environment sensors 1160, 1170 communicate their measurements to the processor 1100. The processor 1100 determines, based on the current biological signals associated with the user, historical biological signals associated with the user, user-specified preferences, exercise data associated with the user, and the environment properties received, a control signal, and a time to send the control signal to an appliance 1120, 1130.

The processor 1100 is any type of microcontroller, or any processor in a mobile terminal, fixed terminal, or portable terminal including a mobile handset, station, unit, device, multimedia computer, multimedia tablet, Internet node, cloud computer, communicator, desktop computer, laptop computer, notebook computer, netbook computer, tablet computer, personal communication system (PCS) device, personal navigation device, personal digital assistants (PDAs), audio/video player, digital camera/camcorder, positioning device, television receiver, radio broadcast receiver, electronic book device, game device, the accessories and peripherals of these devices, or any combination thereof.

The processor 1100 can be connected to the user sensor 1140, 1150, or the environment sensor 1160, 1170 by a computer bus, such as an I2C bus. Also, the processor 1100 can be connected to the user sensor 1140, 1150, or environment sensor 1160, 1170 by a communication network 1110. By way of example, the communication network 1110 connecting the processor 1100 to the user sensor 1140, 1150, or the environment sensor 1160, 1170 includes one or more networks such as a data network, a wireless network, a telephony network, or any combination thereof. The data network may be any local area network (LAN), metropolitan area network (MAN), wide area network (WAN), a public data network (e.g., the Internet), short range wireless network, or any other suitable packet-switched network, such as a commercially owned, proprietary packet-switched network, e.g., a proprietary cable or fiber-optic network, and the like, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks, code division multiple access (CDMA), wideband code division multiple access (WCDMA), wireless fidelity (WiFi), wireless LAN (WLAN), Bluetooth^{®}, Internet Protocol (IP) data casting, satellite, mobile ad-hoc network (MANET), and the like, or any combination thereof.

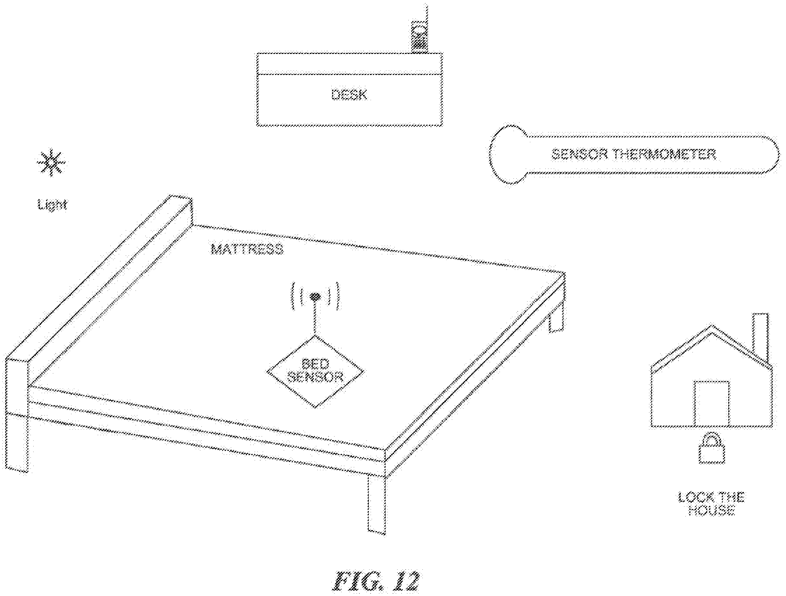

FIG. 12 is an illustration of the system capable of controlling an appliance and a home, according to one embodiment. The appliances, that the system disclosed here can control, comprise an alarm, a coffee machine, a lock, a thermostat, a bed device, a humidifier, or a light. For example, the system detects that the user has fallen asleep, the system sends a control signal to the lights to turn off, to the locks to engage, and to the thermostat to lower the temperature. According to another example, if the system detects that the user has woken up and it is morning, the system sends a control signal to the coffee machine to start making coffee.

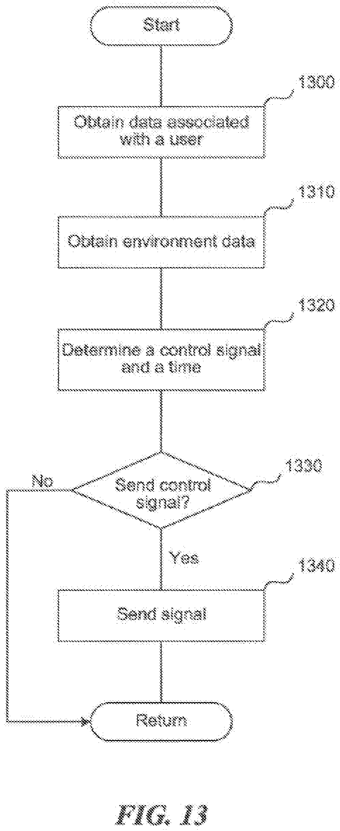

FIG. 13 is a flowchart of the process for controlling an appliance, according to one embodiment. In one embodiment, at block 1300, the process obtains history of biological signals, such as at what time does the user go to bed on a particular day of the week (e.g. the average bedtime associated with the user on Monday, the average bedtime associated with the user on Tuesday etc.). The history of biological signals can be stored in a database associated with the user, or in a database associated with the bed device. In another embodiment, at block 1300, the process also obtains user specified preferences, such as the preferred bed temperature associated with the user. Based on the history of biological signals and user-specified preferences, the process, at block 1320, determines a control signal, and a time to send the control signal to an appliance. It block 1330, the process determines whether to send a control signal to an appliance. For example, if the current time is within half an hour of average bedtime associated with the user on that particular day of the week, the process, at block 1340, sends a control signal to an appliance. For example, the control signal comprises an instruction to turn on the bed device, and the user specified bed temperature. Alternatively, the bed temperature is determined automatically, such as by calculating the average nightly bed temperature associated with a user.

According to another embodiment, at block 1300, the process obtains a current biological signal associated with a user from a sensor associated with the user. At block 1310, the process also obtains environment data, such as the ambient light, from an environment sensor associated with a bed device. Based on the current biological signal, the process identifies whether the user is asleep. If the user is asleep and the lights are on, the process sends an instruction to turn off the lights. In another embodiment, if the user is asleep, the lights are off, and the ambient light is high, the process sends an instruction to the blinds to shut. In another embodiment, if the user is asleep, the process sends an instruction to the locks to engage.

In another embodiment, the process, at block 1300, obtains history of biological signals, such as at what time the user goes to bed on a particular day of the week (e.g. the average bedtime associated with the user on Monday, the average bedtime associated with the user on Tuesday etc.). The history of biological signals can be stored in a database associated with the bed device, or in a database associated with a user. Alternatively, the user may specify a bedtime for the user for each day of the week. Further, the process obtains the exercise data associated with the user, such as the number of hours the user spent exercising, or the heart rate associated with the user during exercising. According to one embodiment, the process obtains the exercise data from a user phone, a wearable device, Fitbit bracelet, or a database associated with the user. Based on the average bedtime for that day of the week, and the exercise data during the day, the process, at block 1320, determines the expected bedtime associated with the user that night. The process then sends an instruction to the bed device to heat to a desired temperature, before the expected bedtime. The desired temperature can be specified by the user, or can be determined automatically, based on the average nightly temperature associated with the user.

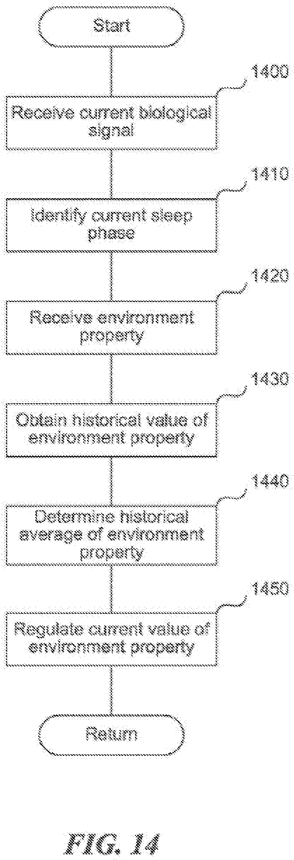

FIG. 14 is a flowchart of the process for controlling an appliance, according to another embodiment. The process, at block 1400, receives current biological signal associated with the user, such as the heart rate, respiration rate, presence, motion, or temperature, associated with the user. Based on the current biological signal, the process, at block 1410, identifies current sleep phase, such as light sleep, deep sleep, or REM sleep. The process, at block 1420 also receives a current environment property value, such as the temperature, the humidity, the light, or the sound. The process, at block 1430, accesses a database, which stores historical values associated with the environment property and the current sleep phase. That is, the database associates each sleep phase with an average historical value of the different environment properties. The database maybe associated with the bed device, maybe associated with the user, or maybe associated with a remote server. The process, at block 1440, then calculates a new average of the environment property based on the current value of the environment property and the historical value of the environment property, and assigns the new average to the current sleep phase in the database. If there is a mismatch between the current value of the environment property, and the historical average, the process, at block 1450, regulates the current value to match the historical average. For example, the environment property can be the temperature associated with the bed device. The database stores the average bed temperature corresponding to each of the sleep phase, light sleep, deep sleep, REM sleep. If the current bed temperature is below the historical average, the process sends a control signal to increase the temperature of the bed to match the historical average.

### Monitoring of biological signals

Biological signals associated with a person, such as a heart rate or a respiration rate, indicate the person's state of health. Changes in the biological signals can indicate an immediate onset of a disease, or a long-term trend that increases the risk of a disease associated with the person. Monitoring the biological signals for such changes can predict the onset of a disease, can enable calling for help when the onset of the disease is immediate, or can provide advice to the person if the person is exposed to a higher risk of the disease in the long-term.

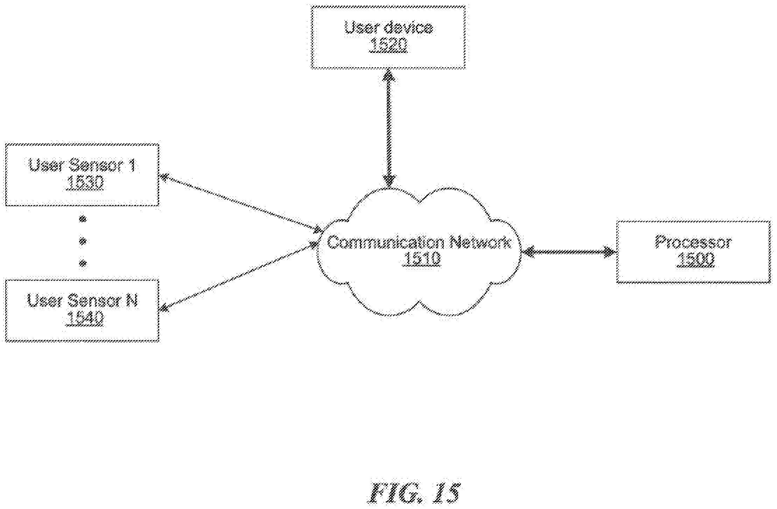

FIG. 15 is a diagram of a system for monitoring biological signals associated with a user, and providing notifications or alarms, according to one embodiment. Any number of user sensors 1530, 1540 monitor bio signals associated with the user, such as temperature, motion, presence, heart rate, or respiration rate. The user sensors 1530, 1540 communicate their measurements to the processor 1500. The processor 1500 determines, based on the bio signals associated with the user, historical biological signals associated with the user, or user-specified preferences whether to send a notification or an alarm to a user device 1520. In some embodiments, the user device 1520 and the processor 1500 can be the same device.

The user device 1520 is any type of a mobile terminal, fixed terminal, or portable terminal including a mobile handset, station, unit, device, multimedia computer, multimedia tablet, Internet node, communicator, desktop computer, laptop computer, notebook computer, netbook computer, tablet computer, personal communication system (PCS) device, personal navigation device, personal digital assistants (PDAs), audio/video player, digital camera/camcorder, positioning device, television receiver, radio broadcast receiver, electronic book device, game device, the accessories and peripherals of these devices, or any combination thereof.

The processor 1500 is any type of microcontroller, or any processor in a mobile terminal, fixed terminal, or portable terminal including a mobile handset, station, unit, device, multimedia computer, multimedia tablet, Internet node, cloud computer, communicator, desktop computer, laptop computer, notebook computer, netbook computer, tablet computer, personal communication system (PCS) device, personal navigation device, personal digital assistants (PDAs), audio/video player, digital camera/camcorder, positioning device, television receiver, radio broadcast receiver, electronic book device, game device, the accessories and peripherals of these devices, or any combination thereof.

The processor 1500 can be connected to the user sensor 1530, 1540 by a computer bus, such as an I2C bus. Also, the processor 1500 can be connected to the user sensor 1530, 1540 by a communication network 1510. By way of example, the communication network 1510 connecting the processor 1500 to the user sensor 1530, 1540 includes one or more networks such as a data network, a wireless network, a telephony network, or any combination thereof. The data network may be any local area network (LAN), metropolitan area network (MAN), wide area network (WAN), a public data network (e.g., the Internet), short range wireless network, or any other suitable packet-switched network, such as a commercially owned, proprietary packet-switched network, e.g., a proprietary cable or fiber-optic network, and the like, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks, code division multiple access (CDMA), wideband code division multiple access (WCDMA), wireless fidelity (WiFi), wireless LAN (WLAN), Bluetooth^{®}, Internet Protocol (IP) data casting, satellite, mobile ad-hoc network (MANET), and the like, or any combination thereof.

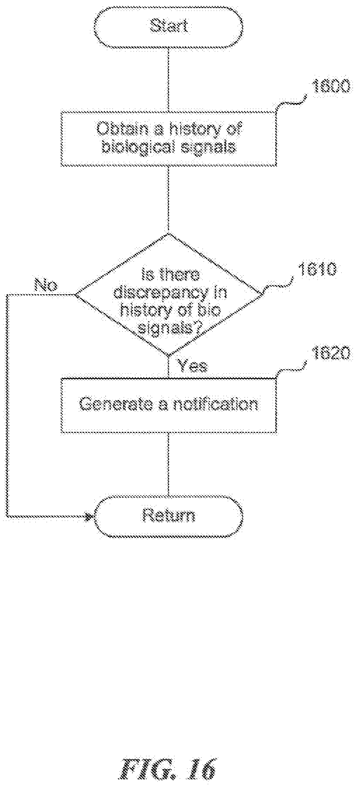

FIG. 16 is a flowchart of a process for generating a notification based on a history of biological signals associated with a user, according to one embodiment. The process, at block 1600, obtains a history of biological signals, such as the presence history, motion history, respiration rate history, or heart rate history, associated with the user. The history of biological signals can be stored in a database associated with a user. At block 1610, the process determines if there is an irregularity in the history of biological signals within a timeframe. If there is an irregularity, at block 1620, the process generates a notification to the user. The timeframe can be specified by the user, or can be automatically determined based on the type of irregularity. For example, the heart rate associated with the user goes up within a one day timeframe when the user is sick. According to one embodiment, the process detects an irregularity, specifically, that a daily heart rate associated with the user is higher than normal. Consequently, the process warns the user that the user may be getting sick. According to another embodiment, the process detects an irregularity, such as that an elderly user is spending at least 10% more time in bed per day over the last several days, than the historical average. The process generates a notification to the elderly user, or to the elderly user's caretaker, such as how much more time the elderly user is spending in bed. In another embodiment, the process detects an irregularity such as an increase in resting heart rate, by more than 15 beats per minute, over a ten-year period. Such an increase in the resting heart rate doubles the likelihood that the user will die from a heart disease, compared to those people whose heart rates remained stable. Consequently, the process warns the user that the user is at risk of a heart disease.

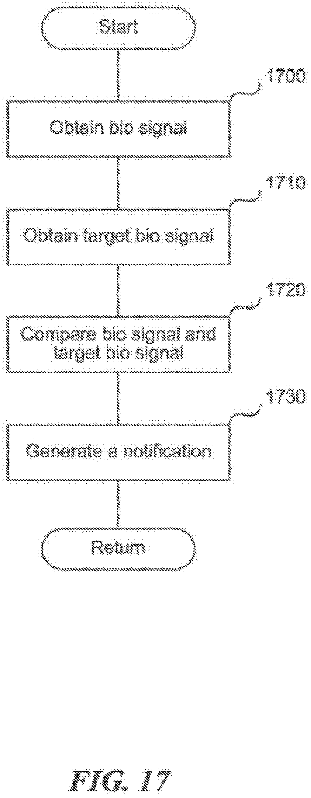

FIG. 17 is a flowchart of a process for generating a comparison between a biological signal associated with a user and a target biological signal, according to one embodiment. The process, at block 1700, obtains a current biological signal associated with a user, such as presence, motion, respiration rate, temperature, or heart rate, associated with the user. The process obtains the current biological signal from a sensor associated with the user. The process, at block 1710, then obtains a target biological signal, such as a user-specified biological signal, a biological signal associated with a healthy user, or a biological signal associated with an athlete. According to one embodiment, the process obtains the target biological signal from a user, or a database storing biological signals. The process, at block 1720, compares current bio signal associated with the user and target bio signal, and generates a notification based on the comparison 1730. The comparison of the current bio signal associated with the user and target bio signal comprises detecting a higher frequency in the current biological signal then in the target biological signal, detecting a lower frequency in the current biological signal than in the target biological signal, detecting higher amplitude in the current biological signal than in the target biological signal, or detecting lower amplitude in the current biological signal than in the target biological signal.

According to one embodiment, the process of FIG. 17 can be used to detect if an infant has a higher risk of sudden infant death syndrome ("SIDS"). In SIDS victims less than one month of age, heart rate is higher than in healthy infants of same age, during all sleep phases. SIDS victims greater than one month of age show higher heart rates during REM sleep phase. In case of monitoring an infant for a risk of SIDS, the process obtains the current bio signal associated with the sleeping infant, and a target biological signal associated with the heart rate of a healthy infant, where the heart rate is at the high end of a healthy heart rate spectrum. The process obtains the current bio signal from a sensor strip associated with the sleeping infant. The process obtains the target biological signal from a database of biological signals. If the frequency of the biological signal of the infant exceeds the target biological signal, the process generates a notification to the infant's caretaker, that the infant is at higher risk of SIDS.

According to another embodiment, the process of FIG. 17 can be used in fitness training. A normal resting heart rate for adults ranges from 60 to 100 beats per minute. Generally, a lower heart rate at rest implies more efficient heart function and better cardiovascular fitness. For example, a well-trained athlete might have a normal resting heart rate closer to 40 beats per minute. Thus, a user may specify a target rest heart rate of 40 beats per minute. The process FIG. 17 generates a comparison between the actual bio signal associated with the user and the target bio signal 1720, and based on the comparison, the process generates a notification whether the user has reached his target, or whether the user needs to exercise more 1730.

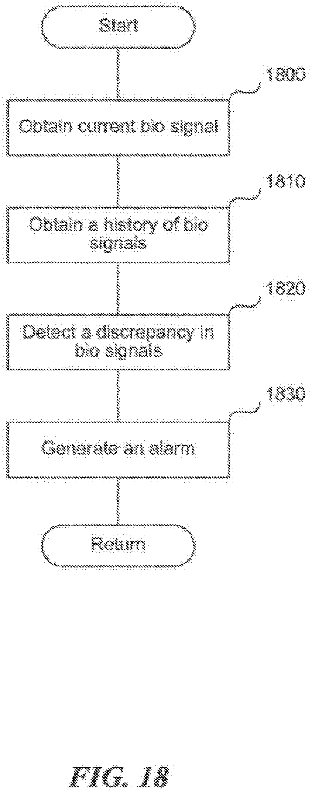

FIG. 18 is a flowchart of a process for detecting the onset of a disease, according to one embodiment. The process, at block 1800, obtains the current bio signal associated with a user, such as presence, motion, temperature, respiration rate, or heart rate, associated with the user. The process obtains the current bio signal from a sensor associated with the user. Further, the process, at block 1810, obtains a history of bio signals associated with the user from a database. The history of bio signals comprises the bio signals associated with the user, accumulated over time. The history of biological signals can be stored in a database associated with a user. The process, at block 1820, then detects a discrepancy between the current bio signal and the history of bio signals, where the discrepancy is indicative of an onset of a disease. The process, at block 1830, then generates an alarm to the user's caretaker. The discrepancy between the current bio signal and the history of bio signals comprises a higher frequency in the current bio signal than in the history of bio signals, or a lower frequency in the current bio signal than in the history of bio signals.

According to one embodiment, the process of FIG. 18 can be used to detect an onset of an epileptic seizure. A healthy person has a normal heart rate between 60 and 100 beats per minute. During epileptic seizures, the median heart rate associated with the person exceeds 100 beats per minute. The process of FIG. 18 detects that the heart rate associated with the user exceeds the normal heart rate range associated with the user. The process then generates an alarm to the user's caretaker that the user is having an epileptic seizure. Although rare, epileptic seizures can cause the median heart rate associated with a person to drop below 40 beats per minute. Similarly, the process of FIG. 18 detects if the current heart rate is below the normal heart rate range associated with the user. The process then generates an alarm to the user's caretaker that the user is having an epileptic seizure.

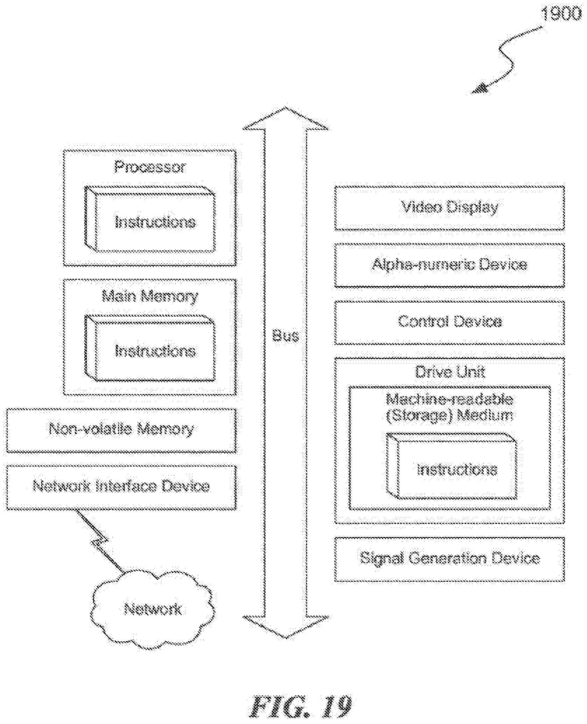

FIG. 19 is a diagrammatic representation of a machine in the example form of a computer system 1900 within which a set of instructions, for causing the machine to perform any one or more of the methodologies or modules discussed herein, may be executed.

In the example of FIG. 19, the computer system 1900 includes a processor, memory, non-volatile memory, and an interface device. Various common components (e.g., cache memory) are omitted for illustrative simplicity. The computer system 1900 is intended to illustrate a hardware device on which any of the components described in the example of FIGs. 1-18 (and any other components described in this specification) can be implemented. The computer system 1900 can be of any applicable known or convenient type. The components of the computer system 1900 can be coupled together via a bus or through some other known or convenient device.

This disclosure contemplates the computer system 1900 taking any suitable physical form. As example and not by way of limitation, computer system 1900 may be an embedded computer system, a system-on-chip (SOC), a single-board computer system (SBC) (such as, for example, a computer-on-module (COM) or system-on-module (SOM)), a desktop computer system, a laptop or notebook computer system, an interactive kiosk, a mainframe, a mesh of computer systems, a mobile telephone, a personal digital assistant (PDA), a server, or a combination of two or more of these. Where appropriate, computer system 1900 may include one or more computer systems 1900; be unitary or distributed; span multiple locations; span multiple machines; or reside in a cloud, which may include one or more cloud components in one or more networks. Where appropriate, one or more computer systems 1900 may perform without substantial spatial or temporal limitation one or more steps of one or more methods described or illustrated herein. As an example and not by way of limitation, one or more computer systems 1900 may perform in real time or in batch mode one or more steps of one or more methods described or illustrated herein. One or more computer systems 1900 may perform at different times or at different locations one or more steps of one or more methods described or illustrated herein, where appropriate.

The processor may be, for example, a conventional microprocessor such as an Intel Pentium microprocessor or Motorola power PC microprocessor. One of skill in the relevant art will recognize that the terms "machine-readable (storage) medium" or "computer-readable (storage) medium" include any type of device that is accessible by the processor.

The memory is coupled to the processor by, for example, a bus. The memory can include, by way of example but not limitation, random access memory (RAM), such as dynamic RAM (DRAM) and static RAM (SRAM). The memory can be local, remote, or distributed.

The bus also couples the processor to the non-volatile memory and drive unit. The non-volatile memory is often a magnetic floppy or hard disk, a magnetic-optical disk, an optical disk, a read-only memory (ROM), such as a CD-ROM, EPROM, or EEPROM, a magnetic or optical card, or another form of storage for large amounts of data. Some of this data is often written, by a direct memory access process, into memory during execution of software in the computer 1900. The non-volatile storage can be local, remote, or distributed. The non-volatile memory is optional because systems can be created with all applicable data available in memory. A typical computer system will usually include at least a processor, memory, and a device (e.g., a bus) coupling the memory to the processor.

Software is typically stored in the non-volatile memory and/or the drive unit. Indeed, storing and entire large program in memory may not even be possible. Nevertheless, it should be understood that for software to run, if necessary, it is moved to a computer readable location appropriate for processing, and for illustrative purposes, that location is referred to as the memory in this paper. Even when software is moved to the memory for execution, the processor will typically make use of hardware registers to store values associated with the software, and local cache that, ideally, serves to speed up execution. As used herein, a software program is assumed to be stored at any known or convenient location (from non-volatile storage to hardware registers) when the software program is referred to as "implemented in a computer-readable medium." A processor is considered to be "configured to execute a program" when at least one value associated with the program is stored in a register readable by the processor.

The bus also couples the processor to the network interface device. The interface can include one or more of a modem or network interface. It will be appreciated that a modem or network interface can be considered to be part of the computer system 1900. The interface can include an analog modem, isdn modem, cable modem, token ring interface, satellite transmission interface (e.g. "direct PC"), or other interfaces for coupling a computer system to other computer systems. The interface can include one or more input and/or output devices. The I/0 devices can include, by way of example but not limitation, a keyboard, a mouse or other pointing device, disk drives, printers, a scanner, and other input and/or output devices, including a display device. The display device can include, by way of example but not limitation, a cathode ray tube (CRT), liquid crystal display (LCD), or some other applicable known or convenient display device. For simplicity, it is assumed that controllers of any devices not depicted in the example of FIG. 9 reside in the interface.

In operation, the computer system 1900 can be controlled by operating system software that includes a file management system, such as a disk operating system. One example of operating system software with associated file management system software is the family of operating systems known as Windows^{®} from Microsoft Corporation of Redmond, Washington, and their associated file management systems. Another example of operating system software with its associated file management system software is the LinuxTM operating system and its associated file management system. The file management system is typically stored in the non-volatile memory and/or drive unit and causes the processor to execute the various acts required by the operating system to input and output data and to store data in the memory, including storing files on the non-volatile memory and/or drive unit.

Some portions of the detailed description may be presented in terms of algorithms and symbolic representations of operations on data bits within a computer memory. These algorithmic descriptions and representations are the means used by those skilled in the data processing arts to most effectively convey the substance of their work to others skilled in the art. An algorithm is here, and generally, conceived to be a self-consistent sequence of operations leading to a desired result. The operations are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

It should be borne in mind, however, that all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or "generating" or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The algorithms and displays presented herein are not inherently related to any particular computer or other apparatus. Various general purpose systems may be used with programs in accordance with the teachings herein, or it may prove convenient to construct more specialized apparatus to perform the methods of some embodiments. The required structure for a variety of these systems will appear from the description below. In addition, the techniques are not described with reference to any particular programming language, and various embodiments may thus be implemented using a variety of programming languages.

In alternative embodiments, the machine operates as a standalone device or may be connected (e.g., networked) to other machines. In a networked deployment, the machine may operate in the capacity of a server or a client machine in a client-server network environment, or as a peer machine in a peer-to-peer (or distributed) network environment.

The machine may be a server computer, a client computer, a personal computer (PC), a tablet PC, a laptop computer, a set-top box (STB), a personal digital assistant (PDA), a cellular telephone, an iPhone, a Blackberry, a processor, a telephone, a web appliance, a network router, switch or bridge, or any machine capable of executing a set of instructions (sequential or otherwise) that specify actions to be taken by that machine.

While the machine-readable medium or machine-readable storage medium is shown in an exemplary embodiment to be a single medium, the term "machine-readable medium" and "machine-readable storage medium" should be taken to include a single medium or multiple media (e.g., a centralized or distributed database, and/or associated caches and servers) that store the one or more sets of instructions. The term "machine-readable medium" and "machine-readable storage medium" shall also be taken to include any medium that is capable of storing, encoding or carrying a set of instructions for execution by the machine and that cause the machine to perform any one or more of the methodologies or modules of the presently disclosed technique and innovation.

In general, the routines executed to implement the embodiments of the disclosure, may be implemented as part of an operating system or a specific application, component, program, object, module or sequence of instructions referred to as "computer programs." The computer programs typically comprise one or more instructions set at various times in various memory and storage devices in a computer, and that, when read and executed by one or more processing units or processors in a computer, cause the computer to perform operations to execute elements involving the various aspects of the disclosure.

Moreover, while embodiments have been described in the context of fully functioning computers and computer systems, those skilled in the art will appreciate that the various embodiments are capable of being distributed as a program product in a variety of forms, and that the disclosure applies equally regardless of the particular type of machine or computer-readable media used to actually effect the distribution.

Further examples of machine-readable storage media, machine-readable media, or computer-readable (storage) media include but are not limited to recordable type media such as volatile and non-volatile memory devices, floppy and other removable disks, hard disk drives, optical disks (e.g., Compact Disk Read-Only Memory (CD ROMS), Digital Versatile Disks, (DVDs), etc.), among others, and transmission type media such as digital and analog communication links.

In some circumstances, operation of a memory device, such as a change in state from a binary one to a binary zero or vice-versa, for example, may comprise a transformation, such as a physical transformation. With particular types of memory devices, such a physical transformation may comprise a physical transformation of an article to a different state or thing. For example, but without limitation, for some types of memory devices, a change in state may involve an accumulation and storage of charge or a release of stored charge. Likewise, in other memory devices, a change of state may comprise a physical change or transformation in magnetic orientation or a physical change or transformation in molecular structure, such as from crystalline to amorphous or vice versa. The foregoing is not intended to be an exhaustive list of all exam page on pies in which a change in state for a binary one to a binary zero or vice-versa in a memory device may comprise a transformation, such as a physical transformation. Rather, the foregoing is intended as illustrative examples.

A storage medium typically may be non-transitory or comprise a non-transitory device. In this context, a non-transitory storage medium may include a device that is tangible, meaning that the device has a concrete physical form, although the device may change its physical state. Thus, for example, non-transitory refers to a device remaining tangible despite this change in state.

In many of the embodiments disclosed in this application, the technology is capable of allowing multiple different users to use the same piece of furniture equipped with the presently disclosed technology. For example, different people can sleep in the same bed. In addition, two different users can switch the side of the bed that they sleep on, and the technology disclosed here will correctly identify which user is sleeping on which side of the bed. The technology identifies the users based on any of the following signals alone or in combination: heart rate, respiration rate, body motion, or body temperature associated with each user.

Methods and systems of the present disclosure may be combined with or modified by other methods and systems for detecting a biological signal or a condition (e.g., a sleep disorder) of a user, regulating a temperature or configuration of a bed (e.g., a mattress or a mattress pad of the bed), regulating a biological signal or condition (e.g., a sleep disorder) of the user, regulating operation of home appliances, etc., such as, for example, those described in U.S. Patent Publication No. 2015/0351556 ("BED DEVICE SYSTEM AND METHODS"), U.S. Patent Publication No. 2016/0128488 ("APPARATUS AND METHODS FOR HEATING OR COOLING A BED BASED ON HUMAN BIOLOGICAL SIGNALS"), U.S. Patent Publication No. 2017/0135882 ("ADJUSTABLE BEDFRAME AND OPERATING METHODS FOR HEALTH MONITORING"), and U.S. Patent Publication No. 2017/0135632 ("DETECTING SLEEPING DISORDERS"), each of which is entirely incorporated herein by reference.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### EMBODIMENTS

### Thermal Alarm

Embodiment 1. A system for changing a temperature of a portion of an article of furniture, comprising: at least one sensor that is a part of the article of furniture, wherein the at least one sensor is configured to detect a biological signal of a user of the article of furniture; a temperature control device coupled to the portion of the article of furniture, wherein the temperature control device is configured to change the temperature of the portion of the article of furniture; and a processor communicatively coupled to the sensor and the temperature control device, wherein the processor is configured to (i) designate, while the user is asleep on the article of furniture, a time for the article of furniture to wake up the user based on the biological signal of the user that is detected by the at least one sensor while the user is using the article of furniture, and (ii) change the temperature of the portion of the article of furniture by the temperature control device prior to the time.

Embodiment 2. The system of Embodiment 1, wherein the processor is configured to change the temperature of the portion of the article of furniture at least 10 minutes prior to the time.

Embodiment 3. The system of Embodiment 1, wherein the processor is configured to change the temperature of the portion of the article of furniture at least 30 minutes prior to the time.

Embodiment 4. The system of any one of Embodiments 1-3, wherein, in (ii), a rate of change of the temperature of the portion of the article of furniture is at most 30 °F/hour.

Embodiment 5. The system of any one of Embodiments 1-3, wherein, in (ii), a rate of change of the temperature of the portion of the article of furniture is at most 10 °F/hour.

Embodiment 6. The system of any one of Embodiments 1-5, wherein, prior to (ii), the processor is further configured to designate a target temperature to which the temperature of the portion of the article of furniture is to be changed to.

Embodiment 7. The system of Embodiment 6, wherein the target temperature is designated based on a current temperature of the user.

Embodiment 8. The system of Embodiment 7, wherein a difference between the target temperature and the current temperature of the user is at least 1.5 °F.

Embodiment 9. The system of Embodiment 7, wherein a difference between the target temperature and the current temperature of the user is at least 3°F.

Embodiment 10. The system of Embodiment 6, wherein the target temperature is designated based on a current temperature of the portion of the article of furniture.

Embodiment 11. The system of Embodiment 10, wherein a difference between the target temperature and the current temperature of the portion of the article of furniture is at least 1.5 °F.

Embodiment 12. The system of Embodiment 10, wherein a difference between the target temperature and the current temperature of the portion of the article of furniture is at least 3 °F.

Embodiment 13. The system of Embodiment 6, wherein the target temperature is designated based on an ambient temperature of an environment surrounding the article of furniture.

Embodiment 14. The system of any one of Embodiments 1-13, wherein the processor is further configured to designate the time based on a circadian rhythm data of the user.

Embodiment 15. The system of any one of Embodiments 1-13, wherein the processor is further configured to designate the time based on a sleep phase data of the user.

Embodiment 16. The system of any one of Embodiments 1-13, wherein the processor is further configured to designate the time based on a health condition of the user.

Embodiment 17. The system of any one of Embodiments 1-13, wherein the processor is further configured to designate the time based on a planned event of the user.

Embodiment 18. The system of any one of Embodiments 1-13, wherein the processor is further configured to designate the time based on geolocation of the article of furniture.

Embodiment 19. The system of Embodiment 18, wherein the processor is further configured to determine the time based on a traffic condition near the geolocation.

Embodiment 20. The system of Embodiment 18, wherein the processor is further configured to determine the time based on a weather condition near the geolocation.

Embodiment 21. The system of any one of Embodiments 1-13, wherein the processor is further configured to determine the time based on an ambient temperature of an environment surrounding the article of furniture.

Embodiment 22. The system of any one of Embodiments 1-21, wherein the changing comprises increasing the temperature of the portion of the article of furniture.

Embodiment 23. The system of any one of Embodiments 1-21, wherein the changing comprises decreasing the temperature of the portion of the article of furniture.

Embodiment 24. The system of any one of Embodiments 1-23, wherein the article of furniture is a bed.

Embodiment 25. The system of any one of Embodiments 1-24, wherein the biological signal of the user comprises a heart signal of the user.

Embodiment 26. The system of any one of Embodiments 1-24, wherein the biological signal of the user comprises a respiration signal of the user.

Embodiment 27. The system of any one of Embodiments 1-24, wherein the biological signal of the user comprises a perspiration signal of the user.

Embodiment 28. The system of any one of Embodiments 1-24, wherein the biological signal of the user comprises a temperature of the user.

Embodiment 29. The system of any one of Embodiments 1-24, wherein the biological signal of the user comprises a motion of the user.

Embodiment 30. The system of any one of Embodiments 1-24, wherein the biological signal of the user comprises two or more members selected from the group consisting of: a heart signal of the user, a respiration signal of the user, a perspiration signal of the user, a temperature of the user, and a motion of the user.

Embodiment 31. The system of any one of Embodiments 1-30, wherein the portion of the article of furniture comprises a plurality of zones, and wherein the temperature control device is configured to selectively change a temperature of an individual zone of the plurality of zones.

Embodiment 32. The system of Embodiment 31, wherein the processor is configured to selectively change the temperature of the individual zone of the plurality of zones prior to the time.

Embodiment 33. The system of any one of Embodiments 1-32, wherein the processor is configured to (i) automatically designate the time for the article of furniture to wake up the user based on the biological signal of the user that is detected by the at least one sensor while the user is using the article of furniture, and (ii) automatically change the temperature of the portion of the article of furniture by the temperature control device prior to the time.

Embodiment 34. The system of any one of Embodiments 1-32, wherein the processor is further configured to designate the time based on the biological signal of the user and a history of biological signal data of the user, wherein the history of biological signal data comprises a plurality of measurements of the user's biological signal while using the article of furniture.

Embodiment 35. The system of Embodiment 34, wherein the processor is communicatively coupled to at least one database, wherein the at least one database comprises a database associated with the article of furniture or a database associated with the user, and wherein the processor is further configured to obtain the history of biological signal data of the user from the at least one database.

Embodiment 36. The system of Embodiment 34, wherein the history of biological signal data of the user comprises measurements of the user's biological signal during a current use of the article of furniture by the user.

Embodiment 37. The system of Embodiment 36, wherein the current use ranges from about 1 to 12 hours prior to the time.

Embodiment 38. The system of Embodiment 36, wherein the current use ranges from about 1 to 8 hours prior to the time.

Embodiment 39. The system of Embodiment 36, wherein the current use ranges from about 1 to 6 hours prior to the time.

Embodiment 40. The system of Embodiment 34, wherein the history of biological signal data of the user comprises measurements of the user's biological signal during one or more previous uses of the article of furniture by the user.

Embodiment 41. The system of Embodiment 40, wherein the one or more previous uses have occurred at least about 1 day to 1 year prior to the time.

Embodiment 42. The system of Embodiment 40, wherein the one or more previous uses have occurred from at least about 1 day to 1 month prior to the time.

Embodiment 43. The system of Embodiment 40, wherein the one or more previous uses have occurred from at least about 1 day to 1 week prior to the time.

Embodiment 44. The system of Embodiment 34, wherein the processor is further configured to (i) identify the user of the article of furniture from a plurality of users of the article of furniture based on the biological signal of the user, and (ii) obtain the history of biological signal data of the user based at least in part on the user's identity.

Embodiment 45. The system of any one of Embodiments 1-44, wherein the processor is further configured to (i) identify the user of the article of furniture from a plurality of users of the article of furniture based on the biological signal of the user, and (ii) designate the time based on the user's identity.

Embodiment 46. The system of Embodiment 45, wherein the user's identity comprises one or more user data selected from the group consisting of: a circadian rhythm data associated with the user, a sleep phase data associated with the user, an activity data associated with the user, a predetermined wake-up time of the user, a history of wake-up time of the user, a historical average wake-up time of the user, a predetermined biological signal level or range of the user, one or more future events of the user, and a geolocation of the user.

Embodiment 47. The system of any one of Embodiments 1-46, wherein the at least one sensor comprises at least one piezo sensor.

Embodiment 48. The system of Embodiment 47, wherein the at least one piezo sensor is configured to measure the heart signal and/or the respiration signal of the user while the user is using the article of furniture.

Embodiment 49. The system of any one of Embodiments 1-46, wherein the at least one sensor comprises at least one temperature sensor.

Embodiment 50. The system of Embodiment 49, wherein the at least one temperature sensor is configured to measure the temperature of the user while the user is using the article of furniture.

Embodiment 51. The system of any one of Embodiments 1-50, wherein the portion of the article of furniture comprises a first zone and a second zone, wherein the temperature control device is configured to independently change a temperature of each of the first and second zones.

Embodiment 52. The system of Embodiment 51, wherein the processor is configured to independently: (i) designate, while a first user is asleep on the first zone of the article of furniture, a first time for the article of furniture to wake up the first user based on a first biological of the first user detected by the at least one sensor, and change a temperature of the first zone of the article of furniture prior to the first time, and (ii) designate, while a second user is asleep on the second zone of the article of furniture, a second time for the article of furniture to wake up the second user based on a second biological of the second user detected by the at least one sensor, and change a temperature of the second zone of the article of furniture prior to the second time.

Embodiment 53. A method of regulating a temperature of a portion of an article of furniture, comprising: (a) providing (i) at least one sensor that is a part of the article of furniture, wherein the at least one sensor is configured to detect a biological signal of a user of the article of furniture, (ii) a temperature control device coupled to the portion of the article of furniture, wherein the temperature control device is configured to change the temperature of the portion of the article of furniture, and (iii) a processor communicatively coupled to the at least one sensor and the temperature control device; (b) with aid of the at least one sensor, detecting the biological signal of the user of the article of furniture while the user is using the article of furniture; (c) with aid of the processor, designating, while the user is asleep on the article of furniture, a time for the article of furniture to wake up the user based at least in part on the detected biological signal of the user; and (d) with the aid of the processor, changing the temperature of the portion of the article of furniture by the temperature control device prior to the time.

Embodiment 54. The method of Embodiment 53, further comprising, with the aid of the processor and the temperature control device, changing the temperature of the portion of the article of furniture at least 10 minutes prior to the time.

Embodiment 55. The method of Embodiment 53, further comprising, with the aid of the processor and the temperature control device, changing the temperature of the portion of the article of furniture at least 30 minutes prior to the time.

Embodiment 56. The method of any one of Embodiments 53-55, wherein a rate of change of the temperature of the portion of the article of furniture is at most 30 °F/hour.

Embodiment 57. The method of any one of Embodiments 53-55, wherein a rate of change of the temperature of the portion of the article of furniture is at most 10 °F/hour.

Embodiment 58. The method of any one of Embodiments 53-57, further comprising, with aid of the processor, designating a target temperature to which the temperature of the portion of the article of furniture is to be changed to.

Embodiment 59. The method of Embodiment 58, wherein the target temperature is designated based on a current temperature of the user.

Embodiment 60. The method of Embodiment 59, wherein a difference between the target temperature and the current temperature of the user is at least 1.5 °F.

Embodiment 61. The method of Embodiment 59, wherein a difference between the target temperature and the current temperature of the user is at least 3 °F.

Embodiment 62. The method of Embodiment 58, wherein the target temperature is designated based on a current temperature of the portion of the article of furniture.

Embodiment 63. The method of Embodiment 62, wherein a difference between the target temperature and the current temperature of the portion of the article of furniture is at least 1.5 °F.

Embodiment 64. The method of Embodiment 62, wherein a difference between the target temperature and the current temperature of the portion of the article of furniture is at least 3 °F.

Embodiment 65. The method of Embodiment 58, wherein the target temperature is designated based on an ambient temperature of an environment surrounding the article of furniture.

Embodiment 66. The method of any one of Embodiments 53-65, further comprising, with the aid of the processor, designating the time based on a circadian rhythm data of the user.

Embodiment 67. The method of any one of Embodiments 53-65, further comprising, with the aid of the processor, designating the time based on a sleep phase data of the user.

Embodiment 68. The method of any one of Embodiments 53-65, further comprising, with the aid of the processor, designating the time based on a health condition of the user.

Embodiment 69. The method of any one of Embodiments 53-65, further comprising, with the aid of the processor, designating the time based on a planned event of the user.

Embodiment 70. The method of any one of Embodiments 53-65, further comprising, with the aid of the processor, designating the time based on geolocation of the article of furniture.

Embodiment 71. The method of Embodiment 70, further comprising, with the aid of the processor, determining the time based on a traffic condition near the geolocation.

Embodiment 72. The method of Embodiment 70, further comprising, with the aid of the processor, determining the time based on a weather condition near the geolocation.

Embodiment 73. The method of any one of Embodiments 53-65, further comprising, with the aid of the processor, determining the time based on an ambient temperature of an environment surrounding the article of furniture.

Embodiment 74. The method of any one of Embodiments 53-73, wherein the changing comprises increasing the temperature of the portion of the article of furniture.

Embodiment 75. The method of any one of Embodiments 53-73, wherein the changing comprises decreasing the temperature of the portion of the article of furniture.

Embodiment 76. The method of any one of Embodiments 53-75, wherein the article of furniture is a bed.

Embodiment 77. The method of any one of Embodiments 53-76, wherein the biological signal of the user comprises a heart signal of the user.

Embodiment 78. The method of any one of Embodiments 53-76, wherein the biological signal of the user comprises a respiration signal of the user.

Embodiment 79. The method of any one of Embodiments 53-76, wherein the biological signal of the user comprises a perspiration signal of the user.

Embodiment 80. The method of any one of Embodiments 53-76, wherein the biological signal of the user comprises a temperature of the user.

Embodiment 81. The method of any one of Embodiments 53-76, wherein the biological signal of the user comprises a motion of the user.

Embodiment 82. The method of any one of Embodiments 53-76, wherein the biological signal of the user comprises two or more members selected from the group consisting of: a heart signal of the user, a respiration signal of the user, a perspiration signal of the user, a temperature of the user, and a motion of the user.

Embodiment 83. The method of any one of Embodiments 53-82, wherein the portion of the article of furniture comprises a plurality of zones, and wherein the temperature control device is configured to selectively change a temperature of an individual zone of the plurality of zones.

Embodiment 84. The method of Embodiment 83, further comprising, with the aid of the processor, selectively changing the temperature of the individual zone of the plurality of zones prior to the time.

Embodiment 85. The method of any one of Embodiments 53-84, further comprising, with the aid of the processor, (i) automatically designating the time for the article of furniture to wake up the user based on the biological signal of the user that is detected by the at least one sensor while the user is using the article of furniture, and (ii) automatically changing the temperature of the portion of the article of furniture by the temperature control device prior to the time.

Embodiment 86. The method of any one of Embodiments 53-84, further comprising, with the aid of the processor, designating the time based on the biological signal of the user and a history of biological signal data of the user, wherein the history of biological signal data comprises a plurality of measurements of the user's biological signal while using the article of furniture.

Embodiment 87. The method of Embodiment 86, wherein the processor is communicatively coupled to at least one database, wherein the at least one database comprises a database associated with the article of furniture or a database associated with the user, the method further comprising, with the aid of the processor, obtaining the history of biological signal data of the user from the at least one database.

Embodiment 88. The method of Embodiment 86, wherein the history of biological signal data of the user comprises measurements of the user's biological signal during a current use of the article of furniture by the user.

Embodiment 89. The method of Embodiment 88, wherein the current use ranges from about 1 to 12 hours prior to the time.

Embodiment 90. The method of Embodiment 88, wherein the current use ranges from about 1 to 8 hours prior to the time.

Embodiment 91. The method of Embodiment 88, wherein the current use ranges from about 1 to 6 hours prior to the time.

Embodiment 92. The method of Embodiment 86, wherein the history of biological signal data of the user comprises measurements of the user's biological signal during one or more previous uses of the article of furniture by the user.

Embodiment 93. The method of Embodiment 92, wherein the one or more previous uses have occurred at least about 1 day to 1 year prior to the time.

Embodiment 94. The method of Embodiment 92, wherein the one or more previous uses have occurred from at least about 1 day to 1 month prior to the time.

Embodiment 95. The method of Embodiment 92, wherein the one or more previous uses have occurred from at least about 1 day to 1 week prior to the time.

Embodiment 96. The method of Embodiment 86, further comprising, with the aid of the processor, (i) identifying the user of the article of furniture from a plurality of users of the article of furniture based on the biological signal of the user, and (ii) obtaining the history of biological signal data of the user based at least in part on the user's identity.

Embodiment 97. The method of any one of Embodiments 53-96, further comprising, with the aid of the processor, (i) identifying the user of the article of furniture from a plurality of users of the article of furniture based on the biological signal of the user, and (ii) designating the time based on the user's identity.

Embodiment 98. The method of Embodiment 97, wherein the user's identity comprises one or more user data selected from the group consisting of: a circadian rhythm data associated with the user, a sleep phase data associated with the user, an activity data associated with the user, a predetermined wake-up time of the user, a history of wake-up time of the user, a historical average wake-up time of the user, a predetermined biological signal level or range of the user, one or more future events of the user, and a geolocation of the user.

Embodiment 99. The method of any one of Embodiments 53-98, wherein the at least one sensor comprises at least one piezo sensor.

Embodiment 100. The method of Embodiment 99, wherein the at least one piezo sensor is configured to measure the heart signal and/or the respiration signal of the user while the user is using the article of furniture.

Embodiment 101. The method of any one of Embodiments 53-98, wherein the at least one sensor comprises at least one temperature sensor.

Embodiment 102. The method of Embodiment 101, wherein the at least one temperature sensor is configured to measure the temperature of the user while the user is using the article of furniture.

Embodiment 103. The method of any one of Embodiments 53-102, wherein the portion of the article of furniture comprises a first zone and a second zone, wherein the temperature control device is configured to independently change a temperature of each of the first and second zones.

Embodiment 104. The method of Embodiment 103, further comprising, with the aid of the processor, independently: (i) designating, while a first user is asleep on the first zone of the article of furniture, a first time for the article of furniture to wake up the first user based on a first biological of the first user detected by the at least one sensor, and change a temperature of the first zone of the article of furniture prior to the first time, and (ii) designating, while a second user is asleep on the second zone of the article of furniture, a second time for the article of furniture to wake up the second user based on a second biological of the second user detected by the at least one sensor, and change a temperature of the second zone of the article of furniture prior to the second time.

Embodiment 105. A non-transitory computer readable medium comprising machine executable code that, upon execution by one or more computer processors, implements the method of any one of Embodiments 53-104.

Embodiment 106. A system comprising one or more computer processors and computer memory coupled thereto. The computer memory comprises machine executable code that, upon execution by the one or more computer processors, implements the method of any one of Embodiments 53-104.

Embodiment 107. A system for regulating a temperature of a portion of an article of furniture, comprising: a temperature control device operatively coupled to the portion of the article of furniture, configured to change the temperature of the portion of the article of furniture; and a processor communicatively coupled to the temperature control device, configured to designate a time to change the temperature of the portion of the article of furniture by the temperature control device based at least in part on a predetermined wake-up time of a user, wherein the time is prior to the predetermined wake-up time of the user.

Embodiment 108. The system of Embodiment 107, wherein the processor is configured to designate the time while the user is asleep on the article of furniture.

Embodiment 109. The system of Embodiment 107, wherein the time is at least a time period prior to the predetermined wake-up time.

Embodiment 110. The system of Embodiment 109, wherein the time period is greater than 1 minute.

Embodiment 111. The system of Embodiment 110, wherein the time period is greater than 10 minutes.

Embodiment 112. The system of Embodiment 111, wherein the time period is greater than 30 minutes.

Embodiment 113. The system of Embodiment 112, wherein the time period is greater than 60 minutes.

Embodiment 114. The system of any one of Embodiments 107-113, further comprising a sensor operatively coupled to the article of furniture, configured to detect a signal associated with (i) the user of the article of furniture, or (ii) the article of furniture.

Embodiment 115. The system of Embodiment 114, wherein the processor is further configured to designate the time to change the temperature of the portion of the article of furniture by the temperature control device based at least in part on the predetermined wake-up time and the signal.

Embodiment 116. The system of Embodiment 114, wherein the signal comprises a biological signal of the user.

Embodiment 117. The system of Embodiment 114, wherein the signal comprises an environmental signal of the article of furniture.

Embodiment 118. The system of Embodiment 114, wherein the sensor is a part of the article of furniture, communicatively coupled to the processor.

Embodiment 119. The system of Embodiment 114, wherein the sensor is an environment sensor communicatively coupled to the processor.

Embodiment 120. The system of any one of Embodiments 107-119, wherein the time is designated by the processor such that a rate of change of the temperature of the portion of the article of furniture by the temperature control device is at most 30 °F/hour.

Embodiment 121. The system of any one of Embodiments 107-119, wherein the time is designated by the processor such that a rate of change of the temperature of the portion of the article of furniture by the temperature control device is at most 10 °F/hour.

Embodiment 122. The system of any one of Embodiments 107-121, wherein the change comprises an increase of the temperature of the portion of the article of furniture by the temperature control device.

Embodiment 123. The system of any one of Embodiments 107-121, wherein the change comprises a decrease of the temperature of the portion of the article of furniture by the temperature control device.

Embodiment 124. A method of regulating a temperature of a portion of an article of furniture, comprising: (a) providing (i) a temperature control device operatively coupled to the portion of the article of furniture, configured to change the temperature of the portion of the article of furniture, and (ii) a processor communicatively coupled to the temperature control device; and (b) with aid of the processor, designating a time to change the temperature of the portion of the article of furniture by the temperature control device based at least in part on a predetermined wake-up time of a user, wherein the time is prior to the predetermined wake-up time of the user.

Embodiment 125. The method of Embodiment 124, further comprising, in (b), designating the time while the user is asleep on the article of furniture.

Embodiment 126. The method of Embodiment 124, wherein the time is at least a time period prior to the predetermined wake-up time.

Embodiment 127. The method of Embodiment 126, wherein the time period is greater than 1 minute.

Embodiment 128. The method of Embodiment 127, wherein the time period is greater than 10 minutes.

Embodiment 129. The method of Embodiment 128, wherein the time period is greater than 30 minutes.

Embodiment 130. The method of Embodiment 129, wherein the time period is greater than 60 minutes.

Embodiment 131. The method of any one of Embodiments 124-130, further comprising, in (a), providing a sensor operatively coupled to the article of furniture, configured to detect a signal associated with (i) the user of the article of furniture, or (ii) the article of furniture.

Embodiment 132. The method of Embodiment 131, further comprising, in (b), designating the time to change the temperature of the portion of the article of furniture by the temperature control device based at least in part on the predetermined wake-up time and the signal.

Embodiment 133. The method of Embodiment 131, wherein the signal comprises a biological signal of the user.

Embodiment 134. The method of Embodiment 131, wherein the signal comprises environmental signal of the article of furniture.

Embodiment 135. The method of Embodiment 131, wherein the sensor is a part of the article of furniture, communicatively coupled to the processor.

Embodiment 136. The method of Embodiment 131, wherein the sensor is an environment sensor communicatively coupled to the processor.

Embodiment 137. The method of any one of Embodiments 124-136, further comprising, in (b), designating the time such that a rate of change of the temperature of the portion of the article of furniture by the temperature control device is at most 30 °F/hour.

Embodiment 138. The method of any one of Embodiments 124-136, further comprising, in (b), designating the time such that a rate of change of the temperature of the portion of the article of furniture by the temperature control device is at most 10 °F/hour.

Embodiment 139. The method of any one of Embodiments 124-138, wherein the change comprises an increase of the temperature of the portion of the article of furniture by the temperature control device.

Embodiment 140. The method of any one of Embodiments 124-138, wherein the change comprises a decrease of the temperature of the portion of the article of furniture by the temperature control device.

Embodiment 141. A non-transitory computer readable medium comprising machine executable code that, upon execution by one or more computer processors, implements the method of any one of Embodiments 124-140.

Embodiment 142. A system comprising one or more computer processors and computer memory coupled thereto. The computer memory comprises machine executable code that, upon execution by the one or more computer processors, implements the method of any one of Embodiments 124-140.

### Temperature control device

Embodiment 143. A system for regulating a temperature of an article of furniture, the system comprising: a portion of the article of furniture configured to hold a fluid; a reservoir in fluid communication with the portion of the article of furniture, wherein the reservoir is configured to contain the fluid; a temperature regulator in fluid communication with the portion of the article of furniture and the reservoir, wherein the temperature regulator is configured to modulate a temperature of the fluid when the fluid is not contained in the reservoir; and a processor operatively coupled to the temperature regulator, wherein the processor is programmed to control the temperature regulator to modulate the temperature of the fluid, thereby to regulate the temperature of the portion of the article of furniture.

Embodiment 144. The system of Embodiment 143, wherein the article of furniture comprises a bed or a seat.

Embodiment 145. The system of Embodiment 144, wherein the bed comprises a mattress, a mattress pad, a blanket, a functional variant thereof, or a combination thereof.

Embodiment 146. The system of any one of Embodiments 143-145, wherein the fluid is a liquid.

Embodiment 147. The system of Embodiment 146, wherein the liquid is water.

Embodiment 148. The system of any one of Embodiments 143-147, wherein the temperature regulator is not part of the reservoir.

Embodiment 149. The system of any one of Embodiments 143-148, wherein the temperature regulator comprises a channel configured to hold the fluid and/or permit flow of the fluid.

Embodiment 150. The system of any one of Embodiments 143-149, wherein the temperature regulator comprises a thermoelectric engine configured to modulate the temperature of the fluid.

Embodiment 151. The system of any one of Embodiments 143-150, wherein the reservoir is not configured to modulate the temperature of the fluid.

Embodiment 152. The system of any one of Embodiments 143-151, wherein the reservoir comprises a removable container configured to contain the fluid.

Embodiment 153. The system of any one of Embodiments 143-152, further comprising a pump configured to retrieve the fluid from the reservoir and direct flow of the fluid from the pump, through the temperature regulator, and to the pump.

Embodiment 154. The system of Embodiment 153, wherein the pump is configured to prevent flow of the fluid from the pump to the reservoir.

Embodiment 155. The system of Embodiment 153, wherein the pump is further configured to separate the fluid in the temperature regulator from the fluid contained in the reservoir.

Embodiment 156. The system of Embodiment 153, wherein the pump is further configured to direct flow of the fluid from the pump, through the temperature regulator, through the portion of the article of furniture, and to the pump.

Embodiment 157. The system of Embodiment 153, wherein the pump is further configured to direct flow of the fluid from the pump, through the portion of the article of furniture, through the temperature regulator, and to the pump.

Embodiment 158. The system of Embodiment 153, wherein the processor is operatively coupled to the pump and programmed to control the pump to retrieve the fluid from the reservoir and direct flow of the fluid from the pump, through the temperature regulator, and to the pump.

Embodiment 159. The system of any one of Embodiments 143-158, further comprising a gate disposed between the reservoir and the temperature regulator, configured to prevent flow of the fluid away from the temperature regulator and towards the reservoir.

Embodiment 160. The system of Embodiment 159, wherein the gate is a one-way valve.

Embodiment 161. The system of any one of Embodiments 143-160, further comprising an additional portion of the article of furniture configured to hold the fluid, wherein the portion and the additional portion are different.

Embodiment 162. The system of Embodiment 161, wherein the additional portion of the article of furniture is in fluid communication with an additional temperature regulator configured to modulate the temperature of the fluid, wherein the temperature regulator and the additional temperature regulator are different.

Embodiment 163. The system of Embodiment 162, wherein the temperature regulator and the additional temperature regulator are not in fluid communication with each other.

Embodiment 164. The system of Embodiment 162, wherein the additional temperature regulator is in fluid communication with the reservoir.

Embodiment 165. The system of Embodiment 162, wherein the processor is operatively coupled to the additional temperature regulator and further programmed to control the additional temperature regulator to modulate the temperature of the fluid, thereby to regulate a temperature of the additional portion of the article of furniture.

Embodiment 166. The system of Embodiment 165, wherein the processor is further programmed to independently control the temperature regulator and the additional temperature regulator, thereby to independently regulate the temperature of the portion of the article of furniture and the temperature of the additional portion of the article of furniture.

Embodiment 167. The system of any one of Embodiments 143-166, wherein the portion of the article of furniture comprises a channel configured to hold the fluid and/or permit flow of the fluid.

Embodiment 168. The system of Embodiment 167, wherein the channel comprises a plurality of interconnected channels configured to hold the fluid and/or permit flow of the fluid.

Embodiment 169. The system of any one of Embodiments 143-168, further comprising an additional portion of the article of furniture that includes at least one sensor operatively coupled to the processor and configured to detect a biological signal of at least one user of the article of furniture.

Embodiment 170. The system of Embodiment 169, wherein the biological signal comprises a heart signal, a respiration signal, a motion, a temperature, or perspiration.

Embodiment 171. The system of Embodiment 169, wherein the processor is further configured to monitor (i) the biological signal of the at least one user, (ii) a sleep pattern of the at least one user based on the detected biological signal of the at least one user over a period of time, and/or (iii) a temperature setting of the portion of the article of furniture over the period of time.

Embodiment 172. The system of Embodiment 171, wherein the processor is further configured to compare the biological signal, the sleep pattern, and/or the temperature setting between two or more users.

Embodiment 173. The system of Embodiment 172, wherein the processor is further configured to start a group of two or more users based on the comparison of the biological signal, the sleep pattern, and/or the temperature setting.

Embodiment 174. The system of any one of Embodiments 143-173, wherein the modulating comprises changing the temperature of the portion of the article of furniture.

Embodiment 175. The system of Embodiment 174, wherein the changing comprises increasing the temperature of the portion of the article of furniture.

Embodiment 176. The system of Embodiment 174, wherein the changing comprises decreasing the temperature of the portion of the article of furniture.

Embodiment 177. A method for regulating a temperature of an article of furniture, the method comprising: (a) providing a temperature regulator in fluid communication with (i) the portion of the article of furniture capable of holding a fluid, and (ii) a reservoir capable of containing the fluid, wherein the temperature regulator is capable of modulating a temperature of the fluid when the fluid is not contained in the reservoir; and (b) controlling, by a computer system, the temperature regulator to modulate the temperature of the fluid, thereby regulating the temperature of the portion of the article of furniture.

Embodiment 178. The method of Embodiment 177, wherein the article of furniture further comprises a bed or a seat.

Embodiment 179. The method of Embodiment 178, wherein the bed comprises a mattress, mattress pad, a blanket, a functional variant thereof, or a combination thereof.

Embodiment 180. The method of any one of Embodiments 177-179, wherein the fluid is a liquid.

Embodiment 181. The method of Embodiment 180, wherein the liquid is water.

Embodiment 182. The method of any one of Embodiments 177-181, wherein the temperature regulator is not part of the reservoir.

Embodiment 183. The method of any one of Embodiments 177-182, further comprising, by the computer system, controlling the temperature regulator to modulate the temperature of the fluid that is not in the reservoir.

Embodiment 184. The method of any one of Embodiments 177-183, wherein the temperature regulator comprises a channel configured to hold the fluid and/or permit flow of the fluid.

Embodiment 185. The method of any one of Embodiments 177-184, wherein the temperature regulator comprises a thermoelectric engine configured to modulate the temperature of the fluid.

Embodiment 186. The method of any one of Embodiments 177-185, wherein the temperature of the fluid is not modulated in the reservoir.

Embodiment 187. The method of any one of Embodiments 177-186, further comprising, by the computer system, (i) retrieving the fluid from the reservoir to a pump, and (ii) directing flow of the fluid from the pump, through the temperature regulator, and to the pump.

Embodiment 188. The method of Embodiment 187, wherein the pump does not direct flow of the fluid from the pump to the reservoir.

Embodiment 189. The method of Embodiment 187, further comprising separating the fluid flowing through the temperature regulator from the fluid contained in the reservoir by using the pump.

Embodiment 190. The method of Embodiment 187, further comprising, by the computer system, directing flow of the fluid from the pump, through the temperature regulator, through the portion of the article of furniture, and to the pump.

Embodiment 191. The method of Embodiment 187, further comprising, by the computer system, directing flow of the fluid from the pump, through the portion of the article of furniture, through the temperature regulator, and to the pump.

Embodiment 192. The method of any one of Embodiments 177-191, further comprising, using a gate disposed between the reservoir and the temperature regulator to prevent flow of the fluid away from the temperature regulator and towards the reservoir.

Embodiment 193. The method of Embodiment 192, wherein the gate is a one-way valve.

Embodiment 194. The method of any one of Embodiments 177-193, further comprising: (a) providing an additional temperature regulator in fluid communication with an additional portion of the article of furniture capable of holding the fluid, wherein the additional temperature regulator is capable of modulating the temperature of the fluid; and (b) controlling, by the computer system, the additional temperature regulator to modulate the temperature of the fluid, thereby regulating an additional temperature of the additional portion of the article of furniture.

Embodiment 195. The method of Embodiment 194, wherein the portion of the article of furniture and the additional portion of the article of furniture are different.

Embodiment 196. The method of Embodiment 194, wherein the temperature regulator and the additional temperature regulator are not in fluid communication with each other.

Embodiment 197. The method of Embodiment 194, wherein the additional temperature regulator is in fluid communication with the reservoir.

Embodiment 198. The method of Embodiment 194, further comprising, by the computer system, independently controlling the temperature regulator and the additional temperature regulator, thereby independently regulating the temperature of the portion of the article of furniture and the additional temperature of the additional portion of the article of furniture.

Embodiment 199. The method of any one of Embodiments 177-198, further comprising detecting, by the computer system, a biological signal of at least one user of the article of furniture by using at least one sensor disposed in an additional portion of the article of furniture.

Embodiment 200. The method of Embodiment 199, wherein the biological signal comprises a heart signal, a respiration signal, a motion, a temperature, or perspiration.

Embodiment 201. The method of Embodiment 199, further comprising monitoring, by the computer system, (i) a sleep pattern of the at least one user based on the detected biological signal of the at least one user over a period of time, and/or (ii) a temperature setting of the portion of the article of furniture over the period of time.

Embodiment 202. The method of Embodiment 201, further comprising comparing, by the computer system, the sleep pattern and/or the temperature setting between two or more users.

Embodiment 203. The method of Embodiment 202, further comprising starting, by the computer system, a group of two or more users based on the comparison of the sleep pattern and/or the temperature setting.

Embodiment 204. The method of any one of Embodiments 177-203, wherein the modulating comprises changing the temperature of the portion of the article of furniture.

Embodiment 205. The method of Embodiment 204, wherein the changing comprises increasing the temperature of the portion of the article of furniture.

Embodiment 206. The method of Embodiment 204, wherein the changing comprises decreasing the temperature of the portion of the article of furniture.

Embodiment 207. A non-transitory computer readable medium comprising machine executable code that, upon execution by one or more computer processors, implements the method of any one of Embodiments 177-206.

Embodiment 208. A system comprising one or more computer processors and computer memory coupled thereto. The computer memory comprises machine executable code that, upon execution by the one or more computer processors, implements the method of any one of Embodiments 177-206.

Embodiment 209. A system for regulating a temperature of an article of furniture, comprising:
the article of furniture comprising a first portion and a second portion, wherein each of the first and second portions is configured to hold a fluid; a common temperature controller configured to modulate a temperature of the fluid, wherein the common temperature controller comprises (i) a first channel in fluid communication with the first portion of the article of furniture, and (ii) a second channel in fluid communication with the second portion of the article of furniture, wherein the first and second channels are configured to hold the fluid; and a processor operatively coupled to the common temperature controller, programmed to control the common temperature controller to modulate the temperature of the fluid, thereby to independently regulate a first temperature of the first portion of the article of furniture and a second temperature of the second portion of the article of furniture.

Embodiment 210. The system of Embodiment 209, wherein the article of furniture is a bed.

Embodiment 211. The system of any one of Embodiments 209-210, wherein the fluid is water.

Embodiment 212. The system of any one of Embodiments 209-210, wherein the first and second portions of the article of furniture are different.

Embodiment 213. The system of any one of Embodiments 209-210, wherein the common temperature controller further comprises a reservoir in fluid communication with the first and second channels of the common temperature controller, configured to contain the fluid.

Embodiment 214. The system of Embodiment 213, wherein the common temperature controller further comprises (i) a first temperature regulator in fluid communication with the first channel and configured to modulate the temperature of the fluid, and (ii) a second temperature regulator in fluid communication with the second channel and configured to modulate the temperature of the fluid.

Embodiment 215. The system of Embodiment 214, wherein the first and second temperature regulators are not part of the reservoir.

Embodiment 216. The system of Embodiment 214, wherein the first and/or second temperature regulator is a thermoelectric engine.

Embodiment 217. The system of Embodiment 214, wherein the reservoir is not configured to modulate the temperature of the fluid.

Embodiment 218. The system of Embodiment 214, wherein the common temperature controller further comprises (i) a first pump in fluid communication with the first channel, configured to direct flow of the fluid between the first channel and the first portion of the article of furniture, and/or (ii) a second pump in fluid communication with the second channel, configured to direct flow of the fluid between the second channel and the second portion of the article of furniture.

Embodiment 219. The system of Embodiment 214, wherein the common temperature controller further comprises (i) a first gate disposed between the reservoir and the first temperature regulator, which first gate is configured to prevent flow of the fluid away from the first temperature regulator and towards the reservoir, and/or (ii) a second gate disposed between the reservoir and the second temperature regulator, which second gate is configured to prevent flow of the fluid away from the second temperature regulator and towards the reservoir.

Embodiment 220. The system of any one of Embodiments 209-219, wherein the regulating comprises changing the first temperature of the first portion of the article of furniture or the second temperature of the second portion of the article of furniture.

Embodiment 221. The system of Embodiment 220, wherein the regulating comprises changing the first temperature of the first portion of the article of furniture and the second temperature of the second portion of the article of furniture.

Embodiment 222. The system of Embodiment 221, wherein the changing comprises (i) increasing the first temperature of the first portion of the article of furniture and (ii) increasing the second temperature of the second portion of the article of furniture.

Embodiment 223. The system of Embodiment 221, wherein the changing comprises (i) increasing the first temperature of the first portion of the article of furniture and (ii) decreasing the second temperature of the second portion of the article of furniture.

Embodiment 224. The system of Embodiment 221, wherein the changing comprises (i) decreasing the first temperature of the first portion of the article of furniture and (ii) decreasing the second temperature of the second portion of the article of furniture.

Embodiment 225. A method for regulating a temperature of an article of furniture, comprising: (a) providing a common temperature controller configured to modulate a temperature of a fluid, wherein the common temperature controller comprises (i) a first channel in fluid communication with a first portion of the article of furniture, and (ii) a second channel in fluid communication with a second portion of the article of furniture, wherein the first and second portions of the article of furniture are configured to hold a fluid, and wherein the first and second channels are configured to hold the fluid; and (b) controlling the common temperature controller to modulate the temperature of the fluid, thereby independently regulating a first temperature of the first portion of the article of furniture and a second temperature of the second portion of the article of furniture.

Embodiment 226. The method of Embodiment 225, wherein the article of furniture is a bed.

Embodiment 227. The method of any one of Embodiments 225-226, wherein the fluid is water.

Embodiment 228. The method of any one of Embodiments 225-226, wherein the first and second portions of the article of furniture are different.

Embodiment 229. The method of any one of Embodiments 225-226, wherein the common temperature controller further comprises a reservoir in fluid communication with the first and second channels of the common temperature controller, configured to contain the fluid.

Embodiment 230. The method of any one of Embodiments 225-226, further comprising controlling (i) a first temperature regulator in fluid communication with the first channel to modulate the temperature of the fluid, and (ii) a second temperature regulator in fluid communication with the second channel to modulate the temperature of the fluid, thereby independently regulating the first temperature of the first portion of the article of furniture and the second temperature of the second portion of the article of furniture.

Embodiment 231. The method of Embodiment 230, wherein the first and second temperature regulators are not part of the reservoir.

Embodiment 232. The method of Embodiment 230, wherein the first and/or second temperature regulator is a thermoelectric engine.

Embodiment 233. The method of Embodiment 230, wherein the reservoir is not configured to modulate the temperature of the fluid.

Embodiment 234. The method of Embodiment 230, further comprising controlling (i) a first pump in fluid communication with the first channel to direct flow of the fluid between the first channel and the first portion of the article of furniture, and/or (ii) a second pump in fluid communication with the second channel to direct flow of the fluid between the second channel and the second portion of the article of furniture.

Embodiment 235. The method of Embodiment 230, wherein the common temperature controller further comprises (i) a first gate disposed between the reservoir and the first temperature regulator, which first gate is configured to prevent flow of the fluid away from the first temperature regulator and towards the reservoir, and/or (ii) a second gate disposed between the reservoir and the second temperature regulator, which second gate is configured to prevent flow of the fluid away from the second temperature regulator and towards the reservoir.

Embodiment 236. The method of any one of Embodiments 225-235, wherein the regulating comprises changing the first temperature of the first portion of the article of furniture or the second temperature of the second portion of the article of furniture

Embodiment 237. The method of Embodiment 236, wherein the regulating comprises changing the first temperature of the first portion of the article of furniture and the second temperature of the second portion of the article of furniture.

Embodiment 238. The method of Embodiment 237, wherein the changing comprises (i) increasing the first temperature of the first portion of the article of furniture and (ii) increasing the second temperature of the second portion of the article of furniture.

Embodiment 239. The method of Embodiment 237, wherein the changing comprises (i) increasing the first temperature of the first portion of the article of furniture and (ii) decreasing the second temperature of the second portion of the article of furniture.

Embodiment 240. The method of Embodiment 237, wherein the changing comprises (i) decreasing the first temperature of the first portion of the article of furniture and (ii) decreasing the second temperature of the second portion of the article of furniture.

Embodiment 241. A non-transitory computer readable medium comprising machine executable code that, upon execution by one or more computer processors, implements the method of any one of Embodiments 225-240.

Embodiment 242. A system comprising one or more computer processors and computer memory coupled thereto. The computer memory comprises machine executable code that, upon execution by the one or more computer processors, implements the method of any one of Embodiments 225-240.
Additional statements of the present disclosure are as follows:
E1. A system for changing a temperature of a portion of an article of furniture, comprising:
   at least one sensor that is a part of said article of furniture, wherein said at least one sensor is configured to detect a biological signal of a user of said article of furniture;
   a temperature control device coupled to said portion of said article of furniture, wherein said temperature control device is configured to change said temperature of said portion of said article of furniture; and
   a processor communicatively coupled to said sensor and said temperature control device, wherein said processor is configured to (i) designate, while said user is asleep on said article of furniture, a time for said article of furniture to wake up said user based on said biological signal of said user that is detected by said at least one sensor while said user is using said article of furniture, and (ii) change said temperature of said portion of said article of furniture by said temperature control device prior to said time.
E2. The system of E 1, wherein said processor is configured to change said temperature of said portion of said article of furniture at least 10 minutes prior to said time.
E3. The system of E 1, wherein said processor is configured to change said temperature of said portion of said article of furniture at least 30 minutes prior to said time.
E4. The system of E 1, wherein, in (ii), a rate of change of said temperature of said portion of said article of furniture is at most 30 °F/hour.
E5. The system of E 1, wherein, in (ii), a rate of change of said temperature of said portion of said article of furniture is at most 10 °F/hour.
E6. The system of E 1, wherein, prior to (ii), said processor is further configured to designate a target temperature to which said temperature of said portion of said article of furniture is to be changed to.
E7. The system of E 6, wherein said target temperature is designated based on a current temperature of said user.
E8. The system of E 7, wherein a difference between said target temperature and said current temperature of said user is at least 1.5 °F.
E9. The system of E 8, wherein said target temperature is designated based on a current temperature of said portion of said article of furniture.
E10. The system of E 9, wherein a difference between said target temperature and said current temperature of said portion of said article of furniture is at least 1.5 °F.
E11. The system of E 6, wherein said target temperature is designated based on an ambient temperature of an environment surrounding said article of furniture.
E12. The system of E 1, wherein said processor is further configured to designate said time based on a circadian rhythm data of said user.
E13. The system of E 1, wherein said processor is further configured to designate said time based on a sleep phase data of said user.
E14. The system of E 1, wherein said processor is further configured to designate said time based on a health condition of said user.
E15. The system of E 1, wherein said processor is further configured to designate said time based on a planned event of said user.
E16. The system of E 1, wherein said processor is further configured to designate said time based on geolocation of said article of furniture.
E17. The system of E 16, wherein said processor is further configured to determine said time based on a traffic condition near said geolocation.
E18. The system of E 16, wherein said processor is further configured to determine said time based on a weather condition near said geolocation.
E19. The system of E 1, wherein said processor is further configured to determine said time based on an ambient temperature of an environment surrounding said article of furniture.
E20. The system of E 1, wherein said changing comprises increasing said temperature of said portion of said article of furniture.
E21. The system of E 1, wherein said changing comprises decreasing said temperature of said portion of said article of furniture.
E22. The system of E 1, wherein said article of furniture is a bed.
E23. The system of E 1, wherein said biological signal of said user comprises a heart signal of said user.
E24. The system of E 1, wherein said biological signal of said user comprises a respiration signal of said user.
E25. The system of E 1, wherein said biological signal of said user comprises a perspiration signal of said user.
E26. The system of E 1, wherein said biological signal of said user comprises a temperature of said user.
E27. The system of E 1, wherein said biological signal of said user comprises a motion of said user.
E28. The system of E 1, wherein said portion of said article of furniture comprises a plurality of zones, and wherein said temperature control device is configured to selectively change a temperature of an individual zone of said plurality of zones.
E29. The system of E 28, wherein said processor is configured to selectively change said temperature of said individual zone of said plurality of zones prior to said time.
E30. The system of E 1, wherein said processor is configured to (i) automatically designate said time for said article of furniture to wake up said user based on said biological signal of said user that is detected by said at least one sensor while said user is using said article of furniture, and (ii) automatically change said temperature of said portion of said article of furniture by said temperature control device prior to said time.

## Claims

1. A method for temperature regulation of a bed device, the method comprising:
(a) obtaining a waking time of a user;
(b) detecting one or more biological signals of the user over a current sleep session of the user on the bed device; and
(c) instructing a temperature regulator coupled to the bed device to adjust a temperature of at least a portion of the bed device to a target temperature at a predetermined time prior to the obtained waking time to wake the user during or after the user's current sleep session.

2. The method of claim 1, wherein the current sleep session comprises at least about four hours.

3. The method of claim 1 or 2, wherein (c) comprises:
(i) instructing the temperature regulator to increase the temperature of the at least the portion of the bed device to the target temperature; or
(ii) instructing the temperature regulator to decrease the temperature of the at least the portion of the bed device to the target temperature.

4. The method of any one of claims 1-3, wherein:
(i) the one or more biological signals comprise a plurality of heart signals of the user detected over the sleep session;
(ii) the one or more biological signals comprise a plurality of respiration signals of the user detected over the sleep session; or
(iii) the one or more biological signals comprise a plurality of motion or presence signals of the user detected over the sleep session.

5. The method of any one of claims 1-4, wherein the adjustment of the temperature is based on (i) the detected one or more biological signals and (ii) a sleep phase of the user at the time of detecting.

6. The method of claim 5, wherein the sleep phase comprises light sleep or wakefulness.

7. The method of any one of claims 1-6, wherein:
(i) the method further comprises instructing the temperature regulator or a different temperature regulator to adjust a temperature of a different portion of the bed device to a different target temperature at a time prior to another obtained waking time of a different user of the bed device, to wake the different user;
(ii) the method further comprises one or more of vibrating, rotating, or translating at least a portion of the bed device at said time prior or during to the obtained waking time to wake the user during or after the user's current sleep session;
(iii) said one or more biological signals are detected by at least one sensor of the bed device; or
(iv) the bed device is a mattress or a mattress cover.

8. The method of any one of claims 1-7, wherein the predetermined time prior to the obtained waking time is from 1 minute to 60 minutes,
optionally wherein the predetermined time prior to the obtained waking time is from 10 minute to 45 minutes.

9. The method of any one of claims 1-8, wherein the adjustment of the temperature to the target temperature is performed at a predetermined temperature regulation rate,
optionally wherein the predetermined temperature regulation rate is from least 20° F/hour to at least 40° F/hour.

10. The method of any one of claims 1-9, wherein the temperature regulator adjusts a temperature of a fluid disposed within the at least the portion of the bed device to the target temperature at the predetermined time, wherein the adjustment of the temperature of the fluid to the target temperature is performed at a rate between 0.01 °C/minute and 5 °C/minute.

11. The method of claim 10, wherein the adjustment of the temperature of the fluid to the target temperature is performed at a rate between 0.1 °C/minute and 5 °C/minute.

12. The method of any one of claims 1-11, wherein the adjustment of the temperature to the target temperature comprises a temperature change from 5° F to 50° F.

13. The method of any one of claims 1-12, wherein the adjustment of the temperature to the target temperature is performed within 0.5 minute to 10 minutes.

14. The method of any one of claims 1-13, further comprising determining that the user is no longer present on the bed device,
optionally wherein the method further comprises triggering a personal device based at least in part on the determining that the user is no longer present on the bed device.

15. A system comprising one or more computer processors and computer memory coupled thereto, wherein the computer memory comprises a machine executable code that, upon execution by the one or more computer processors, implements the method of any one of claims 1-14,
optionally wherein the system further comprises the temperature regulator.
